# EUROPEAN PATENT APPLICATION

(11) **EP 1 336 607 A1**
(43) Date of publication of application: **20.08.2003**
(21) Application number: 02388015.6
(22) Date of filing: 19.02.2002
(51) Int. Cl.: C07D 277/46, A61K 31/426, A61P 3/04, A61P 3/10, C07D 417/12, C07D 213/75, A61K 31/4402, C07D 409/12, A61K 31/505, C07D 239/42, C07D 405/12

(54) **Amide derivatives as glucokinase activators**

(71) Applicant: NOVO NORDISK A/S, 2880 Bagsvaerd (DK)
(72) Inventor: Guzel, Mustafa, Greensboro, NC 27409 (US); Santhosh, Kalpathy Chidambareswaran, High Point, NC 27265 (US); Mjalli, Adnan M.M., Jamestown, NC 27282 (US); Andrews, Robert Carl, Jamestown, NC 27282 (US); Polisetti, Dharma Rao, Greensboro, NC 27409 (US)

(57) **Abstract**

The invention relates to heterocyclic amide derivatives which are used to activate glucokinase e.g. in the treatment of obesity or diabetes.

## Description

### FIELD OF THE INVENTION

This invention relates to compounds which are activators of glucokinase (GK), which may be useful for the management, treatment, control, or adjunct treatment of diseases, where increasing glucokinase activity is beneficial.

### BACKGROUND OF THE INVENTION

Diabetes is characterised by an impaired glucose metabolism manifesting itself among other things by an elevated blood glucose level in the diabetic patients. Underlying defects lead to a classification of diabetes into two major groups: Type 1 diabetes, or insulin demanding diabetes mellitus (IDDM), which arises when patients lack β-cells producing insulin in their pancreatic glands, and type 2 diabetes, or non-insulin dependent diabetes mellitus (NIDDM), which occurs in patients with an impaired β-cell function besides a range of other abnormalities.

Type 1 diabetic patients are currently treated with insulin, while the majority of type 2 diabetic patients are treated either with sulphonylureas that stimulate β-cell function or with agents that enhance the tissue sensitivity of the patients towards insulin or with insulin. Among the agents applied to enhance tissue sensitivity towards insulin metformin is a representative example.

Even though sulphonylureas are widely used in the treatment of NIDDM this therapy is, in most instances, not satisfactory: In a large number of NIDDM patients sulphonylureas do not suffice to normalise blood sugar levels and the patients are, therefore, at high risk for acquiring diabetic complications. Also, many patients gradually lose the ability to respond to treatment with sulphonylureas and are thus gradually forced into insulin treatment. This shift of patients from oral hypoglycaemic agents to insulin therapy is usually ascribed to exhaustion of the β-cells in NIDDM patients.

In normal subjects as well as in diabetic subjects, the liver produces glucose in order to avoid hypoglycaemia. This glucose production is derived either from the release of glucose from glycogen stores or from gluconeogenesis, which is a de novo intracellular synthesis of glucose. In type 2 diabetes, however, the regulation of hepatic glucose output is poorly controlled and is increased, and may be doubled after an overnight fast. Moreover, in these patients there exists a strong correlation between the increased fasting plasma glucose levels and the rate of hepatic glucose production. Similarly, hepatic glucose production will be increased in Type 1 diabetes, if the disease is not properly controlled by insulin treatment.

Since existing forms of therapy of diabetes does not lead to sufficient glycaemic control and therefore are unsatisfactory, there is a great demand for novel therapeutic approaches.

Atherosclerosis, a disease of the arteries, is recognized to be the leading cause of death in the United States and Western Europe. The pathological sequence leading to atherosclerosis and occlusive heart disease is well known. The earliest stage in this sequence is the formation of "fatty streaks" in the carotid, coronary and cerebral arteries and in the aorta. These lesions are yellow in colour due to the presence of lipid deposits found principally within smooth-muscle cells and in macrophages of the intima layer of the arteries and aorta. Further, it is postulated that most of the cholesterol found within the fatty streaks, in turn, give rise to development of the "fibrous plaque", which consists of accumulated intimal smooth muscle cells laden with lipid and surrounded by extra-cellular lipid, collagen, elastin and proteoglycans. The cells plus matrix form a fibrous cap that covers a deeper deposit of cell debris and more extracellular lipid. The lipid is primarily free and esterified cholesterol. The fibrous plaque forms slowly, and is likely in time to become calcified and necrotic, advancing to the "complicated lesion" which accounts for the arterial occlusion and tendency toward mural thrombosis and arterial muscle spasm that characterize advanced atherosclerosis.

Epidemiological evidence has firmly established hyperlipidemia as a primary risk factor in causing cardiovascular disease (CVD) due to atherosclerosis. In recent years, leaders of the medical profession have placed renewed emphasis on lowering plasma cholesterol levels, and low density lipoprotein cholesterol in particular, as an essential step in prevention of CVD. The upper limits of "normal" are now known to be significantly lower than heretofore appreciated. As a result, large segments of Western populations are now realized to be at particular high risk. Independent risk factors include glucose intolerance, left ventricular hypertrophy, hypertension, and being of the male sex. Cardiovascular disease is especially prevalent among diabetic subjects, at least in part because of the existence of multiple independent risk factors in this population. Successful treatment of hyperlipidemia in the general population, and in diabetic subjects in particular, is therefore of exceptional medical importance.

Hypertension (or high blood pressure) is a condition, which occurs in the human population as a secondary symptom to various other disorders such as renal artery stenosis, pheochromocytoma, or endocrine disorders. However, hypertension is also evidenced in many patients in whom the causative agent or disorder is unknown. While such "essential" hypertension is often associated with disorders such as obesity, diabetes, and hypertriglyceridemia, the relationship between these disorders has not been elucidated. Additionally, many patients display the symptoms of high blood pressure in the complete absence of any other signs of disease or disorder.

It is known that hypertension can directly lead to heart failure, renal failure, and stroke (brain haemorrhaging). These conditions are capable of causing short-term death in a patient. Hypertension can also contribute to the development of atherosclerosis and coronary disease. These conditions gradually weaken a patient and can lead to long-term death.

The exact cause of essential hypertension is unknown, though a number of factors are believed to contribute to the onset of the disease. Among such factors are stress, uncontrolled emotions, unregulated hormone release (the renin, angiotensin aldosterone system), excessive salt and water due to kidney malfunction, wall thickening and hypertrophy of the vasculature resulting in constricted blood vessels and genetic factors.

The treatment of essential hypertension has been undertaken bearing the foregoing factors in mind. Thus a broad range of beta-blockers, vasoconstrictors, angiotensin converting enzyme inhibitors and the like have been developed and marketed as antihypertensives. The treatment of hypertension utilizing these compounds has proven beneficial in the prevention of short-interval deaths such as heart failure, renal failure, and brain haemorrhaging. However, the development of atherosclerosis or heart disease due to hypertension over a long period of time remains a problem. This implies that although high blood pressure is being reduced, the underlying cause of essential hypertension is not responding to this treatment.

Hypertension has been associated with elevated blood insulin levels, a condition known as hyperinsulinemia. Insulin, a peptide hormone whose primary actions are to promote glucose utilization, protein synthesis and the formation and storage of neutral lipids, also acts to promote vascular cell growth and increase renal sodium retention, among other things. These latter functions can be accomplished without affecting glucose levels and are known causes of hypertension. Peripheral vasculature growth, for example, can cause constriction of peripheral capillaries, while sodium retention increases blood volume. Thus, the lowering of insulin levels in hyperinsulinemics can prevent abnormal vascular growth and renal sodium retention caused by high insulin levels and thereby alleviates hypertension.

Cardiac hypertrophy is a significant risk factor in the development of sudden death, myocardial infarction, and congestive heart failure. Theses cardiac events are due, at least in part, to increased susceptibility to myocardial injury after ischemia and reperfusion, which can occur in out-patient as well as perioperative settings. There is an unmet medical need to prevent or minimize adverse myocardial perioperative outcomes, particularly perioperative myocardial infarction. Both non-cardiac and cardiac surgery are associated with substantial risks for myocardial infarction or death. Some 7 million patients undergoing non-cardiac surgery are considered to be at risk, with incidences of perioperative death and serious cardiac complications as high as 20-25% in some series. In addition, of the 400,000 patients undergoing coronary by-pass surgery annually, perioperative myocardial infarction is estimated to occur in 5% and death in 1-2%. There is currently no drug therapy in this area, which reduces damage to cardiac tissue from perioperative myocardial ischemia or enhances cardiac resistance to ischemic episodes. Such a therapy is anticipated to be life-saving and reduce hospitalizations, enhance quality of life and reduce overall health care costs of high risk patients.

Another field for the present invention is obesity or appetite regulation.

Obesity is a well-known risk factor for the development of many very common diseases such as atherosclerosis, hypertension, and diabetes. The incidence of obese people and thereby also these diseases is increasing throughout the entire industrialised world. Except for exercise, diet and food restriction no convincing pharmacological treatment for reducing body weight effectively and acceptably currently exist. However, due to its indirect but important effect as a risk factor in mortal and common diseases it will be important to find treatment for obesity and/or means of appetite regulation.

The term obesity implies an excess of adipose tissue. In this context obesity is best viewed as any degree of excess adiposity that imparts a health risk. The cut off between normal and obese individuals can only be approximated, but the health risk imparted by the obesity is probably a continuum with increasing adiposity. The Framingham study demonstrated that a 20% excess over desirable weight clearly imparted a health risk (Mann GV N.Engl.J.Med 291:226, 1974). In the United States a National Institutes of Health consensus panel on obesity agreed that a 20% increase in relative weight or a body mass index (BMI = body weight in kilograms divided by the square of the height in meters) above the 85th percentile for young adults constitutes a health risk. By the use of these criteria 20 to 30 percent of adult men and 30 to 40 percent of adult women in the United States are obese. (NIH, Ann Intern Med 103:147, 1985).

Even mild obesity increases the risk for premature death, diabetes, hypertension, atherosclerosis, gallbladder disease, and certain types of cancer. In the industrialised western world the prevalence of obesity has increased significantly in the past few decades. Because of the high prevalence of obesity and its health consequences, its prevention and treatment should be a high public health priority.

When energy intake exceeds expenditure, the excess calories are stored in adipose tissue, and if this net positive balance is prolonged, obesity results, i.e. there are two components to weight balance, and an abnormality on either side (intake or expenditure) can lead to obesity.

The regulation of eating behaviour is incompletely understood. To some extent appetite is controlled by discrete areas in the hypothalamus: a feeding centre in the ventrolateral nucleus of the hypothalamus (VLH) and a satiety centre in the ventromedial hypothalamus (VMH). The cerebral cortex receives positive signals from the feeding centre that stimulate eating, and the satiety centre modulates this process by sending inhibitory impulses to the feeding centre. Several regulatory processes may influence these hypothalamic centres. The satiety centre may be activated by the increases in plasma glucose and/or insulin that follow a meal. Meal-induced gastric distension is another possible inhibitory factor. Additionally the hypothalamic centres are sensitive to catecholamines, and beta-adrenergic stimulation inhibits eating behaviour. Ultimately, the cerebral cortex controls eating behaviour, and impulses from the feeding centre to the cerebral cortex are only one input. Psychological, social, and genetic factors also influence food intake.

At present a variety of techniques are available to effect initial weight loss. Unfortunately, initial weight loss is not an optimal therapeutic goal. Rather, the problem is that most obese patients eventually regain their weight. An effective means to establish and/or sustain weight loss is the major challenge in the treatment of obesity today.

Glucokinase (GK) plays an essential role in blood glucose homeostasis. GK catalyses glucose phosphorylation, and is the rate-limiting reaction for glycolysis in hepatocytes and pancreatic β-cells. In liver GK determine the rates of both glucose uptake and glycogen synthesis, and it is also thought to be essential for the regulation of various glucose-responsive genes (Girard, J.et al., Annu Rev Nutr 17, 325-352 (1997)). In the β-cells, GK determines glucose utilization and thus is necessary for glucose-stimulated insulin secretion. GK is also expressed in a population of neurones in the hypothalamus where it might be involved in feeding behaviour, and in the gut where it might contribute to the secretion of enteroincretins.

GK has two main distinctive characteristics: its expression, which is limited to tissues that require glucose-sensing (mainly liver and pancreatic β-cells), and its S_{0.5} for glucose, which is much higher (8-12 mM) than that of the other members of the hexokinase family. Due to these kinetic characteristics, changes in serum glucose levels are paralleled by changes in glucose metabolism in liver which in turn regulate the balance between hepatic glucose output and glucose consumption.

Activators of glucokinase may thus be useful for treating diseases where increasing the activity of glucokinase is beneficial. Thus, there is a need for agents which activate glucokinase and increase glucokinase enzymatic activity. Such agents would be useful for the treatment of type I diabetes and type II diabetes.

WO 00/58293, WO 01/44216, WO/0183465, WO/0183478,WO/0185706, and WO 01/85707, to Hoffman-La Roche, discloses compounds as glucokinase activators that increase insulin secretion, for treatment of type 2 diabetes.

### SUMMARY OF THE INVENTION

This invention provides amide derivatives which are activators of glucokinase. The compounds of the invention are useful as activators of glucokinase and thus are useful for the management, treatment, control and adjunct treatment of diseases where increasing the activity of glucokinase is beneficial. Such diseases include Type I diabetes and Type II diabetes. The present invention provides compounds as described below, pharmaceutical compositions comprising the compounds, their use for increasing the activity of glucokinase, their use in preparation of a medicament for treating said diseases and conditions and the use of compounds or pharmaceutical preparations of the present invention for treating said diseases and conditions as well as methods for treating said diseases and conditions, which methods comprise administering to a subject in need thereof an effective amount of a compound according to the present invention. Other embodiments and aspects can be seen below.

### DETAILED DESCRIPTION OF THE INVENTION

The present invention provides carboxamide or sulfonamide activator of glucokinase having a heteroatom in the alpha, beta, or gamma position relative to the carboxamide or sulfonamide, respectively.

In one aspect, the present invention provides compounds of the general formula (I) 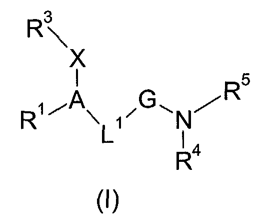 wherein
G is -S(O₂)-, or -C(O)-;
A is >N-, and
   X is a direct bond, -O-, -S-, -S(O)-, -S(O₂)-, or -N(R⁶) -, wherein
      R⁶ is hydrogen or alkyl, which may optionally be substituted with one or more substituents R¹⁶, R¹⁷, and R¹⁸, and
   L¹ is -(CH₂)ₙ-C(R⁹)(R¹⁰)ₘ-Y-, or a direct bond, wherein
      n is an integer of from 1 to 6,
      R⁹ and R¹⁰ independently are selected from alkyl, or cycloalkyl, optionally substituted by one or more substituents R¹⁹, R²⁰, and R²¹; or from aryl optionally substituted by one or more substituents R⁴⁰, R⁴¹, R⁴², and R⁴³, m is an integer of 0 to 1, and
      Y is a direct bond, -O- or -N(R⁷)-, wherein
         R⁷ is hydrogen or alkyl, which may optionally be substituted with one or more substituents R²², R²³, and R²⁴;
         or
   X is alkylene, which may optionally be substituted with one or more substituents R²⁵, R²⁶, and R²⁷, or a direct bond, and
   L¹ is -O-, or -N(R⁸)-, wherein
      R⁸ is hydrogen or alkyl, which may optionally be substituted with one or more substituents R²⁸, R²⁹, and R³⁰;
   or
A is >C(R²)-, wherein R² is hydrogen or alkyl, optionally substituted with one or more substituents R³¹, R³², and R³³, and
   X is -O-, -S-, -S(O)-, -S(O₂)-, or -N(R⁶)-, wherein
      R⁶ is as defined above, and
   L¹ is -(CH₂)ₙ-Y-, or a direct bond, wherein
      n is an integer of from 1 to 6, and
      Y is a direct bond, O, or -N(R⁷)-, wherein
         R⁷ is as defined above;
      or
   X is alkylene, which may optionally be substituted with one or more substituents R²⁵, R²⁶, and R²⁷, or a direct bond, and
   L¹ is -O-, or -N(R⁸)-, wherein
      R⁸ is as defined above;
R¹ and R³ independently are selected from alkyl, alkenyl, alkynyl, cycloalkyl, and heterocyclyl, optionally substituted with one or more substituents R³⁴, R³⁵, and R³⁶; or from aryl, heteroaryl, fused heterocyclylaryl, fused heteroarylheterocyclyl, fused heterocyclylheteroaryl, fused arylcycloalkyl, fused cycloalkylaryl, fused heteroarylcycloalkyl, and fused cycloalkylheteroaryl, optionally substituted with one or more substituents R⁴⁴, R⁴⁵, R⁴⁶, and R⁴⁷;
   or
R¹ and R³ may be taken together with the atoms to which they are attached to form a cycloalkyl or heterocyclyl ring, optionally substituted with one or more substituents R³⁴, R³⁵, and R³⁶, and optionally fused to a heteroaryl or aryl ring, optionally substituted with one or more substituents R⁴⁴, R⁴⁵, R⁴⁶, and R⁴⁷;
   or, when A is >C(R²)-, then
   R¹ and R² may be taken together with the atoms to which they are attached to form a cycloalkyl or heterocyclyl ring, optionally substituted with one or more substituents
   R³⁴, R³⁵, and R³⁶, and optionally fused to a heteroaryl or aryl ring, optionally substituted with one or more substituents R⁴⁴, R⁴⁵, R⁴⁶, and R⁴⁷;
      or
   R² and R³ may be taken together with the atoms to which they are attached to form a cycloalkyl or heterocyclyl ring, optionally substituted with one or more substituents
   R³⁴, R³⁵, and R³⁶, and optionally fused to a heteroaryl or aryl ring, optionally substituted with one or more substituents R⁴⁴, R⁴⁵, R⁴⁶, and R⁴⁷;
R⁴ is hydrogen or alkyl, optionally substituted with one or more substituents R³⁷, R³⁸, and R³⁹; and
R⁵ is aryl or heteroaryl, optionally substituted with one or more substituents R⁴⁸, R⁴⁹, R⁵⁰, and R⁵¹;
   wherein
R¹⁶, R¹⁷, R¹⁸, R¹⁹, R²⁰, R²¹, R²², R²³, R²⁴; R²⁵, R²⁶, R²⁷ R²⁸, R²⁹, R³⁰; R³¹, R³², R³³, R³⁴, R³⁵, R³⁶, R³⁷, R³⁸, and R³⁹ independently are selected from
   -CHF₂, -CF₃, -OCF₃, -OCHF₂, -OCH₂CF₃, -OCF₂CHF₂, -S(O)₂CF₃, -SCF₃, -OR⁵², -NR⁵²R⁵³, -SR⁵², -NR⁵²S(O)₂R⁵³, -S(O)₂NR⁵²R⁵³, -S(O)NR⁵²R⁵³, -S(O)R⁵², -S(O)₂R⁵², -C(O)NR⁵²R⁵³, -OC(O)NR⁵²R⁵³, -NR⁵²C(O)R⁵³, -CH₂C(O)NR⁵²R⁵³, -OCH₂C(O)NR⁵²R⁵³, -CH₂OR⁵², -CH₂NR⁵²R⁵³, -OC(O)R⁵², -C(O)R⁵² and -C(O)OR⁵²; C₂₋₆-alkenyl and C₂₋₆-alkynyl, which may optionally be substituted with one or more substituents selected from -CN, -CF₃, -OCF₃, -OR⁵², -NR⁵²R⁵³ and C₁₋₆-alkyl;
   C₃₋₈-cycloalkyl, C₄₋₈-cycloalkenyl, heterocyclyl, C₃₋₈-cycloalkyl-C₁₋₆-alkyl, C₃₋₈-cycloalkyl-C₁₋₆-alkoxy, C₃₋₈-cycloalkyloxy, C₃₋₈-cycloalkyl-C₁₋₆-alkylthio, C₃₋₈-cycloalkylthio, C₃₋₈-cycloalkyl-C₂₋₆-alkenyl, C₃₋₈-cycloalkyl-C₂₋₆-alkynyl, C₄₋₈-cycloalkenyl-C₁₋₆-alkyl, C₄₋₈-cycloalkenyl-C₂₋₆-alkenyl, C₄₋₈-cycloalkenyl-C₂₋₆-alkynyl, heterocyclyl-C₁₋₆-alkyl, heterocyclyl-C₂₋₆-alkenyl, heterocyclyl-C₂₋₆-alkynyl, aryl, aryloxy, aryloxycarbonyl, aroyl, aryl-C₁₋₆-alkoxy, aryl-C₁₋₆-alkyl, aryl-C₂₋₆-alkenyl, aryl-C₂₋₆-alkynyl, heteroaryl, heteroaryl-C₁₋₆-alkyl, heteroaryl-C₂₋₆-alkenyl and heteroaryl-C₂₋₆-alkynyl, of which the aryl and heteroaryl moieties optionally may be substituted with one or more substituents selected from halogen, -C(O)OR⁵², -CN, -CF₃, -OCF₃, -NO₂, -OR⁵², -NR⁵²R⁵³ and C₁₋₆-alkyl; and
R⁴⁰, R⁴¹, R⁴², R⁴³, R⁴⁴, R⁴⁵, R⁴⁶, and R⁴⁷ independently are selected from
   halogen, -CN, -CH₂CN, -CHF₂, -CF₃, -OCF₃, -OCHF₂, -OCH₂CF₃, -OCF₂CHF₂, -S(O)₂CF₃, -SCF₃, -NO₂, -OR⁵², -NR⁵²R⁵³, -SR⁵², -NR⁵²S(O)₂R⁵³, -S(O)₂NR⁵²R⁵³, -S(O)NR⁵²R⁵³, -S(O)R⁵², -S(O)₂R⁵², -C(O)NR⁵²R⁵³, -OC(O)NR⁵²R⁵³, -NR⁵²C(O)R⁵³, -CH₂C(O)NR⁵²R⁵³, -OCH₂C(O)NR⁵²R⁵³, -CH₂OR⁵², -CH₂NR⁵²R⁵³, -OC(O)R⁵², -C(O)R⁵², and -C(O)OR⁵²;
   C₁₋₆-alkyl, C₂₋₆-alkenyl and C₂₋₆-alkynyl, which may optionally be substituted with one or more substituents selected from -CN_{,} -CF_{3,} -OCF₃, -OR⁵², -NR⁵²R⁵³, and C₁₋₆-alkyl;
   C₃₋₈-cycloalkyl, C₄₋₈-cycloalkenyl, heterocyclyl, C₃₋₈-cycloalkyl-C₁₋₆-alkyl, C₃₋₈-cycloalkyl-C₁₋₆-alkoxy, C₃₋₈-cycloalkyloxy, C₃₋₈-cycloalkyl-C₁₋₆-alkylthio, C₃₋₈-cycloalkylthio, C₃₋₈-cycloalkyl-C₂₋₆-alkenyl, C₃₋₈-cycloalkyl-C₂₋₆-alkynyl, C₄₋₈-cycloalkenyl-C₁₋₆-alkyl, C₄₋₈-cycloalkenyl-C₂₋₆-alkenyl, C₄₋₈-cycloalkenyl-C₂₋₆-alkynyl, heterocyclyl-C₁₋₆-alkyl, heterocyclyl-C₂₋₆-alkenyl, heterocyclyl-C₂₋₆-alkynyl, aryl, aryloxy, aryloxycarbonyl, aroyl, aryl-C₁₋₆-alkoxy, aryl-C₁₋₆-alkyl, aryl-C₂₋₆-alkenyl, aryl-C₂₋₆-alkynyl, heteroaryl, heteroaryl-C₁₋₆-alkyl, heteroaryl-C₂₋₆-alkenyl and heteroaryl-C₂₋₆-alkynyl, of which the aryl and heteroaryl optionally may be substituted with one or more substituents selected from halogen, -C(O)OR⁵², -CN, -CF₃, -OCF₃, -NO₂, -OR⁵², -NR⁵²R⁵³, and C₁₋₆-alkyl;
      and
   two of R⁴⁰, R⁴¹, R⁴², and R⁴³, or two of R⁴⁴, R⁴⁵, R⁴⁶, and R⁴⁷ on adjacent carbon atoms may independently be taken together to form -O-CH₂-O-;
      wherein
   R⁵² and R⁵³ independently are hydrogen, C₁₋₆-alkyl, aryl-C₁₋₆-alkyl or aryl; or
   R⁵² and R⁵³,when attached to the same nitrogen atom, together with the said nitrogen atom may form a 3 to 8 membered heterocyclic ring optionally containing one or two further heteroatoms selected from nitrogen, oxygen and sulfur, and optionally containing one or two double bonds; and
R⁴⁸, R⁴⁹, R⁵⁰, and R⁵¹ independently are selected from
   halogen, perfluoroalkyl, cyano, alkyl-Z-, aryl-Z-, aryl-alkylene-Z-, N(R⁶³)(R⁶⁴)-alkylene-Z-; and R⁶⁵-W-alkylene-Z-; wherein
      Z and W independently are selected from a direct bond, alkylene, -O-, -N(R⁶⁶)-, -S-, -SO₂-, -C(O)N(R⁶⁶)-, -N(R⁶⁶)C(O)-, -N(R⁶⁶)C(O)N(R⁶⁷)-, -N(R⁶⁶)SO₂-, -SO₂N(R⁶⁶)-, -C(O)C-, -OC(O)-, and -N(R⁶⁶)SO₂N(R⁶⁷)- ; wherein
         R⁶⁶ and R⁶⁷ independently are hydrogen or alkyl;
         R⁶³, R⁶⁴, and R⁶⁵ are selected from the group consisting of hydrogen, aryl, alkyl, and aryl-alkylene-; or
         R⁶³ and R⁶⁴ may be taken together to form a ring having the formula -(CH₂)ⱼ-E-(CH₂)ₖ- bonded to the nitrogen atom to which R⁶³ and R⁶⁴ are attached, wherein
            j is an integer of from 1 to 4;
            k is an integer of from 1 to 4; and
            E is a direct bond, -CH₂-, -O-, -S-, -S(O₂)-, -C(O)-, -C(O)N(H)-, -NHC(O)-, -NHC(O)N(H)-, -NHSO₂-, -SO₂NH-, -C(O)O-, -OC(O)-, -NHSO₂NH-, 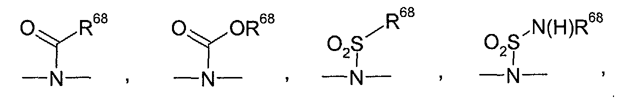 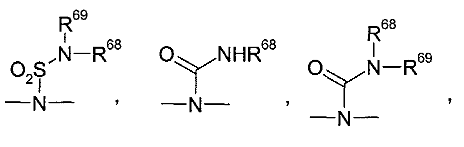 and 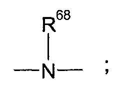 wherein
               R⁸⁸ and R⁶⁹ are selected from the group consisting of hydrogen, aryl, alkyl, and aryl-alkylene-;
or a pharmaceutically acceptable salt, solvate or prodrug thereof.

In one embodiment, the present invention provides compounds of the general formula (II) 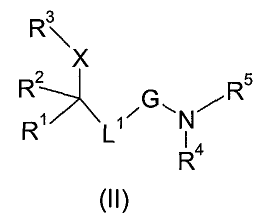 wherein
G is -S(O₂)-, or -C(O)-;
R² is hydrogen or alkyl, which may optionally be substituted with one or more substituents R³¹, R³², and R³³;
X is -O-, -S-, -S(O)-, -S(O₂)-, or -N(R⁶)-, wherein
   R⁶ is as described above;
L¹ is -(CH₂)ₙ-Y-, or a direct bond, wherein
   n is an integer of from 1 to 6,
   Y is a direct bond, O, or -N(R⁷)-, wherein
      R⁷ is as described above;
R¹ and R³ independently are selected from alkyl, alkenyl, alkynyl, cycloalkyl, and heterocyclyl, optionally substituted with one or more substituents R³⁴, R³⁵, and R³⁶; or from aryl, heteroaryl, fused heterocyclylaryl, fused heteroarylheterocyclyl, fused heterocyclylheteroaryl, fused arylcycloalkyl, fused cycloalkylaryl, fused heteroarylcycloalkyl, and fused cycloalkylheteroaryl, optionally substituted with one or more substituents R⁴⁴, R⁴⁵, R⁴⁶, and R⁴⁷;
or
R¹ and R² may be taken together with the atoms to which they are attached to form a cycloalkyl or heterocyclyl ring, optionally substituted with one or more substituents R³⁴, R³⁵, and R³⁶, and optionally fused to a heteroaryl or aryl ring, optionally substituted with one or more substituents R⁴⁴, R⁴⁵, R⁴⁶, and R⁴⁷;
R⁴ is as described above; and
R⁵ is as described above;
wherein R³¹, R³², R³³, R³⁴, R³⁵, R³⁶, R⁴⁴, R⁴⁵, R⁴⁶, and R⁴⁷ are as described above, or a pharmaceutically acceptable salt, solvate or prodrug thereof.

In a further embodiment of the above embodiment,
R¹ is selected from alkyl, alkenyl, alkynyl, cycloalkyl, and heterocyclyl, optionally substituted with one or more substituents R³⁴, R³⁵, and R³⁶; or from aryl-, and heteroaryl-, optionally substituted with one or more substituents R⁴⁴, R⁴⁵, R⁴⁶, and R⁴⁷; and
R² is hydrogen or alkyl, which may optionally be substituted with one or more substituents R³¹, R³², and R³³;
   wherein R³¹, R³², R³³, R³⁴, R³⁵, R³⁶, R⁴⁴, R⁴⁵, R⁴⁶, and R⁴⁷ are as defined above.

In another embodiment, the present invention provides compounds of the general formula (II) 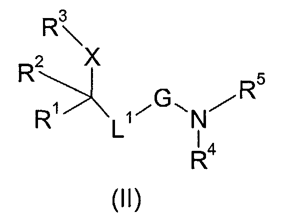 wherein
G is -S(O₂)-, or -C(O)-, and
   X is -O-, -S-, -S(O)-, -S(O₂)-, or -N(R⁶)-, wherein
      R⁶ is as described above, and
   L¹ is -(CH₂)ₙ-Y-, or a direct bond, wherein
      n is an integer of from 1 to 6, and
      Y is a direct bond, O, or -N(R⁷)-, wherein
         R⁷ is as described above;
      or
   X is alkylene, which may optionally be substituted with one or more substituents R²⁵, R²⁶, and R²⁷, or a direct bond, and
   L¹ is -O-, or -N(R⁸)-, wherein
      R⁸ is as described above;
R¹ is selected from alkyl, alkenyl, alkynyl, cycloalkyl, and heterocyclyl, optionally substituted with one or more substituents R³⁴, R³⁵, and R³⁶; or from aryl, heteroaryl, fused heterocyclylaryl, fused heteroarylheterocyclyl, fused heterocyclylheteroaryl, fused arylcycloalkyl, fused cycloalkylaryl, fused heteroarylcycloalkyl, and fused cycloalkylheteroaryl, optionally substituted with one or more substituents R⁴⁴, R⁴⁵, R⁴⁶, and R⁴⁷;
R² and R³ are taken together with the atoms to which they are attached to form a cycloalkyl or heterocyclyl ring, optionally substituted with one or more substituents R³⁴, R³⁵, and R³⁶, and optionally fused to a heteroaryl or aryl ring, optionally substituted with one or more substituents R⁴⁴, R⁴⁵, R⁴⁶, and R⁴⁷;
R⁴ is as described above; and
R⁵ is as described above;
wherein R²⁵, R²⁶, R²⁷, R³⁴, R³⁵, R³⁶, R⁴⁴, R⁴⁵, R⁴⁶, and R⁴⁷ are as described above; or a pharmaceutically acceptable salt, solvate or prodrug thereof.

In a further embodiment of the above embodiment, the present invention provides compounds of the general formula (IIa) 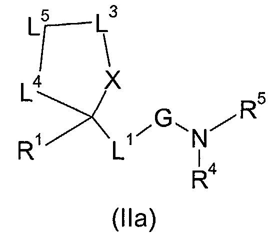 wherein
G, X, L¹, R¹, R⁴ and R⁵ are as defined above;
L₃ is -(C(R¹¹)(R¹²))ₚ-;
L₄ is -(C(R¹³)(R¹⁴))_{q}-; wherein
   R¹¹, R¹², R¹³, and R¹⁴ independently are hydrogen or alkyl optionally substituted with lower alkyl, phenyl or hydroxy,
   p is an integer of from 0 to 3, and
   q is an integer of from 0 to 3;
   and
L⁵ is a direct bond, alkenylene, alkynylene, arylene, heteroarylene, -O-, -C(O)-, -S-, -S(O)-, -S(O₂)-, or -N(R¹⁵)-, wherein
   R¹⁵ is hydrogen or alkyl, which may optionally be substituted with one or more substituents R⁵⁴, R⁵⁵, and R⁵⁶, wherein
      R⁵⁴, R⁵⁵, and R⁵⁶ are independently selected from -CHF₂, -CF₃, -OCF₃, -OCHF₂, -OCH₂CF₃, -OCF₂CHF₂, -S(O)₂CF₃, -SCF₃, -OR⁵⁷, -NR⁵⁷R⁵⁸, -SR⁵⁷, -NR⁵⁷S(O)₂R⁵⁸, -S(O)₂NR⁵⁷R⁵⁸, -S(O)NR⁵⁷R⁵⁸, -S(O)R⁵⁷, -S(O)₂R⁵⁷, -C(O)NR⁵⁷R⁵⁸, -OC(O)NR⁵⁷R⁵⁸, -NR⁵⁷C(O)R⁵⁸, -CH₂C(O)NR⁵⁷R⁵⁸, -OCH₂C(O)NR⁵⁷R⁵⁸, -CH₂OR⁵⁷, -CH₂NR⁵⁷R⁵⁸, -OC(O)R⁵⁷, -C(O)R⁵⁷, and -C(O)OR⁵⁷;
      C₂₋₆-alkenyl and C₂₋₆-alkynyl, which may optionally be substituted with one or more substituents selected from -CN, -CF₃, -OCF₃, -OR⁵⁷, -NR⁵⁷R⁵⁸ and C₁₋₆-alkyl;
      C₃₋₈-cycloalkyl, C₄₋₈-cycloalkenyl, heterocyclyl, C₃₋₈-cycloalkyl-C₁₋₆-alkyl, C₃₋₈-cycloalkyl-C₁₋₆-alkoxy, C₃₋₈-cycloalkyloxy, C₃₋₈-cycloalkyl-C₁₋₆-alkylthio, C₃₋₈-cycloalkylthio, C₃₋₈-cycloalkyl-C₂₋₆-alkenyl, C₃₋₈-cycloalkyl-C₂₋₆-alkynyl, C₄₋₈-cycloalkenyl-C₁₋₆-alkyl, C₄₋₈-cycloalkenyl-C₂₋₆-alkenyl, C₄₋₈-cycloalkenyl-C₂₋₆-alkynyl, heterocyclyl-C₁₋₆-alkyl, heterocyclyl-C₂₋₆-alkenyl, heterocyclyl-C₂₋₆-alkynyl, aryl, aryloxy, aryloxycarbonyl, aroyl, aryl-C₁₋₆-alkoxy, aryl-C₁₋₆-alkyl, aryl-C₂₋₆-alkenyl, aryl-C₂₋₆-alkynyl, heteroaryl, heteroaryl-C₁₋₆-alkyl, heteroaryl-C₂₋₆-alkenyl and heteroaryl-C₂₋₆-alkynyl, of which the aryl and heteroaryl moities optionally may be substituted with one or more substituents selected from halogen, -C(O)OR⁵⁷, -CN, -CF₃, -OCF₃, -NO₂, -OR⁵⁷, -NR⁵⁷R⁵⁸ and C₁₋₆-alkyl;
wherein
R⁵⁷ and R⁵⁸ independently are hydrogen, C₁₋₆-alkyl, aryl-C₁₋₆-alkyl or aryl,
   or
R⁵⁷ and R⁵⁸,when attached to the same nitrogen atom, together with the said nitrogen atom may form a 3 to 8 membered heterocyclic ring optionally containing one or two further heteroatoms selected from nitrogen, oxygen and sulfur, and optionally containing one or two double bonds.

In a further embodiment of the above embodiment, R¹¹, R¹², R¹³, and R¹⁴ are independently selected from hydrogen and alkyl, and in a still further embodiment of the above embodiment, R¹², R¹³, and R¹⁴ are hydrogen.

In another embodiment, the present invention provides compounds of the general formula (III) 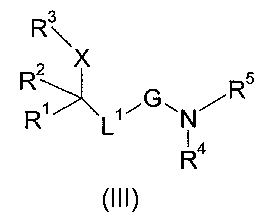 wherein
G is -S(O₂)-, or -C(O)-;
R² is hydrogen or alkyl, which may optionally be substituted with one or more substituents R³¹, R³², and R³³;
X is alkylene, which may optionally be substituted with one or more substituents R²⁵, R²⁶, and R²⁷, or a direct bond;
L¹ is -O-, or -N(R⁸)-, wherein R⁸ is as described above;
R¹ and R³ independently are selected from alkyl, alkenyl, alkynyl, cycloalkyl, and heterocyclyl, optionally substituted with one or more substituents R³⁴, R³⁵, and R³⁶; or from aryl, heteroaryl, fused heterocyclylaryl, fused heteroarylheterocyclyl, fused heterocyclylheteroaryl, fused arylcycloalkyl, fused cycloalkylaryl, fused heteroarylcycloalkyl, and fused cycloalkylheteroaryl, optionally substituted with one or more substituents R⁴⁴, R⁴⁵, R⁴⁶, and R⁴⁷;
   or
R¹ and R² may be taken together with the atoms to which they are attached to form a cycloalkyl or heterocyclyl ring, optionally substituted with one or more substituents R³⁴, R³⁵, and R³⁶, and optionally fused to a heteroaryl or aryl ring, optionally substituted with one or more substituents R⁴⁴, R⁴⁵, R⁴⁶, and R⁴⁷;
   or
R² and R³ may be taken together with the atoms to which they are attached to form a cycloalkyl or heterocyclyl ring, optionally substituted with one or more substituents R³⁴, R³⁵, and R³⁶, and optionally fused to a heteroaryl or aryl ring, optionally substituted with one or more substituents R⁴⁴, R⁴⁵, R⁴⁶, and R⁴⁷;
R⁴ is as described above; and
R⁵ is as described above;
wherein R²⁵, R²⁶, R²⁷, R³¹, R³², R³³, R³⁴, R³⁵, R³⁶, R⁴⁴, R⁴⁵, R⁴⁶, and R⁴⁷ are as described above;
or a pharmaceutically acceptable salt, solvate or prodrug thereof.

In a further embodiment of the above embodiment, R² is hydrogen or alkyl, which may optionally be substituted with one or more substituents R³¹, R³², and R³³, wherein R³¹, R³², and R³³ is as described above and does thus not form a cycloalkyl or heterocyclyl ring with R¹ or R³.

In another embodiment, the present invention provides compounds of the general formula (IV) 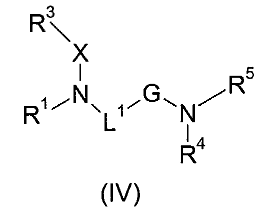 wherein
G is -S(O₂)-, or -C(O)-; and
   X is a direct bond, -O-, -S-, -S(O)-, -S(O₂)-, or -N(R⁶)-, wherein
      R⁶ is as described above, and
   L¹ is -(CH₂)ₙ- C(R⁹)(R¹⁰)ₘ-Y-, or a direct bond, wherein
      n is an integer of from 1 to 6,
      R⁹ and R¹⁰ are as described above;
      m is an integer of 0 to 1, and
      Y is a direct bond, -O- or -N(R⁷)-, wherein
         R⁷ is as described above;
         or
   X is alkylene, which may optionally be substituted with one or more substituents R²⁵, R²⁶, and R²⁷, or a direct bond, and
   L¹ is -O-, or -N(R⁸)-; wherein
      R⁸ is as described above;
R¹ and R³ independently are selected from alkyl, alkenyl, alkynyl, cycloalkyl, and heterocyclyl, optionally substituted with one or more substituents R³⁴, R³⁵, and R³⁶, or from aryl, heteroaryl, fused heterocyclylaryl, fused heteroarylheterocyclyl, fused heterocyclylheteroaryl, fused arylcycloalkyl, fused cycloalkylaryl, fused heteroarylcycloalkyl, and fused cycloalkylheteroaryl, optionally substituted with one or more substituents R⁴⁴, R⁴⁵, R⁴⁶, and R⁴⁷;
   or
R¹ and R³ may be taken together with the atoms to which they are attached to form a cycloalkyl or heterocyclyl ring, optionally substituted with one or more substituents R³⁴, R³⁵, and R³⁶, and optionally fused to a heteroaryl or aryl ring, optionally substituted with one or more substituents R⁴⁴, R⁴⁵, R⁴⁶, and R⁴⁷;
R⁴ is as described above; and
R⁵ is as described above;
wherein R²⁵, R²⁶, R²⁷, R³⁴, R³⁵, R³⁶, R⁴⁴, R⁴⁵, R⁴⁶, and R⁴⁷ are as described above; or a pharmaceutically acceptable salt, solvate or prodrug thereof.

In a further embodiment of the above embodiment, R¹ and R³ are independently selected from alkyl, alkenyl, alkynyl, cycloalkyl, and heterocyclyl, optionally substituted with one or more substituents R³⁴, R³⁵, and R³⁶, or from aryl-, and heteroaryl-, optionally substituted with one or more substituents R⁴⁴, R⁴⁵, R⁴⁶, and R⁴⁷ and does thus not form a cycloalkyl or heterocyclyl ring with each other.

In a further embodiment of each of the above embodiments, R⁴ is hydrogen or alkyl.

In a still further embodiment of the above embodiment, R⁴ is hydrogen.

In a further embodiment of each of the above embodiments, R⁵ is heteroaryl, optionally substituted with one or more substituents R⁴⁸, R⁴⁹, R⁵⁰, and R⁵¹, wherein R⁴⁸, R⁴⁹, R⁵⁰, and R⁵¹ are as defined above.

In a still further embodiment of the above embodiment, R⁵ is a five or six membered heteroaryl, optionally substituted with one or more substituents R⁴⁸, R⁴⁹, R⁵⁰, and R⁵¹,
wherein R⁴⁸, R⁴⁹, R⁵⁰, and R⁵¹ are as defined above.

In a still further embodiment of the above embodiment, R⁵ is selected from the group consisting of furan, thiophene, pyrrole, imidazole, pyrazole, triazole, tetrazole, thiazole, oxazole, isoxazole, oxadiazole, thiadiazole, isothiazole, pyridine, pyridazine, pyrazine, pyrimidine, quinoline, isoquinoline, benzofuran, benzothiophene, indole, and indazole; optionally substituted with one or more substituents R⁴⁸, R⁴⁹, R⁵⁰, and R⁵¹, wherein R⁴⁸, R⁴⁹, R⁵⁰, and R⁵¹ are as defined above.

In a further embodiment of each of the above embodiments, R⁴⁸, R⁴⁹, R⁵⁰, and R⁵¹ are independently selected from halogen, perfluoroalkyl, cyano, alkyl-Z-, aryl-alkylene-Z-, and R⁶⁵-W-alkylene-Z-; wherein
Z and W independently are selected from a direct bond, -O-, -N(R⁶⁶)-, -N(R⁶⁶)C(O)-, and -OC(O)-; wherein
   R⁶⁶ is hydrogen or alkyl; and
   R⁶⁵ is hydrogen or alkyl.

In a further embodiment of the above embodiment, R⁴⁸, R⁴⁹, R⁵⁰, and R⁵¹ are independently selected from from the group consisting of halogen, perfluoroalkyl, cyano, alkyl-OC(O)-alkylene-, alkyl-NHC(O)-alkylene-, N(alkyl)₂-C(O)-alkylene-, alkyl-OC(O)-, NH(alkyl)-C(O)-, N(alkyl)₂-C(O)-, aryl-alkylene-O-, and aryl-alkylene-NH-.

Further embodiments of these embodiments are clear from the appended claims.

Included within the scope of the invention are the individual enantiomers of the compounds represented by Formula (I) above as well as any wholly or partially racemic mixtures thereof. The present invention also covers the individual enantiomers of the compounds represented by Formula (I) above as mixtures with diastereoisomers thereof in which one or more stereocenters are inverted.

In the structural formulas given herein and throughout the present specification, the following terms have the indicated meaning:

The term "optionally substituted" as used herein means that the group in question is either unsubstituted or substituted with one or more of the substituents specified. When the group in question are substituted with more than one substituent the substituent may be the same or different.

The term "halogen" or "halo" means fluorine, chlorine, bromine or iodine.

The term "perhalomethyl" means trifluoromethyl, trichloromethyl, tribromomethyl, or triiodomethyl.

The use of prefixes of this structure: C_{x-y}-alkyl, C_{x-y}-alkenyl, C_{x-y}-alkynyl, C_{x-y}-cycloalyl or C_{x-y}-cycloalkyl-C_{x-y}-alkenyl designates radical of the designated type having from x to y carbon atoms.

The term "alkyl" as used herein, alone or in combination, refers to a straight or branched chain saturated monovalent hydrocarbon radical having from one to ten carbon atoms, for example C₁₋₈-alkyl. Typical C₁₋₈-alkyl groups include, but are not limited to e.g. methyl, ethyl, n-propyl, isopropyl, n-butyl, sec-butyl, isobutyl, tert-butyl, n-pentyl, 2-methylbutyl, 3-methylbutyl, 4-methylpentyl, neopentyl, n-pentyl, n-hexyl, 1,2-dimethylpropyl, 2,2-dimethylpropyl, 1,2,2-trimethylpropyl and the like. The term "C₁₋₈-alkyl" as used herein also includes secondary C₃₋₈-alkyl and tertiary C₄₋₈-alkyl.

The term "alkylene" as used herein, alone or in combination, refers to a straight or branched chain saturated divalent hydrocarbon radical having from one to ten carbon atoms, for example C₁₋₈-alkylene. Examples of "alkylene" as used herein include, but are not limited to, methylene, ethylene, and the like.

The term " alkenyl" as used herein, alone or in combination, refers to a straight or branched chain monovalent hydrocarbon radical containing from two to ten carbon atoms and at least one carbon-carbon double bond, for example C₂₋₈-alkenyl. Typical C₂₋₈-alkenyl groups include, but are not limited to, vinyl, 1-propenyl, 2-propenyl, iso-propenyl, 1,3-butadienyl, 1-butenyl, 2-butenyl, 3-butenyl, 2-methyl-1-propenyl, 1-pentenyl, 2-pentenyl, 3-pentenyl, 4-pentenyl, 3-methyl-2-butenyl, 1-hexenyl, 2-hexenyl, 3-hexenyl, 2,4-hexadienyl, 5-hexenyl and the like.

The term "alkenylene" as used herein, alone or in combination, refers to a straight or branched chain divalent hydrocarbon radical having from two to ten carbon atoms and at least one carbon-carbon double bond, for example C(₂₋₈)-alkenylene. Typical C(₂₋₈)-alkenylene groups include, but are not limited to, ethene-1,2-diyl, propene-1,3-diyl, methylene-1,1-diyl, and the like.

The term "alkynyl" as used herein alone or in combination, refers to a straight or branched hydrocarbon group containing from 2 to the specified number of carbon atoms and at least one triple carbon-carbon bond, for example C₂₋₈-alkynyl. Typical C₂₋₈-alkynyl groups include, but are not limited to, ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 5-hexynyl, 2,4-hexadiynyl and the like.

The term " alkynylene " as used herein alone or in combination, refers to a straight or branched chain divalent hydrocarbon radical having from two to ten carbon atoms and at least one carbon-carbon triple bond, for example C₂₋₈-alkynylene. Typical C₂₋₈-alkynylene groups include, but are not limited to, ethyne-1,2-diyl, propyne-1,3-diyl, and the like.

The term "cycloalkyl" as used herein, alone or in combination, refers to a nonaromatic carbocyclic monovalent hydrocarbon radical having from three to twelve carbon atoms, and optionally with one or more degrees of unsaturation, for example C₃₋₈-cycloalkyl. Such a ring may be optionally fused to one or more benzene rings or to one or more of other cycloalkyl ring(s). Typical C₃₋₈-cycloalkyl groups include, but are not limited to, cyclopropyl, cyclobutyl, cyclopentyl, cyclohexyl, cycloheptyl, cyclooctyl and the like.

The term "cycloalkylene" as used herein, alone or in combination, refers to a nonaromatic carbocyclic divalent hydrocarbon radical having from three to twelve carbon atoms and optionally possessing one or more degrees of unsaturation, for example C₃₋₈-cycloalkylene. Such a ring may be optionally fused to one or more benzene rings or to one or more of other cycloalkyl ring(s). Typical C₃₋₈-cycloalkylene groups include, but are not limited to, cyclopropyl-1,1-diyl, cyclopropyl-1,2-diyl, cyclobutyl-1,2-diyl, cyclopentyl-1,3-diyl, cyclohexyl-1,4-diyl, cycloheptyl-1,4-diyl, or cyclooctyl-1,5-diyl, and the like.

The term "heterocyclic" or the term "heterocyclyl" as used herein, alone or in combination, refers to a three to twelve membered heterocyclic ring having one or more degrees of unsaturation containing one or more heteroatomic substitutions selected from S, SO, SO₂, O, or N, for example C₃₋₈-heterocyclyl. Such a ring may be optionally fused to one or more of another "heterocyclic" ring(s) or cycloalkyl ring(s). Typical C₃₋₈-heterocyclyl groups include, but are not limited to, tetrahydrofuran, 1,4-dioxane, 1,3-dioxane, piperidine, pyrrolidine, morpholine, piperazine, and the like.

The term "heterocyclylene" as used herein, alone or in combination, refers to a three to twelve-membered heterocyclic ring diradical optionally having one or more degrees of unsaturation containing one or more heteroatoms selected from S, SO, SO₂, O, or N. Such a ring may be optionally fused to one or more benzene rings or to one or more of another "heterocyclic" rings or cycloalkyl rings. Examples of "heterocyclylene" include, but are not limited to, tetrahydrofuran-2,5-diyl, morpholine-2,3-diyl, pyran-2,4-diyl, 1,4-dioxane-2,3-diyl, 1,3-dioxane-2,4-diyl, piperidine-2,4-diyl, piperidine-1,4-diyl, pyrrolidine-1,3-diyl, morpholine-2,4-diyl, piperazine-1,4-dyil, and the like.

The term "alkoxy" as used herein, alone or in combination, refers to the monovalent radical R^{a}O-, where R^{a} is alkyl as defined above, for example C₍₁₋₈₎-alkyl giving C₍₁₋₈₎-alkoxy. Typical C₍₁₋₈₎-alkoxy groups include, but are not limited to, methoxy, ethoxy, n-propoxy, isopropoxy, butoxy, sec-butoxy, *tert*-butoxy, pentoxy, isopentoxy, hexoxy, isohexoxy and the like.

The term "alkylthio" as used herein, alone or in combination, refers to a straight or branched monovalent radical comprising an alkyl group as described above linked through a divalent sulphur atom having its free valence bond from the sulphur atom, for example C₁₋₈-alkylthio. Typical C₁₋₈-alkylthio groups include, but are not limited to, methylthio, ethylthio, propylthio, butylthio, pentylthio, hexylthio and the like.

The term "alkoxycarbonyl" as used herein refers to the monovalent radical R^{a}OC(O)-, where R^{a} is alkyl as described above, for example C₁₋₈-alkoxycarbonyl. Typical C₁₋₈-alkoxycarbonyl groups include, but are not limited to, methoxycarbonyl, ethoxycarbonyl, propoxycarbonyl, isopropoxycarbonyl, n-butoxycarbonyl, sec-butoxycarbonyl, tertbutoxycarbonyl, 3-methylbutoxycarbonyl, n-hexoxycarbonyl and the like.

The term "carbamoyl" as used herein refers to NH₂C(O)-.

The term "aryl" as used herein refers to a carbocyclic aromatic ring radical or to a aromatic ring system radical. Aryl is also intended to include the partially hydrogenated derivatives of the carbocyclic systems.

The term "heteroaryl", as used herein, alone or in combination, refers to a five to seven membered aromatic ring radical, or to a aromatic ring system radical, containing one or more heteroatoms selected from nitrogen, oxygen, or sulfur heteroatoms, wherein N-oxides and sulfur monoxides and sulfur dioxides are permissible heteroaromatic substitutions; such as e.g. furanyl, thienyl, thiophenyl, pyrrolyl, imidazolyl, pyrazolyl, triazolyl, tetrazolyl, thiazolyl, oxazolyl, isoxazolyl, oxadiazolyl, thiadiazolyl, isothiazolyl, pyridinyl, pyridazinyl, pyrazinyl, pyrimidinyl, quinolinyl, isoquinolinyl, benzofuranyl, benzothiophenyl, indolyl, and indazolyl, and the like. Heteroaryl is also intended to include the partially hydrogenated derivatives of the heterocyclic systems enumerated below.

Examples of "aryl" and "heteroaryl" includes, but are not limited to phenyl, biphenyl, indene, fluorene, naphthyl (1-naphthyl, 2-naphthyl), anthracene (1-anthracenyl, 2-anthracenyl, 3-anthracenyl), thiophene (2-thienyl, 3-thienyl), furyl (2-furyl, 3-furyl), indolyl, oxadiazolyl, isoxazolyl, thiadiazolyl, oxatriazolyl, thiatriazolyl, quinazolin, fluorenyl, xanthenyl, isoindanyl, benzhydryl, acridinyl, thiazolyl, pyrrolyl (1-pyrrolyl, 2-pyrrolyl, 3-pyrrolyl), pyrazolyl (1-pyrazolyl, 3-pyrazolyl, 4-pyrazolyl, 5-pyrazolyl), imidazolyl (1-imidazolyl, 2-imidazolyl, 4-imidazolyl, 5-imidazolyl), triazolyl (1,2,3-triazol-1-yl, 1,2,3-triazol-4-yl 1,2,3-triazol-5-yl, 1,2,4-triazol-3-yl, 1,2,4-triazol-5-yl), oxazolyl (2-oxazolyl, 4-oxazolyl, 5-oxazolyl), isooxazolyl (isooxazo-3-yl, isooxazo-4-yl, isooxaz-5-yl), isothiazolyl (isothiazo-3-yl, isothiazo-4-yl, isothiaz-5-yl) thiazolyl (2-thiazolyl, 4-thiazolyl, 5-thiazolyl), pyridyl (2-pyridyl, 3-pyridyl, 4-pyridyl), pyrimidinyl (2-pyrimidinyl, 4-pyrimidinyl, 5-pyrimidinyl, 6-pyrimidinyl), pyrazinyl, pyridazinyl (3- pyridazinyl, 4-pyridazinyl, 5-pyridazinyl), quinolyl (2-quinolyl, 3-quinolyl, 4-quinolyl, 5-quinolyl, 6-quinolyl, 7-quinolyl, 8-quinolyl), isoquinolyl (1-isoquinolyl, 3-isoquinolyl, 4-isoquinolyl, 5-isoquinolyl, 6-isoquinolyl, 7-isoquinolyl, 8-isoquinolyl), benzo[b]furanyl (2-benzo[b]furanyl, 3-benzo[b]furanyl, 4-benzo[b]furanyl, 5-benzo[b]furanyl, 6-benzo[b]furanyl, 7-benzo[b]furanyl), 2,3-dihydro-benzo[b]furanyl (2-(2,3-dihydro-benzo[b]furanyl), 3-(2,3-dihydro-benzo[b]furanyl), 4-(2,3-dihydro-benzo[b]furanyl), 5-(2,3-dihydro-benzo[b]furanyl), 6-(2,3-dihydro-benzo[b]furanyl), 7-(2,3-dihydro-benzo[b]furanyl)), benzo[b]thiophenyl (benzo[b]thiophen-2-yl, benzo[b]thiophen-3-yl, benzo[b]thiophen-4-yl, benzo[b]thiophen-5-yl, benzo[b]thiophen-6-yl, benzo[b]thlophen-7-yl), 2,3-dihydro-benzo[b]thiophenyl (2,3-dihydro-benzo[b]thiophen-2-yl, 2,3-dihydro-benzo[b]thiophen-3-yl, 2,3-dihydro-benzo[b]thiophen-4-yl, 2,3-dihydro-benzo[b]thiophen-5-yl, 2,3-dihydro-benzo[b]thiophen-6-yl, 2,3-dihydro-benzo[b]thiophen-7-yl), indolyl (1-indolyl, 2-indolyl, 3-indolyl, 4-indolyl, 5-indolyl, 6-indolyl, 7-indolyl), indazole (1-indazolyl, 3-indazolyl, 4-indazolyl, 5-indazolyl, 6-indazolyl, 7-indazolyl), benzimidazolyl (1-benzimidazolyl, 2-benzimidazolyl, 4-benzimidazolyl, 5-benzimidazolyl, 6-benzimidazolyl, 7-benzimidazolyl, 8-benzimidazolyl), benzoxazolyl (2-benzoxazolyl, 3-benzoxazolyl, 4-benzoxazolyl, 5-benzoxazolyl, 6-benzoxazolyl, 7-benzoxazolyl), benzothiazolyl (2-benzothiazolyl, 4-benzothiazolyl, 5-benzothiazolyl, 6-benzothiazolyl, 7-benzothiazolyl), carbazolyl (1-carbazolyl, 2-carbazolyl, 3-carbazolyl, 4-carbazolyl), 5H-dibenz[b,f]azepine (5H-dibenz[b,f]azepin-1-yl, 5H-dibenz[b,f]azepine-2-yl, 5H-dibenz[b,f]azepine-3-yl, 5H-dibenz[b,f]azepine-4-yl, 5H-dibenz[b,f]azepine-5-yl), 10,11-dihydro-5H-dibenz[b,f]azepine (10,11-dihydro-5H-dibenz[b,f]azepine-1-yl, 10,11-dihydro-5H-dibenz[b,f]azepine-2-yl, 10,11-dihydro-5H-dibenz[b,f]azepine-3-yl, 10,11 -dihydro-5H-dibenz[b,f]azepine-4-yl, 10,11-dihydro-5H-dibenz[b,f]azepine-5-yl), benzo[1,3]dioxole (2-benzo[1,3]dioxole, 4-benzo[1,3]dioxole, 5-benzo[1,3]dioxole, 6-benzo[1,3]dioxole, 7-benzo[1,3]dioxole), and tetrazolyl (5-tetrazolyl, N-tetrazolyl).

The present invention also relates to partly or fully saturated analogues of the ring systems mentioned above.

As used herein, the term "fused arylheterocyclyl" refers to an aryl group fused to a heterocyclyl group, the two having two atoms in common, and wherein the aryl group is the point of substitution. Examples of " fused arylheterocyclyl " used herein include 4-(2,3-benzo-dioxin), 3,4-methylenedioxy-1-phenyl, and the like.

As used herein, the term "fused heterocyclylaryl" refers to a heterocyclyl group fused to an aryl group, the two having two atoms in common, and wherein the heterocyclyl group is the point of substitution. Examples of " fused heterocyclylaryl" used herein include 2-(1,3-benzodioxole and the like.

As used herein, the term "fused heteroarylheterocyclyl " refers to a heteroaryl group fused to an heterocyclyl group, the two having two atoms in common, and wherein the heteroaryl group is the point of substitution. Examples of "fused heteroarylheterocyclyl" used herein include 1,2,3,4-tetrahydro-beta-carboline and the like.

As used herein, the term "fused heterocyclylheteroaryl" refers to a heterocyclyl group fused to an heteroaryl group, the two having two atoms in common, and wherein the heterocyclyl group is the point of substitution. Examples of "fused heterocyclylheteroaryl" used herein include 2-[1,3]-dioxolo[4,5-c]pyridine and the like.

As used herein, the term "fused arylcycloalkyl" refers to a aryl group fused to a cycloalkyl group, the two having two atoms in common, and wherein the aryl group is the point of substitution. Examples of "fused cycloalkylaryl" used herein include 5-indanyl, 6-(1,2,3,4-tetrahydronaphthyl), and the like.

As used herein, the term "fused cycloalkylaryl" refers to a cycloalkyl group fused to an aryl group, the two having two atoms in common, and wherein the cycloalkyl group is the point of substitution Examples of "fused cycloalkylaryl" used herein include 1-indanyl, 2-indanyl, 1-(1,2,3,4-tetrahydronaphthyl), and the like.

As used herein, the term "fused heteroarylcycloalkyl" refers to a heteroaryl group fused to an cycloalkyl group, the two having two atoms in common, and wherein the heteroaryl group is the point of substitution. Examples of "fused heteroarylcycloalkyl" used herein include 5-aza-6-indanyl and the like.

As used herein, the term "fused cycloakylheteroaryl" refers to a cycloalkyl group fused to an heteroaryl group, the two having two atoms in common, and wherein the cycloalkyl group is the point of substitution. Examples of "fused cycloalkylheteroaryl" used herein include 5-aza-1-indanyl and the like.

The term "arylene", as used herein, alone or in combination, refers to carbocyclic aromatic ring diradical or to a aromatic ring system diradical. Examples of "arylene" include, but are not limited to, benzene-1,4-diyl, naphthalene-1,8-diyl, and the like.

The term "heteroarylene", as used herein, alone or in combination, refers to a five to seven membered aromatic ring diradical, or to a aromatic ring system diradical, containing one or more heteroatoms selected from nitrogen, oxygen, or sulfur heteroatoms, wherein N-oxides and sulfur monoxides and sulfur dioxides are permissible heteroaromatic substitutions. Examples of "heteroarylene" used herein are furan-2,5-diyl, thiophene-2,4-diyl, 1,3,4-oxadiazole-2,5-diyl, 1,3,4-thiadiazole-2,5-diyl, 1,3-thiazole-2,4-diyl, 1,3-thiazole-2,5-diyl, pyridine-2,4-diyl, pyridine-2,3-diyl, pyridine-2,5-diyl, pyrimidine-2,4-diyl, quinoline-2,3-diyl, and the like.

The term "alkylsulfanyl", as used herein, refers to the group R^{a}S-, where R^{a} is alkyl as described above.

The term "alkylsulfenyl", as used herein, refers to the group R^{a}S(O)-, where R^{a} is alkyl as described above.

The term "alkylsulfonyl", as used herein, refers to the group R^{a}SO₂-, where R^{a} is alkyl as described above.

The term "acyl", as used herein, refers to the group R^{a}C(O)- , where R^{a} is alkyl, alkenyl, alkynyl, cycloalkyl, cycloalkenyl, or heterocyclyl as described above.

The term "aroyl", as used herein, refers to the group R^{a}C(O)- , where R^{a} is aryl as described above.

The term "heteroaroyl", as used herein, refers to the group RₐC(O)-, where R^{a} is heteroaryl as described above.

The term "acyloxy", as used herein, refers to the group R^{a}C(O)O- , where R^{a} is alkyl, alkenyl, alkynyl, cycloalkyl, cycloalkenyl, or heterocyclyl as described above.

The term "aroyloxy", as used herein, refers to the group R^{a}C(O)O- , where R^{a} is aryl as described above.

The term "heteroaroyloxy", as used herein, refers to the group R^{a}C(O)O- , where R^{a} is heteroaryl as described above.

Whenever the terms "alkyl" or "aryl" or either of their prefix roots appear in a name of a substituent (e.g. arylalkoxyaryloxy) they shall be interpreted as including those limitations given above for "alkyl" and "aryl".

As used herein, the term "oxo" shall refer to the substituent =O.

As used herein, the term "mercapto" shall refer to the substituent -SH.

As used herein, the term "carboxy" shall refer to the substituent -COOH.

As used herein, the term "cyano" shall refer to the substituent -CN.

As used herein, the term "aminosulfonyl" shall refer to the substituent -SO₂NH₂.

As used herein, the term "sulfanyl" shall refer to the substituent -S-.

As used herein, the term "sulfenyl" shall refer to the substituent -S(O)-.

As used herein, the term "sulfonyl" shall refer to the substituent -S(O)₂-.

As used herein, the term "direct bond", where part of a structural variable specification, refers to the direct joining of the substituents flanking (preceding and succeeding) the variable taken as a "direct bond".

The term "lower", as used herein, refers to an group having between one and six carbons, and may be indicated with the prefix Cₓ₋₆-. Lower alkyl may thus be indicated as C₁₋₆-alkyl, while lower alkylene may be indicated as C₂₋₆-alkylene.

A radical such as C_{x-y}-cycloalkyl-C_{a-b}-alkenyl shall designate that the radical's point of attachment is in part of the radical mentioned last.

As used herein, the term "optionally" means that the subsequently described event(s) may or may not occur, and includes both event(s) which occur and events that do not occur.

As used herein, the term "substituted" refers to substitution with the named substituent or substituents, multiple degrees of substitution being allowed unless otherwise stated.

As used herein, the terms "contain" or "containing" can refer to in-line substitutions at any position along the above defined alkyl, alkenyl, alkynyl or cycloalkyl substituents with one or more of any of O, S, SO, SO₂, N, or N-alkyl, including, for example, -CH₂-O-CH₂-, -CH₂-SO₂-CH₂-, -CH₂-NH-CH₃ and so forth.

Certain of the above defined terms may occur more than once in the structural formulae, and upon such occurrence each term shall be defined independently of the other.

As used herein, the term "solvate" is a complex of variable stoichiometry formed by a solute (in this invention, a compound of Formula (I)) and a solvent. Such solvents for the purpose of the invention may not interfere with the biological activity of the solute. Solvents may be, by way of example, water, ethanol, or acetic acid.

As used herein, the term "biohydrolyzable ester" is an ester of a drug substance (in this invention, a compound of Formula (I) ) which either a) does not interfere with the biological activity of the parent substance but confers on that substance advantageous properties *in vivo* such as duration of action, onset of action, and the like, or b) is biologically inactive but is readily converted in vivo by the subject to the biologically active principle. The advantage is that, for example, the biohydrolyzable ester is orally absorbed from the gut and is transformed to (I) in plasma. Many examples of such are known in the art and include by way of example lower alkyl esters (e.g., C₁-C₄), lower acyloxyalkyl esters, lower alkoxyacyloxyalkyl esters, alkoxyacyloxy esters, alkyl acylamino alkyl esters, and choline esters.

As used herein, the term "biohydrolyzable amide" is an amide of a drug substance (in this invention, a compound of general Formula (I)) which either a) does not interfere with the biological activity of the parent substance but confers on that substance advantageous properties in vivo such as duration of action, onset of action, and the like, or b) is biologically inactive but is readily converted in vivo by the subject to the biologically active principle. The advantage is that, for example, the biohydrolyzable amide is orally absorbed from the gut and is transformed to (I) in plasma. Many examples of such are known in the art and include by way of example lower alkyl amides, α-amino acid amides, alkoxyacyl amides, and alkylaminoalkylcarbonyl amides.

As used herein, the term "prodrug" includes biohydrolyzable amides and biohydrolyzable esters and also encompasses a) compounds in which the biohydrolyzable functionality in such a prodrug is encompassed in the compound of Formula (I): for example, the lactam formed by a carboxylic group in R² and an amine in R⁴, and b) compounds which may be oxidized or reduced biologically at a given functional group to yield drug substances of Formula (I). Examples of these functional groups include, but are not limited to, 1,4-dihydropyridine, N-alkylcarbonyl-1,4-dihydropyridine, 1,4-cyclohexadiene, tert-butyl, and the like.

The term "pharmacologically effective amount" or shall mean that amount of a drug or pharmaceutical agent that will elicit the biological or medical response of a tissue, animal or human that is being sought by a researcher or clinician. This amount can be a therapeutically effective amount. The term "therapeutically effective amount" shall mean that amount of a drug or pharmaceutical agent that will elicit the therapeutic response of an animal or human that is being sought.

The term "treatment" and "treating" as used herein means the management and care of a patient for the purpose of combating a disease, disorder or condition. The term is intended to include the full spectrum of treatments for a given disorder from which the patient is suffering, such as the delaying of the progression of the disease, disorder or condition, the alleviation or relief of symptoms and complications, and/or the cure or elimination of the disease, disorder or condition. The patient to be treated is preferably a mammal, in particular a human being.

The present compounds are activators of glucokinase and are as such useful for the activation of glucokinase.

Accordingly, the present invention provides a method for activating glucokinase in a patient in need thereof, which method comprises administering to a subject in need thereof a compound of the present invention, preferably a compound of formula (I), (II), (III), or (IV), preferably a pharmacologically effective amount, more preferably a therapeutically effective amount. The present invention also provides a method for lowering blood glucose in a patient in need thereof, which method comprises administering to a subject in need thereof a compound of the present invention, preferably a compound of formula (I), (II), (III), or (IV), preferably a pharmacologically effective amount, more preferably a therapeutically effective amount. The present invention also provides a method for prevention and/or treatment of glucokinase deficiency-mediated human diseases, the method comprising administration to a human in need thereof a therapeutically effective amount of a compound of the present invention, preferably a compound of formula (I), (II), (III), or (IV). As used herein, the phrase "a subject in need thereof" includes mammalian subjects, preferably humans, who either suffer from one or more of the aforesaid diseases or disease states or are at risk for such. Accordingly, in the context of the therapeutic method of the invention, this method also is comprised of a method for treating a mammalian subject prophylactically, or prior to the onset of diagnosis such disease(s) or disease state(s). Other embodiments of such methods will be clear from the following description.

Compounds according to the present invention are useful for the treatment of disorders, diseases and conditions, wherein the activation of glucokinase is beneficial.

Accordingly, the present compounds are useful for the treatment of hyperglycemia, IGT (impaired glucose tolerance), insulin resistance syndrome, syndrome X, type 1 diabetes, type 2 diabetes, dyslipidemia, dyslipoproteinemia (abnormal lipoproteins in the blood) including diabetic dyslipidemia, hyperlipidemia, hyperlipoproteinemia (excess of lipoproteins in the blood) including type I, II-a (hypercholesterolemia), II-b, III, IV (hypertriglyceridemia) and V (hypertriglyceridemia) hyperlipoproteinemias, and obesity. Furthermore, they may be useful for the treatment of albuminuria, cardiovascular diseases such as cardiac hypertrophy, hypertension and arteriosclerosis including atherosclerosis; gastrointestinal disorders; acute pancreatitis; and appetite regulation or energy expenditure disorders.

Accordingly, in a further aspect the invention relates to a compound according to the present invention for use as a medicament.

The invention also relates to pharmaceutical compositions comprising, as an active ingredient, at least one compound of the present invention together with one or more pharmaceutically acceptable carriers or excipients.

The pharmaceutical composition may be in unit dosage form, comprising from about 0.05 mg to about 1000 mg, preferably from about 0.1 mg to about 500 mg and especially preferred from about 0.5 mg to about 200 mg of the compound of the present invention, such as for instance a compound with formula (I), (II), (III), or (IV).

A pharmaceutical composition according to the present invention may comprise one or more further antidiabetic agents, for instance insulin or an insulin analogue, a sulphonylurea, a biguanide, a meglitinide, an insulin sensitizer, for instance a thiazolidinedione insulin sensitizer, an α-glucosidase inhibitor, a glycogen phosphorylase inhibitor, and an agent acting on the ATP-dependent potassium channel of the pancreatic β-cells, or any combination thereof.

A pharmaceutical composition according to the present invention may also comprise (for instance in addition to a further antidiabetic agent) a further antihyperlipidemic agent, such as a antihyperlipidemic agent selected from the group consisting of cholestyramine, colestipol, clofibrate, gemfibrozil, lovastatin, pravastatin, simvastatin, probucol and dextrothyroxine, a further antiobesity or appetite regulating agent, or a further antihypertensive agent or any combination thereo

In one embodiment of the invention, the present compounds are used for the preparation of a medicament for the treatment of hyperglycemia. As used herein hyperglycemia is to be taken as generally understood in the art, with reference for example to the Report of the Expert Committee of the Diagnosis and Classification of Diabetes Mellitus, published in Diabetes Care 20, 1183-1197, (1997), but is usually taken to mean an elevated plasma glucose level exceeding about 110 mg/dl. The present compounds are effective in lowering the blood glucose both in the fasting and postprandial stage.

In one embodiment of the invention, the present compounds are used for the preparation of a pharmaceutical composition for the treatment of IGT.

In one embodiment of the invention, the present compounds are used for the preparation of a pharmaceutical composition for the treatment of Syndrome X.

In one embodiment of the invention, the present compounds are used for the preparation of a pharmaceutical composition for the treatment of type 2 diabetes. Such treatment includes ia treatment for the purpose of the delaying of the progression from IGT to type 2 diabetes as well as delaying the progression from non-insulin requiring type 2 diabetes to insulin requiring type 2 diabetes.

In one embodiment of the invention the present compounds are used for the preparation of a pharmaceutical composition for the treatment of type 1 diabetes. Such therapy is normally accompanied by insulin administration.

In one embodiment of the invention the present compounds are used for the preparation of a pharmaceutical composition for the treatment of dyslipidemia and hyperlipidemia.

In one embodiment of the invention the present compounds are used for the preparation of a pharmaceutical composition for the treatment of obesity.

In another aspect of the invention treatment of a patient with the present compounds are combined with diet and/or exercise.

The present invention provides methods of activating glucokinase activity in a mammal, which methods comprise administering, to a mammal in need of activation of glucokinase activity, a therapeutically defined amount of a compound of the present invention, such as a compound of formula (I), (II), (III), or (IV), defined above, as a single or polymorphic crystalline form or forms, an amorphous form, a single enantiomer, a racemic mixture, a single stereoisomer, a mixture of stereoisomers, a single diastereoisomer, a mixture of diastereoisomers, a solvate, a pharmaceutically acceptable salt, a solvate, a prodrug, a biohydrolyzable ester, or a biohydrolyzable amide thereof.

The present invention provides a method of activating glucokinase, comprising the step of administering to a mammal in need thereof a pharmacologically effective amount of a compound of the present invention. The invention further provides a pharmaceutical composition comprising a pharmaceutically acceptable carrier and a pharmacologically effective amount of a compound of the present invention sufficient to activate glucokinase. A glucokinase - activating amount can be an amount that reduces or inhibits a PTPase activity in the subject.

Additionally provided by the present invention is a pharmaceutical composition comprising a pharmaceutically acceptable carrier and a pharmacologically effective amount of a compound of the present invention sufficient to treat type I diabetes.

Also provided by the present invention is a pharmaceutical composition comprising a pharmaceutically acceptable carrier and a pharmacologically effective amount of a compound of the present invention sufficient to treat type II diabetes.

The compounds of the present invention can be administered to any mammal in need of activation of glucokinase activity. Such mammals can include, for example, horses, cows, sheep, pigs, mice, dogs, cats, primates such as chimpanzees, gorillas, rhesus monkeys, and, most preferably humans.

In a further aspect of the invention the present compounds are administered in combination with one or more further active substances in any suitable ratios. Such further active agents may be selected from antidiabetic agents, antihyperlipidemic agents, antiobesity agents, antihypertensive agents and agents for the treatment of complications resulting from or associated with diabetes.

Suitable antidiabetic agents include insulin, GLP-1 (glucagon like peptide-1) derivatives such as those disclosed in WO 98/08871 (Novo Nordisk A/S), which is incorporated herein by reference, as well as orally active hypoglycemic agents.

Suitable orally active hypoglycemic agents preferably include imidazolines, sulfonylureas, biguanides, meglitinides, oxadiazolidinediones, thiazolidinediones, insulin sensitizers, α-glucosidase inhibitors, agents acting on the ATP-dependent potassium channel of the pancreatic β-cells eg potassium channel openers such as those disclosed in WO 97/26265, WO 99/03861 and WO 00/37474 (Novo Nordisk A/S) which are incorporated herein by reference, potassium channel openers, such as ormitiglinide, potassium channel blockers such as nateglinide or BTS-67582, glucagon antagonists such as those disclosed in WO 99/01423 and WO 00/39088 (Novo Nordisk A/S and Agouron Pharmaceuticals, Inc.), all of which are incorporated herein by reference, GLP-1 agonists such as those disclosed in WO 00/42026 (Novo Nordisk A/S and Agouron Pharmaceuticals, Inc.), which are incorporated herein by reference, DPP-IV (dipeptidyl peptidase-IV) inhibitors, PTPase (protein tyrosine phosphatase) inhibitors, inhibitors of hepatic enzymes involved in stimulation of gluconeogenesis and/or glycogenolysis, glucose uptake modulators, GSK-3 (glycogen synthase kinase-3) inhibitors, compounds modifying the lipid metabolism such as antihyperlipidemic agents and antilipidemic agents, compounds lowering food intake, and PPAR (peroxisome proliferator-activated receptor) and RXR (retinoid X receptor) agonists such as ALRT-268, LG-1268 or LG-1069.

In one embodiment of the invention, the present compounds are administered in combination with insulin or insulin analogues.

In one embodiment of the invention, the present compounds are administered in combination with a sulphonylurea eg tolbutamide, chlorpropamide, tolazamide, glibenclamide, glipizide, glimepiride, glicazide or glyburide.

In one embodiment of the invention, the present compounds are administered in combination with a biguanide eg metformin.

In one embodiment of the invention, the present compounds are administered in combination with a meglitinide eg repaglinide or senaglinide/nateglinide.

In one embodiment of the invention, the present compounds are administered in combination with a thiazolidinedione insulin sensitizer eg troglitazone, ciglitazone, pioglitazone, rosiglitazone, isaglitazone, darglitazone, englitazone, CS-011/Cl-1037 or T 174 or the compounds disclosed in WO 97/41097 (DRF-2344), WO 97/41119, WO 97/41120, WO 00/41121 and WO 98/45292 (Dr. Reddy's Research Foundation), which are incorporated herein by reference.

In one embodiment of the invention the present compounds may be administered in combination with an insulin sensitizer eg such as GI 262570, YM-440, MCC-555, JTT-501, AR-H039242, KRP-297, GW-409544, CRE-16336, AR-H049020, LY510929, MBX-102, CLX-0940, GW-501516 or the compounds disclosed in WO 99/19313 (NN622/DRF-2725), WO 00/50414, WO 00/63191, WO 00/63192, WO 00/63193 (Dr. Reddy's Research Foundation) and WO 00/23425, WO 00/23415, WO 00/23451, WO 00/23445, WO 00/23417, WO 00/23416, WO 00/63153, WO 00/63196, WO 00/63209, WO 00/63190 and WO 00/63189 (Novo Nordisk A/S), which are incorporated herein by reference.

In one embodiment of the invention the present compounds are administered in combination with an α-glucosidase inhibitor eg voglibose, emiglitate, miglitol or acarbose.

In one embodiment of the invention the present compounds are administered in combination with a glycogen phosphorylase inhibitor eg the compounds described in WO 97/09040 (Novo Nordisk A/S).

In one embodiment of the invention the present compounds are administered in combination with an agent acting on the ATP-dependent potassium channel of the pancreatic β-cells eg tolbutamide, glibenclamide, glipizide, glicazide, BTS-67582 or repaglinide.

In one embodiment of the invention the present compounds are administered in combination with nateglinide.

In one embodiment of the invention the present compounds are administered in combination with an antihyperlipidemic agent or a antilipidemic agent eg cholestyramine, colestipol, clofibrate, gemfibrozil, lovastatin, pravastatin, simvastatin, probucol or dextrothyroxine.

In another aspect of the invention, the present compounds are administered in combination with more than one of the above-mentioned compounds eg in combination with metformin and a sulphonylurea such as glyburide; a sulphonylurea and acarbose; nateglinide and metformin; acarbose and metformin; a sulfonylurea, metformin and troglitazone; insulin and a sulfonylurea; insulin and metformin; insulin, metformin and a sulfonylurea; insulin and troglitazone; insulin and lovastatin; etc.

Furthermore, the compounds according to the invention may be administered in combination with one or more antiobesity agents or appetite regulating agents.

Such agents may be selected from the group consisting of CART (cocaine amphetamine regulated transcript) agonists, NPY (neuropeptide Y) antagonists, MC3 (melanocortin 3) agonists, MC4 (melanocortin 4) agonists, orexin antagonists, TNF (tumor necrosis factor) agonists, CRF (corticotropin releasing factor) agonists, CRF BP (corticotropin releasing factor binding protein) antagonists, urocortin agonists, β3 adrenergic agonists such as CL-316243, AJ-9677, GW-0604, LY362884, LY377267 or AZ-40140, MSH (melanocyte-stimulating hormone) agonists, MCH (melanocyte-concentrating hormone) antagonists, CCK (cholecystokinin) agonists, serotonin reuptake inhibitors (fluoxetine, seroxat or citalopram), serotonin and norepinephrine reuptake inhibitors, 5HT (serotonin) agonists, bombesin agonists, galanin antagonists, growth hormone, growth factors such as prolactin or placental lactogen, growth hormone releasing compounds, TRH (thyreotropin releasing hormone) agonists, UCP 2 or 3 (uncoupling protein 2 or 3) modulators, leptin agonists, DA (dopamine) agonists (bromocriptin, doprexin), lipase/amylase inhibitors, PPAR modulators, RXR modulators, TR β agonists, adrenergic CNS stimulating agents, AGRP (agouti related protein) inhibitors, H3 histamine antagonists such as those disclosed in WO 00/42023, WO 00/63208 and WO 00/64884, which are incorporated herein by reference, exendin-4, GLP-1 agonists and ciliary neurotrophic factor. Further antiobesity agents are bupropion (antidepressant), topiramate (anticonvulsant), ecopipam (dopamine D1/D5 antagonist) and naltrexone (opioid antagonist).

In one embodiment of the invention the antiobesity agent is leptin.

In one embodiment of the invention the antiobesity agent is a serotonin and norepinephrine reuptake inhibitor eg sibutramine.

In one embodiment of the invention the antiobesity agent is a lipase inhibitor eg orlistat.

In one embodiment of the invention the antiobesity agent is an adrenergic CNS stimulating agent eg dexamphetamine, amphetamine, phentermine, mazindol phendimetrazine, diethylpropion, fenfluramine or dexfenfluramine.

Furthermore, the present compounds may be administered in combination with one or more antihypertensive agents. Examples of antihypertensive agents are β-blockers such as alprenolol, atenolol, timolol, pindolol, propranolol and metoprolol, ACE (angiotensin converting enzyme) inhibitors such as benazepril, captopril, enalapril, fosinopril, lisinopril, quinapril and ramipril, calcium channel blockers such as nifedipine, felodipine, nicardipine, isradipine, nimodipine, diltiazem and verapamil, and α-blockers such as doxazosin, urapidil, prazosin and terazosin. Further reference can be made to Remington: The Science and Practice of Pharmacy, 19th Edition, Gennaro, Ed., Mack Publishing Co., Easton, PA, 1995.

It should be understood that any suitable combination of the compounds according to the invention with diet and/or exercise, one or more of the above-mentioned compounds and optionally one or more other active substances are considered to be within the scope of the present invention.

### PHARMACEUTICAL COMPOSITIONS

The compounds of the invention may be administered alone or in combination with pharmaceutically acceptable carriers or excipients, in either single or multiple doses. The pharmaceutical compositions according to the invention may be formulated with pharmaceutically acceptable carriers or diluents as well as any other known adjuvants and excipients in accordance with conventional techniques such as those disclosed in Remington: The Science and Practice of Pharmacy, 19^{th} Edition, Gennaro, Ed., Mack Publishing Co., Easton, PA, 1995.

The pharmaceutical compositions may be specifically formulated for administration by any suitable route such as the oral, rectal, nasal, pulmonary, topical (including buccal and sublingual), transdermal, intracisternal, intraperitoneal, vaginal and parenteral (including subcutaneous, intramuscular, intrathecal, intravenous and intradermal) route, the oral route being preferred. It will be appreciated that the preferred route will depend on the general condition and age of the subject to be treated, the nature of the condition to be treated and the active ingredient chosen.

Pharmaceutical compositions for oral administration include solid dosage forms such as hard or soft capsules, tablets, troches, dragees, pills, lozenges, powders and granules. Where appropriate, they can be prepared with coatings such as enteric coatings or they can be formulated so as to provide controlled release of the active ingredient such as sustained or prolonged release according to methods well known in the art.

Liquid dosage forms for oral administration include solutions, emulsions, aqueous or oily suspensions, syrups and elixirs.

Pharmaceutical compositions for parenteral administration include sterile aqueous and non-aqueous injectable solutions, dispersions, suspensions or emulsions as well as sterile powders to be reconstituted in sterile injectable solutions or dispersions prior to use. Depot injectable formulations are also contemplated as being within the scope of the present invention.

Other suitable administration forms include suppositories, sprays, ointments, cremes, gels, inhalants, dermal patches, implants etc.

A typical oral dosage is in the range of from about 0.001 to about 100 mg/kg body weight per day, preferably from about 0.01 to about 50 mg/kg body weight per day, and more preferred from about 0.05 to about 10 mg/kg body weight per day administered in one or more dosages such as 1 to 3 dosages. The exact dosage will depend upon the frequency and mode of administration, the sex, age, weight and general condition of the subject treated, the nature and severity of the condition treated and any concomitant diseases to be treated and other factors evident to those skilled in the art.

The formulations may conveniently be presented in unit dosage form by methods known to those skilled in the art. A typical unit dosage form for oral administration one or more times per day such as 1 to 3 times per day may contain from 0.05 to about 1000 mg, preferably from about 0.1 to about 500 mg, and more preferred from about 0.5 mg to about 200 mg.

For parenteral routes such as intravenous, intrathecal, intramuscular and similar administration, typically doses are in the order of about half the dose employed for oral administration.

The compounds of this invention are generally utilized as the free substance or as a pharmaceutically acceptable salt thereof. Examples are an acid addition salt of a compound having the utility of a free base and a base addition salt of a compound having the utility of a free acid. The term "pharmaceutically acceptable salts" refers to non-toxic salts of the compounds of this invention which are generally prepared by reacting the free base with a suitable organic or inorganic acid or by reacting the acid with a suitable organic or inorganic base. When a compound of the present invention, such as a compound of formula (I), (II), (III), or (IV), contains a free base such salts are prepared in a conventional manner by treating a solution or suspension of the compound with a chemical equivalent of a pharmaceutically acceptable acid. When a compound of the present invention, such as a compound of formula (I), (II), (III), or (IV), contains a free acid such salts are prepared in a conventional manner by treating a solution or suspension of the compound with a chemical equivalent of a pharmaceutically acceptable base. Physiologically acceptable salts of a compound with a hydroxy group include the anion of said compound in combination with a suitable cation such as sodium or ammonium ion. Other salts which are not pharmaceutically acceptable may be useful in the preparation of compounds of the invention and these form a further aspect of the invention.

For parenteral administration, solutions of the novel compounds of the formula (I) in sterile aqueous solution, aqueous propylene glycol or sesame or peanut oil may be employed. Such aqueous solutions should be suitably buffered if necessary and the liquid diluent first rendered isotonic with sufficient saline or glucose. The aqueous solutions are particularly suitable for intravenous, intramuscular, subcutaneous and intraperitoneal administration. The sterile aqueous media employed are all readily available by standard techniques known to those skilled in the art.

Suitable pharmaceutical carriers include inert solid diluents or fillers, sterile aqueous solution and various organic solvents. Examples of solid carriers are lactose, terra alba, sucrose, cyclodextrin, talc, gelatine, agar, pectin, acacia, magnesium stearate, stearic acid and lower alkyl ethers of cellulose. Examples of liquid carriers are syrup, peanut oil, olive oil, phospholipids, fatty acids, fatty acid amines, polyoxyethylene and water. Similarly, the carrier or diluent may include any sustained release material known in the art, such as glyceryl monostearate or glyceryl distearate, alone or mixed with a wax. The pharmaceutical compositions formed by combining the novel compounds of the present invention and the pharmaceutically acceptable carriers are then readily administered in a variety of dosage forms suitable for the disclosed routes of administration. The formulations may conveniently be presented in unit dosage form by methods known in the art of pharmacy.

Formulations of the present invention suitable for oral administration may be presented as discrete units such as capsules or tablets, each containing a predetermined amount of the active ingredient, and which may include a suitable excipient. Furthermore, the orally available formulations may be in the form of a powder or granules, a solution or suspension in an aqueous or non-aqueous liquid, or an oil-in-water or water-in-oil liquid emulsion.

Compositions intended for oral use may be prepared according to any known method, and such compositions may contain one or more agents selected from the group consisting of sweetening agents, flavoring agents, coloring agents, and preserving agents in order to provide pharmaceutically elegant and palatable preparations. Tablets may contain the active ingredient in admixture with non-toxic pharmaceutically-acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example corn starch or alginic acid; binding agents, for example, starch, gelatin or acacia; and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the techniques described in U.S. Patent Nos. 4,356,108; 4,166,452; and 4,265,874, incorporated herein by reference, to form osmotic therapeutic tablets for controlled release.

Formulations for oral use may also be presented as hard gelatin capsules where the active ingredient is mixed with an inert solid diluent, for example, calcium carbonate, calcium phosphate or kaolin, or a soft gelatin capsules wherein the active ingredient is mixed with water or an oil medium, for example peanut oil, liquid paraffin, or olive oil.

Aqueous suspensions may contain the active compounds in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide such as lecithin, or condensation products of an alkylene oxide with fatty acids, for example polyoxyethylene stearate, or condensation products of ethylene oxide with long chain aliphatic alcohols, for example, heptadecaethyl-eneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more coloring agents, one or more flavoring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as a liquid paraffin. The oily suspensions may contain a thickening agent, for example beeswax, hard paraffin or cetyl alchol. Sweetening agents such as those set forth above, and flavoring agents may be added to provide a palatable oral preparation. These compositions may be preserved by the addition of an anti-oxidant such as ascorbic acid.

Dispersible powders and granules suitable for preparation of an aqueous suspension by the addition of water provide the active compound in admixture with a dispersing or wetting agent, suspending agent and one or more preservatives. Suitable dispersing or wetting agents and suspending agents are exemplified by those already mentioned above. Additional excipients, for example, sweetening, flavoring, and coloring agents may also be present.

The pharmaceutical compositions of the invention may also be in the form of oil-in-water emulsions. The oily phase may be a vegetable oil, for example, olive oil or arachis oil, or a mineral oil, for example a liquid paraffin, or a mixture thereof. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents. The pharmaceutical compositions may be in the form of a sterile injectible aqueous or oleaginous suspension. This suspension may be formulated according to the known methods using suitable dispersing or wetting agents and suspending agents described above. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution, and isotonic sodium chloride solution. In addition, sterile, fixed oils are conveniently employed as solvent or suspending medium. For this purpose, any bland fixed oil may be employed using synthetic mono- or diglycerides. In addition, fatty acids such as oleic acid find use in the preparation of injectables.

The compositions may also be in the form of suppositories for rectal administration of the compounds of the invention. These compositions can be prepared by mixing the drug with a suitable non-irritating excipient which is solid at ordinary temperatures but liquid at the rectal temperature and will thus melt in the rectum to release the drug. Such materials include cocoa butter and polyethylene glycols, for example.

For topical use, creams, ointments, jellies, solutions of suspensions, etc., containing the compounds of the invention are contemplated. For the purpose of this application, topical applications shall include mouth washes and gargles.

The compounds of the present invention may also be administered in the form of liposome delivery systems, such as small unilamellar vesicles, large unilamellar vesicles, and multilamellar vesicles. Liposomes may be formed from a variety of phospholipids, such as cholesterol, stearylamine, or phosphatidylcholines.

In addition, some of the compounds of the present invention may form solvates with water or common organic solvents. Such solvates are also encompassed within the scope of the invention.

Thus, in a further embodiment, there is provided a pharmaceutical composition comprising a compound of the present invention, or a pharmaceutically acceptable salt, solvate, or prodrug therof, and one or more pharmaceutically acceptable carriers, excipients, or diluents.

If a solid carrier is used for oral administration, the preparation may be tabletted, placed in a hard gelatine capsule in powder or pellet form or it can be in the form of a troche or lozenge. The amount of solid carrier will vary widely but will usually be from about 25 mg to about 1 g. If a liquid carrier is used, the preparation may be in the form of a syrup, emulsion, soft gelatine capsule or sterile injectable liquid such as an aqueous or non-aqueous liquid suspension or solution.

A typical tablet that may be prepared by conventional tabletting techniques may contain:

| Core: | |
|---|---|
| Active compound (as free compound or salt thereof) | 5.0 mg |
| Lactosum Ph. Eur. | 67.8 mg |
| Cellulose, microcryst. (Avicel) | 31.4 mg |
| Amberlite® IRP88* | 1.0 mg |
| Magnesii stearas Ph. Eur. | q.s. |

| Coating: | |
|---|---|
| Hydroxypropyl methylcellulose approx. | 9 mg |
| Mywacett 9-40 T** approx. | 0.9 mg |

| | |
|---|---|
| * Polacrillin potassium NF, tablet disintegrant, Rohm and Haas. | |
| ** Acylated monoglyceride used as plasticizer for film coating. | |

If desired, the pharmaceutical composition of the invention may comprise a compound according to the present invention in combination with further active substances such as those described in the foregoing.

The present invention also provides a method for the synthesis of compounds useful as intermediates in the preparation of compounds of Formula (I) along with methods for the preparation of compounds of Formula (I). The compounds can be prepared readily according to the following reaction Schemes (in which all variables are as defined before, unless so specified) using readily available starting materials, reagents and conventional synthesis procedures. In these reactions, it is also possible to make use of variants which are themselves known to those of ordinary skill in this art, but are not mentioned in greater detail.

### Abbreviations

Abbreviations used in the Schemes and Examples are as follows:
- d: = days
- g: = grams
- h: = hours
- Hz: = hertz
- kD: = kiloDalton
- L: = liters
- M: = molar
- mbar: = millibar
- mg: = milligrams
- min: = minutes
- mL: = milliliters
- mM: = millimolar
- mmol: = millimoles
- mol: = moles
- N: = normal
- ppm: = parts per million
- psi: = pounds per square inch
- APCI: = atmospheric pressure chemical ionization
- ESI: = electrospray ionization
- i.v.: = intravenous
- m/z: = mass to charge ratio
- mp: = melting point
- MS: = mass spectrometry
- NMR: = nuclear magnetic resonance spectroscopy
- p.o.: = per oral
- R_{f}: = relative TLC mobility
- rt: = room temperature
- s.c.: = subcutaneous
- TLC: = thin layer chromatography
- tᵣ: = retention time
- BOP: = (1-benzotriazolyloxy)tris(dimethylamino)phosphonium hexafluorophosphate
- DCM: = dichloromethane
- DIEA: = diisopropylethylamine
- DMF: = N, N-dimethylformamide
- DMPU: = 1,3-dimethypropylene urea
- DMSO: = dimethylsulfoxide
- EDC: =1-ethyl-3-(3-dimethylaminopropyl)-carbodiimide hydrochloride
- ether: = diethyl ether
- EtOAc: = ethyl acetate
- HMPA: = hexamethylphosphoric triamide
- HOBt: =1-hydroxybenzotriazole
- LAH: = lithium aluminum hydride
- LDA: = lithium diisopropylamide
- MeOH: = methanol
- NMM: = N-methylmorpholine, 4-methylmorpholine
- TEA: = triethylamine
- TFA: = trifluoroacetic acid
- THF: = tetrahydrofuran
- THP: = tetrahydropyranyl
- TTF: = Fluoro-N,N,N'-tetramethylformamidinium hexafluorophosphate

### Reaction schemes

Unless otherwise specified, the variables in the Schemes are as defined for Formula (I).

Scheme 1 describes the preparation of compounds of formula (I). 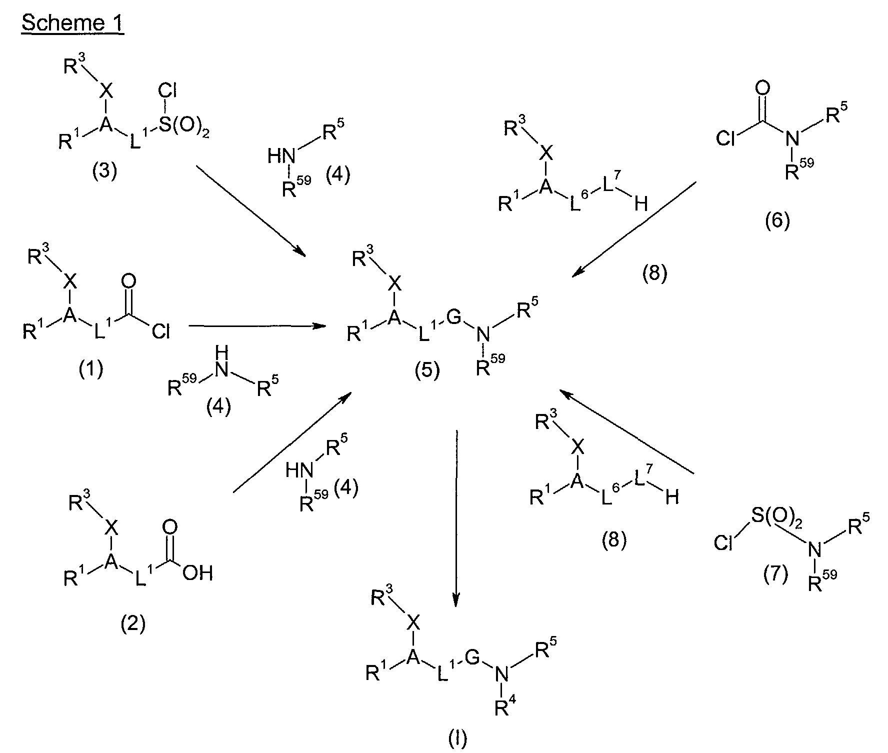 L⁶ is -(CH₂)ₙ-C(R⁹)(R¹⁰)ₘ- , wherein n, m, R⁹, and R¹⁰ are as defined for Formula (I).

The sulfonyl chloride (3) may be treated with the amine (4) in the presence of a tertiary amine base such as TEA to afford (5) where G is S(O)₂. R⁵⁹ may be R⁴, or R⁵⁹ may be a linkage to a polymer support such as Wang Resin. Treatment of such a polymer-supported (5) with TFA in a suitable solvent such as dichloromethane affords (I) where R⁴ is H. (1) may be treated with the amine (4) to afford (5), and subsequently (I), in like manner. The acid (2) may be activated by treatment with a carbodiimide reagent such as EDC, or with a coupling agent such as TFFH, in a solvent such as DCM or DMF, in the presence of (4) to afford (5). (5) may be converted in like manner to (I). The carbamyl chloride (6) may be prepared by treatment of (4) with a reagent such as phosgene, diphosgene, or triphosgene in a solvent such as DCM in the presence of a tertiary amine base such as TEA. Treatment of (8) with (6) in the presence of a tertiary amine base such as TEA affords (5), and in like manner, (I). The chlorosulfonamide (7) may be prepared by treatment of (4) with sulfuryl chloride in the presence of a tertiary amine base such as TEA or DIEA. (7) may be treated with (8) in a suitable solvent such as DCM in the presence of a tertiary amine base such as TEA or DIEA to afford 5, and, in like manner to the previous, (I).

Scheme 2 describes the preparation of the compound of formula (2).

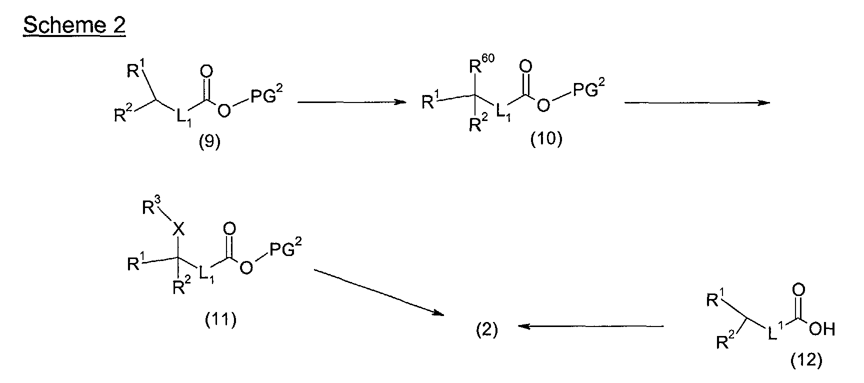

The ester (9), where PG² is a carboxy protecting group, may be treated with N-bromosuccinimide in the presence of benzoyl peroxide to afford (10) where R⁶⁰ is bromide. Typically this procedure is preferable where R¹ is aryl or heteroaryl. (10) where R⁶⁰ is bromide may be treated with a reagent R³-SH, R³-N(R⁶)H, or R³-OH and a base such as sodium hydride or potassium tert-butoxide to afford (11) where X is S, N(R⁶), or O, respectively. (11) may be deprotected with, for example, aqueous alkali where PG² is methyl or ethyl, to afford (2). Where L¹ is a direct bond, treatment of (9) with base such as LDA and an oxidizing agent such as a sulfonyl-oxaziridine reagent affords (10) where R⁶⁰ is OH. Treatment of such with a reagent R³-LG¹, where LG¹ is a nucleofugal group such as Br, Cl, I, or sulfonate, and a base such as DBU or sodium hydride affords (11). Further, treatment of (12) with two equivalents of strong base such as LDA and a reagent R³-S-LG², where LG² is an arylsulfinate group or halogen, affords (2) where X is S.

Scheme 3 describes an alternate preparation of a compund of formula (2).

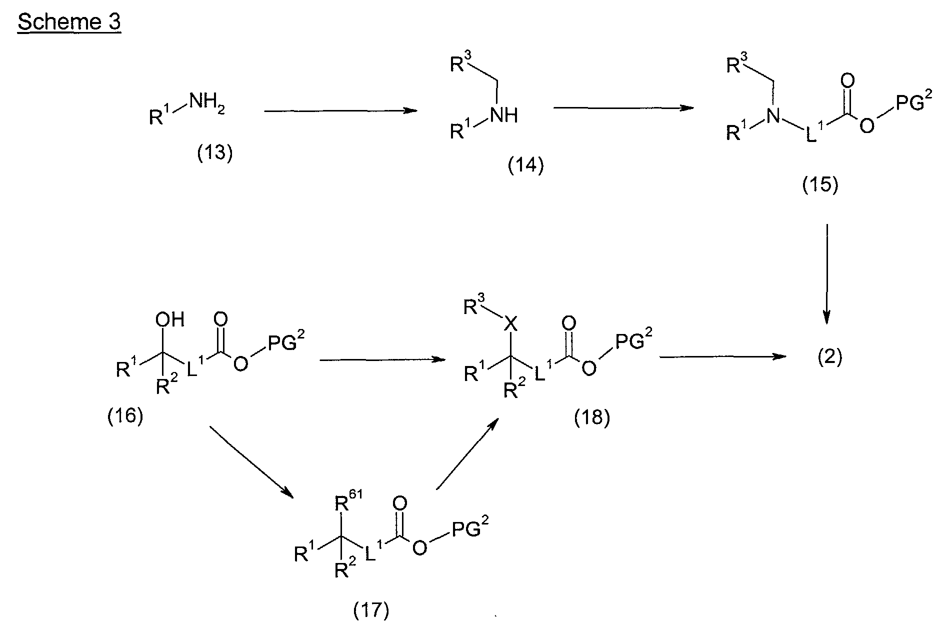

An amine (13) may be alkylated with an alkyl halide R³-CH₂-LG¹, where LG¹ is a nucleofugal group such as tosylate or bromide or iodide, in the presence of a base such as potassium carbonate in a solvent such as DMF to afford (14). Alternately, (13) may be treated with an aldehyde R³-CHO in the presence of a reducing agent such as sodium cyanoborohydride to afford (14). The amine (14) may be treated with a reagent LG¹-L¹-COO-PG², where LG¹ is a nucleofugal group such as tosylate, iodide, or bromide, in the presence of an organic base such as potassium carbonate, in a solvent such as DMF, to afford (15). PG² is a carboxyl protecting group such as allyl or methyl, or benzyl, which may be removed by hydrolysis in, for example, aqueous base to afford (2). The hydroxyester (16) may be treated with R³- LG¹ in the presence of a base such as DBU, DIEA, or sodium hydride, to afford (18) where X is O. Alternately, (16) may be treated with methanesulfonyl chloride, toluenesulfonyl chloride, or trifluoromethanesulfonic anhydride to afford (17) where R⁶¹ is an arylsulfonate or alkylsulfonate group. (17) may then be treated with R³-XH, where X is O, S, or N-R⁶, in the presence of a suitable base such as TEA, DIEA, NaH, DBU, potassium t-butoxide, or the like, to afford (18). (18) may be deprotected as described above to afford (2).

Scheme 4 describes an alternate preparation of compounds of Formula (I).

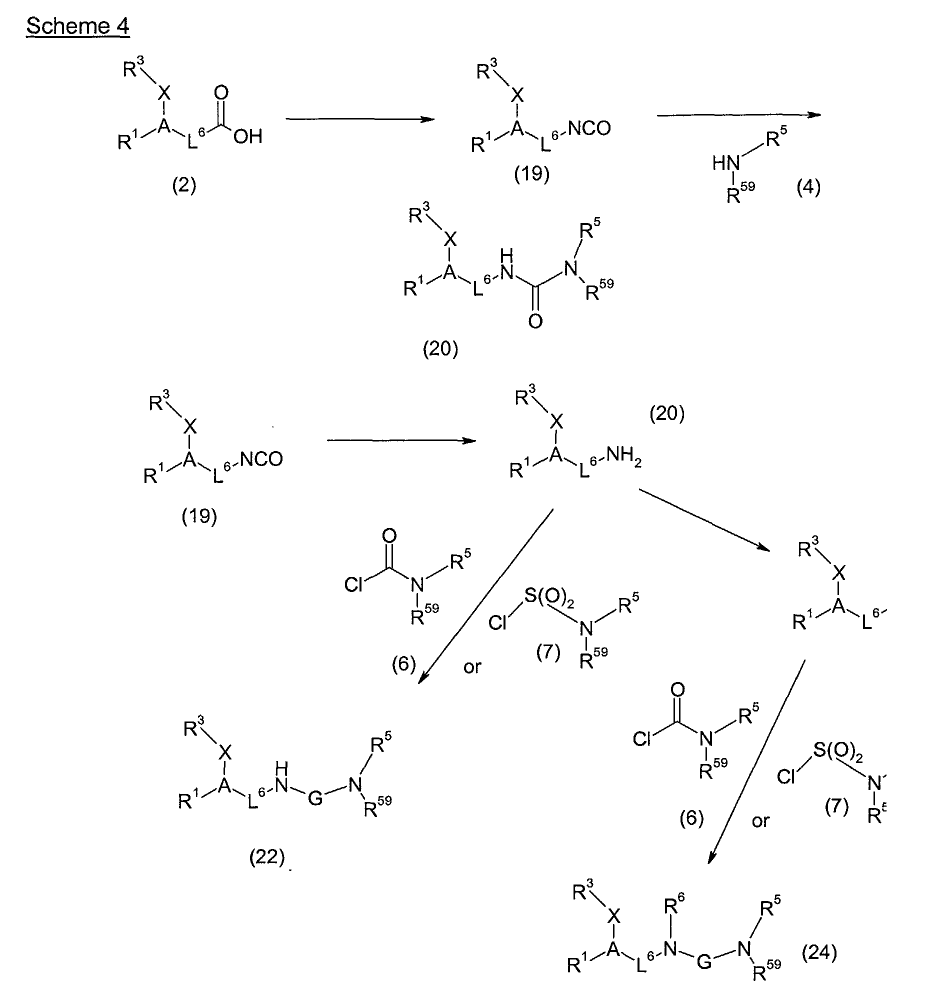

L⁶ is as defined for Scheme 1.

The acid (2) may be treated with oxalyl chloride or thionyl chloride in a solvent such as DCM, followed by sodium azide, to afford an acyl azide intermediate. Alternately, (2) may be treated with diphenylphosphoryl azide in the presence of a base such as DIEA, to afford an acyl azide intermediate. The acyl azide intermediate is heated at a temperature of from 25 to 100°C to afford the isocyanate (19), which may be treated with an amine (4) to afford (20). Alternately, (19) may be hydrolyzed in weak aqueous acid or weak aqueous base to afford the amine (20). The amine (20) may be treated with an aldehyde or ketone embodying the R⁶ group, in the presence of a reducing agent such as sodium triacetoxyborohydride, to afford (23). (23) may be treated with reagents (6) or (7) in manner analogous to Scheme 1 to afford (24). The amine (20) may be treated with reagents (6) or (7) in like manner to afford (22). Where R³⁰ is a solid support such as Wang Resin, (22) and (24) may be treated with TFA as in Scheme 1 to afford (I).

In the above schemes, "PG¹" represents an amino protecting group. The term "amino protecting group" as used herein refers to substituents of the amino group commonly employed to block or protect the amino functionality while reacting other functional groups on the compound. Examples of such amino-protecting groups include the formyl group, the trityl group, the phthalimido group, the trichloroacetyl group, the chloroacetyl, bromoacetyl and iodoacetyl groups, urethane-type blocking groups (PG¹ as used herein) such as benzyloxycarbonyl, 4-phenylbenzyloxycarbonyl, 2-methylbenzyloxycarbonyl, 4-methoxybenzyloxy-carbonyl, 4-fluorobenzyloxycarbonyl, 4-chlorobenzyloxycarbonyl, 3-chlorobenzyloxycarbonyl, 2-chlorobenzyloxycarbonyl, 2,4-dichlorobenzyloxycarbonyl, 4-bromobenzyloxycarbonyl, 3-bromobenzyloxycarbonyl, 4-nitrobenzyloxycarbonyl, 4-cyanobenzyloxy-carbonyl, 2-(4-xenyl)iso-propoxycarbonyl, 1,1-diphenyleth-1-yloxycarbonyl, 1,1-diphenylprop-1-yloxycarbonyl, 2-phenylprop-2-yloxycarbonyl, 2-(p-toluyl)prop-2-yloxycarbonyl, cyclopentanyloxycarbonyl, 1-methylcyclopentanyloxycarbonyl, cyclohexanyloxycarbonyl, 1-methylcyclohexanyloxycarbonyl, 2-methylcyclohexanyloxycarbonyl, 2-(4-toluylsulfonyl)ethoxycarbonyl, 2(methylsulfonyl)ethoxycarbonyl, 2-(triphenylphosphino)ethoxycarbonyl, 9-fluorenylmethoxycarbonyl ("FMOC"), t-butoxycarbonyl ("BOC"), 2-(trimethylsilyl)ethoxycarbonyl, allyloxycarbonyl, 1-(trimethylsilylmethyl)prop-1-enyloxycarbonyl, 5-benzisoxalylmethoxycarbonyl, 4-acetoxybenzyloxycarbonyl, 2,2,2-trichloroethoxycarbonyl, 2-ethynyl-2-propoxycarbonyl, cyclopropylmethoxycarbonyl, 4-(decyloxy)benzyloxycarbonyl, isobornyloxycarbonyl, 1-piperidyloxycarbonyl and the like; the benzoylmethylsulfonyl group, the 2-(nitro)phenyl-sulfenyl group, the diphenylphosphine oxide group and like amino-protecting groups. The species of amino-protecting group employed is not critical so long as the derivatized amino group is stable to the condition of subsequent reaction(s) on other positions of the compound of Formula (I) and can be removed at the desired point without disrupting the remainder of the molecule. Preferred amino-protecting groups are the allyloxycarbonyl, the t-butoxycarbonyl, 9-fluorenylmethoxycarbonyl, and the trityl groups. Similar amino-protecting groups used in the cephalosporin, penicillin and peptide art are also embraced by the above terms. Further examples of groups referred to by the above terms are described by J. W. Barton, "Protective Groups In Organic Chemistry", J. G. W. McOmie, Ed., Plenum Press, New York, N.Y., 1973, Chapter 2, and T. W. Greene, "Protective Groups in Organic Synthesis", John Wiley and Sons, New York, N.Y., 1981, Chapter 7. The related term "protected amino" defines an amino group substituted with an amino-protecting group discussed above.In the above schemes, "PG²" represents carboxyl protecting group. The term "carboxyl protecting group" as used herein refers to substituents of the carboxyl group commonly employed to block or protect the -OH functionality while reacting other functional groups on the compound. Examples of such alcohol -protecting groups include the 2-tetrahydropyranyl group, 2-ethoxyethyl group, the trityl group, the methyl group, the ethyl group, the allyl group, the trimethylsilylethoxymethyl group, the 2,2,2-trichloroethyl group, the benzyl group, and the trialkylsilyl group, examples of such being trimethylsilyl, tert-butyldimethylsilyl, phenyldimethylsilyl, triiospropylsilyl and thexyldimethylsilyl. The choice of carboxyl protecting group employed is not critical so long as the derivatized alcohol group is stable to the condition of subsequent reaction(s) on other positions of the compound of the formulae and can be removed at the desired point without disrupting the remainder of the molecule. Further examples of groups referred to by the above terms are described by J. W. Barton, "Protective Groups In Organic Chemistry", J. G. W. McOmie, Ed., Plenum Press, New York, N.Y., 1973, and T. W. Greene, "Protective Groups in Organic Synthesis", John Wiley and Sons, New York, N.Y., 1981. The related term "protected carboxyl" defines a carboxyl group substituted with a carboxyl -protecting group as discussed above.

### EXAMPLES

### General Procedure A. Synthesis of 2-aryloxyalkanoic acids

To a mixture of ethyl or methyl ester of 2-hydroxyalkanoic acid (2.0 mmol), phenol (2.4 mmol) and triphenylphosphine (0.63 g, 2.4 mmol) in anhydrous THF (6 mL) is added drop wise diisopropyl azodicarboxylate (0.47 mL, 2.4 mmol) with stirring at 0°C. The mixture is stirred at 0°C for 1 h and then at 25°C for 8 h. The solvent is removed and the residue is purified by flash chromatography [silica, ethyl acetate-hexanes (1:9)] to afford the ethyl or methyl ester of 2-aryloxyalkanoic acid. The ester is dissolved in methanol (10 mL) and is added 1 M solution of lithium hydroxide (5 mL). The resulting mixture is stirred for 2 h at 25°C. The reaction mixture is concentrated *in vacuo*. The residue is acidified with dilute HCI and extracted with ethyl acetate (2 x 10 mL). The organic layer is dried (Na₂SO₄) and concentrated to provid the desired 2-aryloxyalkanoic acid.

### General Procedure B. Synthesis of 2-aryloxyalkanoic acids

To a mixture of phenol (2 mmol) and potassium *t*-butoxide (0.47 g, 4.2 mmol) in DMF (6 mL) is added a solution of 2-bromohexanoic acid in DMF (2 mL) rapidly at 0°C. The reaction mixture is stirred at 25°C for 15 h. The resulting mixture is poured into cold 1 N HCI (10 mL) and extracted with ether (2 x 20 mL). The combined extracts are washed with brine, dried (Na₂SO₄) and concentrated *in vacuo* to obtain the desired acid in 70-80% yield. The crude acids are used as such in the preparation of amides.

### General Procedure C. Synthesis of 2-alkyl- and 2-arylthioalkanoic acids

A mixture of methyl ester of arylacetic acid (10 mmol), NBS (10.5 mmol) and benzoyl peroxide (0.2 mmol) in CCl₄ (50 mL) is heated to reflux for 5 h. The precipitated succinimide is filtered and the filtrate is concentrated in *vacuo*. The residue is dissolved in ethyl acetate (50 mL) and is washed with water (2 x 50 mL), Na₂HCO₃ (2 x 30 mL) and brine (2 x 30 mL) and dried (anhyd. Na₂SO₄). Concentration in *vacuo* furnished the 2-bromo-2-aryl-acetic acid methyl ester in 75-82% yield.

To a solution of 2-bromo-2-arylacetic acid methyl ester (2 mmol) in THF (8 mL) is added alkyl thiol (2.4 mmol) followed by Et₃N (4.4 mmol). The mixture is stirred at 25°C for 2 h. The reaction mixture is concentrated in *vacuo* and the residue is purified by flash column chromatography (silica, hexanes-ethyl acetate, 9:1) to give the 2-alkylthio-2-arylacetic acid methyl ester. Similar procedure is adopted for the synthesis of 2-arylthio-2-arylacetic acid methyl ester.

### General Procedure D. Synthesis of 2-alkyl- and 2-arylthioalkanoic acids

To a solution of 2-hydroxy 2-arylacetic acid methyl ester (2 mmol) in CH₂Cl₂ (8 mL) are added MsCI (2.4 mmol) and Et₃N (4.4 mmol) at 0°C and stirred for 1 h. To this solution is added alkyl thiol (2.4 mmol) at 0°C and is stirred for 2 h. The mixture is concentrated in *vacuo* and the residue is dissolved in ethyl acetate (30 mL). The organic layer is washed with water (2 x 20 mL), brine (2 x 20 mL) and dried (anhyd. Na₂SO₄). Concentration in vacuo afforded an oil which is purification by flash chromatography (silica, hexanes-ethyl acetate, 9:1) to furnish the 2-alkylthio-2-arylacetic acid methyl ester. Similar procedure is adopted for the synthesis of 2-arylthio-2-arylacetic acid methyl ester. Hydrolysis of this ester is carried out as described in procedure A.

### General Procedure E. Synthesis of heteroaryl amides of substituted alkanoic acids

To a solution of 2-aryloxyalkanoic acid (0.25 mmol) in THF (3 mL) are added TFFH (80 mg, 0.3 mmol) and DIEA (0.1 mL). The mixture is stirred for 30 min and amine is added (0.6 mmol). The contents are stirred for 10 h and concentrated *in vacuo*. The residue is purified by flash chromatography [silica, ethyl acetate-hexanes (3:7)] to afford the desired amide.

### General Procedure F: Synthesis of hetero aromatic amides of aryl-N-cycloalkyl amino acids

N-Boc aryl amino acid is coupled with heteroaromatic amine as described in procedure E. The N-Boc protected aryl amino acid amide (2 mmol) is treated with 4N HCI in dioxane (5 mL). The mixture is stirred at 25°C for 30 min. The solution is concentrated in *vacuo* and the residue is treated with TEA (5 mmol). The mixture is diluted with ethyl acetate (30 mL). The organic layer is washed with water (2 x 20 mL), brine (2 x 20 mL) and dried (Na₂SO₄). Concentration in vacuo afforded the corresponding Boc-deprotected amine. The amine (1 mmol) is treated with cycloalkanone (1.1 mmol) in 1,2-dichloroethane. The mixture is stirred at 25°C for 30 min. To this solution is added sodium triacetoxy borohydride (1.1 mmol) and the mixture is stirred for 12 h. The mixture is concentrated in vacuo and the residue is dissolved in ethyl acetate (30 mL). The solution is washed with water (2 x 20 mL) and brine (2 x 20 mL) and dried (anhyd. Na₂SO₄). Concentration in vacuuo and purification by flash chromatography (silica, hexanes-ethyl acetate, 7:3) furnished the aryl-N-cycloalkyl amide in 52-63% yield.

### General Procedure G. Synthesis of substituted alkanoic acids

To a solution of alkanoic acid (1.0 g, 5 mmol) in a mixture of anhydrous THF (10 mL) and HMPA (5 mL) is added dropwise LDA (5 mL, 2 M in heptanes/THF/ethylbenzene) at-78°C. The contents are stirred for 1 h at -78°C and then at 25°C for 30 min. The reaction mixture is cooled again to -78°C and is added drop wise a solution of bromoalkane (5 mmol) in THF (5 mL). The cooling bath is removed and the contents are stirred for 12 h at 25°C. The contents of the flask are poured into cold 1N HCI (30 mL) and extracted with ethyl acetate (3 x 50 mL). The combined extracts are washed with brine, dried (Na₂SO₄) and concentrated. The residue is purified by flash chromatography (silica, 1% methanol in chloroform) to obtain the corresponding acid.

### General Procedure H. Synthesis of ureas via Acyl Azide

To a solution of a carboxylic acid (0.5 mmol) in anhydrous dichloromethane (2 mL) are added a drop of DMF and oxalyl chloride (88 µL, 1 mmol) at 0°C. The contents are stirred for 10 min at 0°C and then at 25°C for 1 h. The volatiles are stripped off *in vacuo* and the residue is dissolved in dichloroethane (4 mL). Sodium azide (195 mg, 3 mmol) is added and the mixture is heated at 80°C for 3 h. The resulting isocyanate solution is cooled and amine (1 mmol) is added. The resulting mixture is heated at 80°C for 12 h. The solvent is removed by rotary evaporation and the residue is purified by flash chromatography [silica, ethyl acetate-dichloromethane (1:4)] to obtain the corresponding urea.

### General Procedure I. Reductive Amination

Amine is dissolved in dry THF (0.1-1 M) in a round-bottomed flask, and then aledhyde or ketone (1 eq, or slight excess) is added to the solution and stirred for a few min at 25°C. Sodium triacetoxyborohydride (2 eq) is added and the mixture stirred at rt. The reaction mixture is then washed with saturated sodium bicarbonate (200 ml) and extracted with ethyl acetate. The organic extracts are combined, dried, and concentrated *in vacuo* to give the product secondary or tertiaty amine.

### General Procedure J. Preparation of Ureas via Carbamoyl Chloride

The secondary amine (1 eq) is dissolved dry DCM (0.1-1 M) and triethylamine (1-3 eq) is added. Then triphosgene (1-3 eq) is added at -20°C. After a few hours at 25°C a primary or secondary amine (1 eq) is added to the reaction mixture. After consumption of starting material, the mixture is subjected to aqueous workup. Concentration in vacuo afforded the product. urea.

### General Procedure K. Amine Alkylation

The amine is dissolved in dry DMF and alkali metal carbonate (1-5 eq) is added to the solution. Alkyl halide or alkyl sulfonate (1-5 eq) is added and resulting mixture is stirred for a few hours at 0°C to 100°C. The mixture was given an aqueous workup. Drying and concentration afforded the alkylated amine.

### General Procedure L. Preparation of Polymer - Supported Amine

A primary or secondary amine (5 eq) is dissolved in DCE (1-5 M), then solid supported 2-(4-formyl-3-methoxyphenoxy) ethyl - functionalized polystyrene (1 eq, based on loading of the aldehyde functionality) is added to the solution and stirred for 30 min. Acetic acid (0.5 eq) is added to the mixture followed by addition of sodium triacetoxyborohydride (5 eq). The resin mixture is shaken at 25°C for 4-48 h, then washed with three cycles of DMF/methanol/DCM, in turn. Then the resin is dried *in vacuo* to give solid-supported primary or secondary amine.

### General Procedure M. Preparation of Polymer - Supported Carbamoyl Chloride

The solid-supported primary or secondary 4-aminomethyl-3-methoxyphenoxy)-1-ethyl polystyrene (1 eq) is treated with triphosgene (2 eq) in the presence of DCM (0.1-3 M) and diisopropylethylamine (3-10 eq). The product on solid support is then washed with three cycles of DMF/methanol/DCM. Then the resin is dried *in vacuo* to give corresponding carbamyl chloride.

### General Procedure N. Preparation of Polymer - Supported Ureas From Carbamoyl Chloride

Polymer supported carbamyl chloride (1.0 g, 1.46 mmol 1 eq ) is treated with primary or secondary amine (3-10 eq) in the presence of DCE (0.02-3 M)) and diisopropylethylamine (3-20 eq). After 1-10 h, the resin is then washed with three cycles of DMF/methanol/DCM and dried *in vacuo* to afford the urea.

### General Procedure O. Alkylation of Urea, or Carbamate, or Amide Nitrogen on Polymer Support

To the corresponding polymer supported urea, carbamate, or amide having a free NH group (1 eq) 0.117 g, 0.73 mmol) is added potassium *t*-butoxide (3-10 eq). The resin mixture is shaken for 1 h at 25°C and then alkyl halide or alkyl sulfonate 3-10 eq is added. After 16 h the polymer is then washed with three cycles of DMF/methanol/DCM and dried *in vacuo* to give the alkylated product.

### General Procedure P. Cleavage from Solid Support

The polymer is treated with TFA solution (5-50% v/v/ in DCM, excess) at 25°C to cleave the product from the polymer support. Filtration and concentration in vacuo affords the product.

### General Procedure Q. Preparation of N,O-Dimethyl N-Hydroxycarboxamides

Carboxylic acid (1 eq) in DCM (0.02-2 M) is treated with *N,O*- dimethylhydroxyl amine hydrochloride (1 eq) and triethylamine (1 eq), DCC, EDC, or other carbodiimide reagent (1 eq) is added. After 1-24 hr, The solution is concentrated *in vacuo* and the residue is removed by filtration. Alternately, the mixture may be given an aqueous workup. The filtrate is concentrate in vacuo.. The product is used directly or is purified by silica gel chromatography.

### General Procedure R. Preparation of Ketones from N,O-Dimethyl N-Hydroxycarboxamides

Organolithium or organomagnesium reagent, whether prepared in situ or commercially obatined, is treated in THF or ether (0.02 - 1 M) with N,O-dimethyl N-hydroxy-carboxamides (1 eq) at a temperature of from -20°C to 25°C. Once starting amide is consumed, the mixture is given an aqueous workup. Concentration in vacuo afforded the product ketone.

### General Procedure S1. Preparation of Secondary alcohol from Ketone

The ketone (1 eq) in ethanol or methanol (0.02 - 2 M) treated with sodium borohydride (0.25 - 2 eq,) at 0°C - 25°C, followed by aqueous workup affords the secondary alcohol.

### General Procedure S2. Preparation of Secondary alcohol from Ketone

The ketone 1 eq in THF or ether (0.02 - 2 M) is treated with an aluminum hydride reagent such as LiAlH₄ (0.25 - 2 eq) at - 78°C - 25°C, followed by quench and filtration, affording the secondary alcohol.

### General Procedure S3. Preparation of tertiary alcohol from Ketone

The ketone(1 eq) in THF or ether (0.02 - 2 M) is treated with an organolithium or organomagnesium reagent at a temperature of - 78°C to 25°C. Aqueous workup affords the tertiary alcohol.

### General Procedure T. Preparation of Chloroformates

An alcohol (1 eq (0.259 g, 1.0 mmol) solution in DCM (0.02 - 2 M) 10 ml) is treated with triethylamine (excess)) and phosgene (1.5 eq, solution in toluene) at-20°C and stirred for 1-5 h. Excess phosgene and triethylamine is removed *in vacuo*. The product chloroformate is used immimmediately .

### General Procedure U. Preparation of Carbamates

A solution of chloroformate (1 eq) in DCM (0.02 - 2 M ) is treated with tertiary amine (2 -5 eq)mmol) and primary or secondary amine (1 eq). After consumption of starting material The mixture is then given an aqueous sorkup. Concentration in vacuo afforded the product carbamate.

### Example 1

### 2-(3,4-Dichloro-phenoxy)-hexanoic acid thiazol-2-yl amide

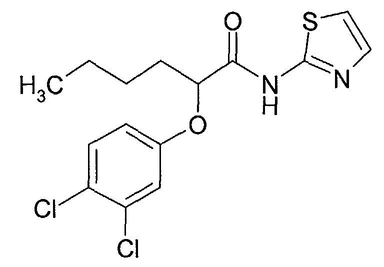

2-(3,4-Dichlorophenoxy)hexanoic acid (0.3 g, 55%) is prepared from ethyl 2-hydroxyhexanoate (0.32 g, 2.0 mmol) and 3,4-dichlorophenol (0.39 g, 2.4 mmol) following general procedure A. A solution of this acid (69 mg, 0.25 mmol) in THF (3 mL) is reacted with 2-aminothiazole (60 mg, 0.6 mmol) following general procedure E to obtain 2-(3,4-dichloro-phenoxy)-hexanoic acid thiazol-2-yl amide (64 mg, 72%).
LCMS (*m*/*z*): 360 (M + H)⁺

### Example 2

### 2-(4-Fluorophenoxy)-N-1,3-thiazol-2-ylhexanamide

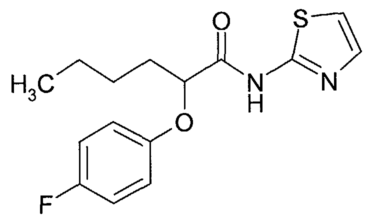

2-(4-Fluorophenoxy)hexanoic acid (0.23 g, 52%) is prepared from ethyl 2-hydroxyhexanoate (0.32 g, 2.0 mmol) and 4-fluorophenol (0.27 g, 2.4 mmol) following the general procedure A. A solution of this acid (56 mg, 0.25 mmol) in THF (3 mL) is reacted with 2-aminothiazole (60 mg, 0.6 mmol) following the general procedure E to obtain 2-(4-fluoro-phenoxy)-N-1,3-thiazol-2-ylhexanamide (58 mg, 76%).
LCMS (*m*/*z*): 310 (M + 2H)⁺

### Example 3

### 2-(4-Methoxyphenoxy)-N-1,3-thiazol-2-ylhexanamide

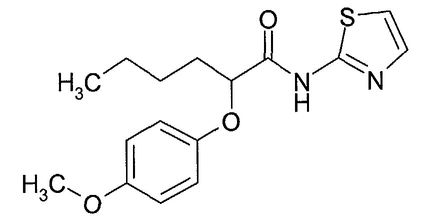

2-(4-Methoxyphenoxy)hexanoic acid (0.23 g, 48%) is prepared from ethyl 2-hydroxyhexanoate (0.32 g, 2.0 mmol) and 4-methoxyphenol (0.3 g, 2.4 mmol) following the general procedure A. A solution of this acid (60 mg, 0.25) in THF (3 mL) is reacted with 2-aminothiazole (60 mg, 0.6 mmol) following the general procedure E to obtain 2-(4-methoxy-phenoxy)-N-1,3-thiazol-2-ylhexanamide (130 mg, 82%).
LCMS (*m*/*z*): 322 (M + 2H)⁺

### Example 4

### 2-(4-Methoxyphenoxy)-N-pyridin-2-ylhexanamide

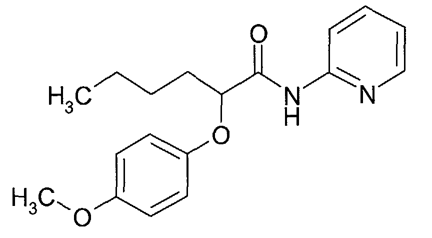

A solution of 2-(4-methoxyphenoxy)hexanoic acid (60 mg, 0.25 mmol) in THF (3 mL) is reacted with 2-aminopyridine (56 mg, 0.6 mmol) following the general procedure E to obtain 2-(4-methoxyphenoxy)-N-pyridin-2-ylhexanamide (58 mg, 75%).
LCMS (*m*/*z*): 316 (M + 2H)⁺

### Example 5

### 2-(3,4-Dichlorophenoxy)-4-methyl-N-1,3-thiazol-2-ylpentanamide

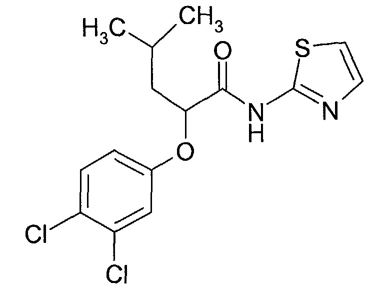

2-(3,4-dichlorophenoxy)-4-methylpentanoic acid (0.26 g, 46%) is prepared from methyl 2-hydroxy-4-methylpentanoate (0.32 g, 2.0 mmol) and 3,4-dichlorophenol (0.39 g, 2.4 mmol) following the general procedure A. A solution of this acid (69 mg, 0.25 mmol) in THF (3 mL) is reacted with 2-aminothiazole (60 mg, 0.6 mmol) following the general procedure E to obtain 2-(3,4-dichlorophenoxy)-4-methyl-N-1,3-thiazol-2-ylpentanamide (74 mg, 82%).
LCMS (*m*/*z*): 360 (M + H)⁺

### Example 6

### 2-(1,1'-Biphenyl-4-vloxy)-N-1,3-thiazol-2-ylhexanamide

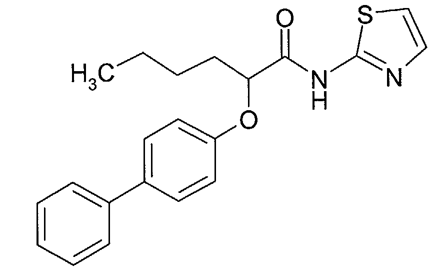

2-(4-phenylphenoxy)hexanoic acid (0.15 g, 26%) is prepared from ethyl 2-hydroxyhexanoate (0.32 g, 2.0 mmol) and 4-hydroxybiphenyl (0.39 g, 2.4 mmol) following the general procedure A. A solution of this acid (71 mg, 0.25 mmol) in THF (3 mL) is reacted with 2-aminothiazole (60 mg, 0.6 mmol) following the general procedure E to obtain 2-(1,1'-biphenyl-4-yloxy)-N-1,3-thiazol-2-ylhexanamide (60 mg, 66%).
LCMS (*m*/*z*): 368 (M + 2H)⁺

### Example 7

### 2-(4-Isopropylphenoxy)-N-1,3-thiazol-2-ylhexanamide

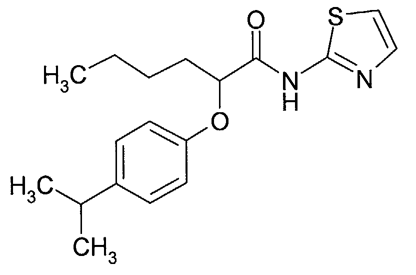

2-(4-Isopropylphenoxy)hexanoic acid (0.23 g, 46%) is prepared from ethyl 2-hydroxyhexanoate (0.32 g, 2.0 mmol) and 4-isopropylphenol (0.33 g, 2.4 mmol) following the general procedure A. A solution of this acid (63 mg, 0.25 mmol) in THF (3 mL) is reacted with 2-aminothiazole (60 mg, 0.6 mmol) following the general procedure E to obtain 2-(4-iso-propylphenoxy)-N-1,3-thiazol-2-ylhexanamide (68 mg, 82%).
LCMS (*m*/*z*): 334 (M + 2H) ⁺

### Example 8

### 2-(3-Methoxyphenoxy)-N-1,3-thiazol-2-ylhexanamide

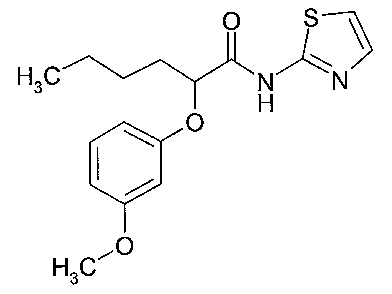

2-(3-Methoxyphenoxy)hexanoic acid (0.37 g, 82%) is prepared from 2-bromohexanoic acid (0.39 g, 2.0 mmol) and 3-methoxyphenol (0.25 g, 2.0 mmol) following the general procedure B. A solution of this crude acid (60 mg, 0.25 mmol) in THF (3 mL) is reacted with 2-aminothiazole (60 mg, 0.6 mmol) following the general procedure E to obtain 2-(3-methoxyphenoxy)-N-1,3-thiazol-2-ylhexanamide (61 mg, 75%).
LCMS (*m*/*z*): 322 (M + 2H)⁺

### Example 9

### 2-(2,3-Dimethoxyphenoxy)-N-1,3-thiazol-2-ylhexanamide

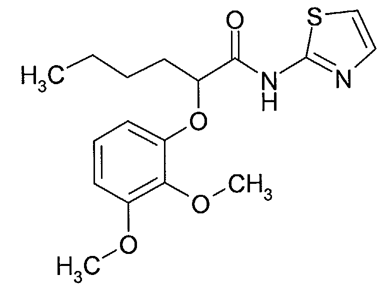

2-(2,3-Dimethoxyphenoxy)hexanoic acid (0.34 g, 64%) is prepared from 2-bromohexanoic acid (0.39 g, 2.0 mmol) and 2,3-dimethoxyphenol (0.31 g, 2.0 mmol) following the general procedure B. A solution of this crude acid (67 mg, 0.25 mmol) in THF (3 mL) is reacted with 2-aminothiazole (60 mg, 0.6 mmol) following the general procedure E to obtain 2-(2,3-dimethoxyphenoxy)-N-1,3-thiazol-2-ylhexanamide (64 mg, 73%).
LCMS (*m*/*z*): 352 (M + 2H)⁺

### Example 10

### 2-(3,4-Dimethoxyphenoxy)-N-1,3-thiazol-2-ylhexanamide

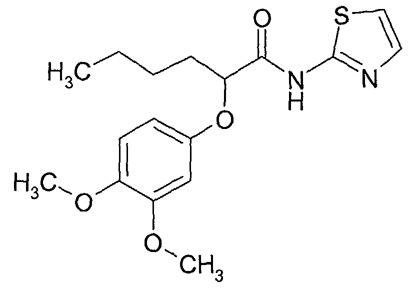

2-(3,4-Dimethoxyphenoxy)hexanoic acid (0.34 g, 63%) is prepared from 2-bromohexanoic acid (0.39 g, 2.0 mmol) and 3,4-dimethoxyphenol (0.31 g, 2.0 mmol) following the general procedure B. A solution of this crude acid (67 mg, 0.25 mmol) in THF (3 mL) is reacted with 2-aminothiazole (60 mg, 0.6 mmol) following the general procedure E to obtain 2-(3,4-dimethoxyphenoxy)-N-1,3-thiazol-2-ylhexanamide (61 mg, 69%).
LCMS (*m* /*z*): 352 (M + 2H)⁺

### Example 11

### 2-(3,5-dimethoxyphenoxy)-N-1,3-thiazol-2-ylhexanamide

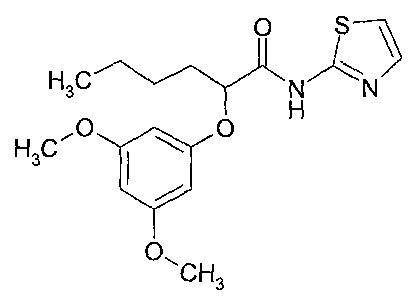

2-(3,5-Dimethoxyphenoxy)hexanoic acid (0.43 g, 81%) is prepared from 2-bromohexanoic acid (0.39 g, 2.0 mmol) and 3,5-dimethoxyphenol (0.31 g, 2.0 mmol) following the general procedure B. A solution of this crude acid (67 mg, 0.25 mmol) in THF (3 mL) is reacted with 2-aminothiazole (60 mg, 0.6 mmol) following the general procedure E to obtain 2-(3,5-dimethoxyphenoxy)-N-1,3-thiazol-2-ylhexanamide (77 mg, 89%).
LCMS (*m*/*z*): 352 (M + 2H)⁺

### Example 12

### 2-(2-Naphthyloxy)-N-1,3-thiazol-2-ylhexanamide

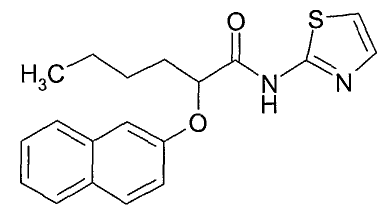

2-(2-Naphthoxy)hexanoic acid (0.39 g, 75%) is prepared from 2-bromohexanoic acid (0.39 g, 2.0 mmol) and 2-naphthol (0.29 g, 2.0 mmol) following the general procedure B. A solution of this crude acid (65 mg, 0.25 mmol) in THF (3 mL) is reacted with 2-aminothiazole (60 mg, 0.6 mmol) following the general procedure E to obtain 2-(2-naphthyloxy)-N-1,3-thiazol-2-ylhexanamide (62 mg, 73%).
LCMS (*m*/*z*): 342 (M + 2H)⁺

### Example 13

### 2-(2,4-Difluorophenoxy)-N-1,3-thiazol-2-ylhexanamide

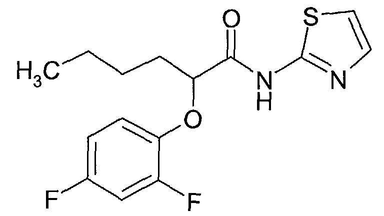

2-(2,4-Difluorophenoxy)hexanoic acid (0.34 g, 71%) is prepared from 2-bromohexanoic acid (0.39 g, 2.0 mmol) and 2,4-difluorophenol (0.26 g, 2.0 mmol) following the general procedure B. A solution of this crude acid (61 mg, 0.25 mmol) in THF (3 mL) is reacted with 2-aminothiazole (60 mg, 0.6 mmol) following the general procedure E to obtain 2-(2,4-difluorophenoxy)-N-1,3-thiazol-2-ylhexanamide (61 mg, 74%).
LCMS (*m*/*z*): 328 (M + 2H)⁺

### Example 14

### 2-(3,4-Difluorophenoxy)-N-1,3-thiazol-2-ylhexanamide

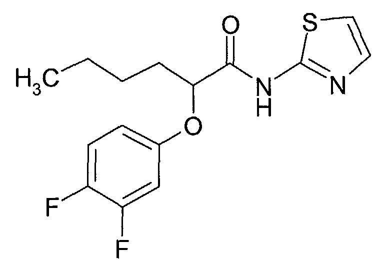

2-(3,4-Difluorophenoxy)hexanoic acid (0.4 g, 82%) is prepared from 2-bromohexanoic acid (0.39 g, 2.0 mmol) and 3,4-difluorophenol (0.26 g, 2.0 mmol) following the general procedure B. A solution of this crude acid (61 mg, 0.25 mmol) in THF (3 mL) is reacted with 2-aminothiazole (60 mg, 0.6 mmol) following the general procedure E to obtain 2-(3,4-difluorophenoxy)-N-1,3-thiazol-2-ylhexanamide (71 mg, 88%).
LCMS (*m*/*z*): 328 (M + 2H)⁺

### Example 15

### 2-(1,3-Benzodioxol-5-yloxy)-N-1,3-thiazol-2-ylhexanamide

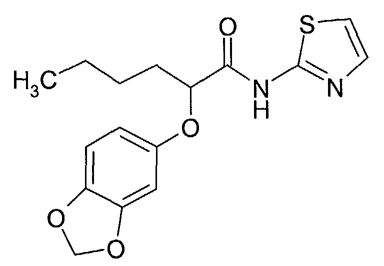

2-(3,4-Methylenedioxyphenoxy)hexanoic acid (0.42 g, 83%) is prepared from 2-bromohexanoic acid (0.39 g, 2.0 mmol) and 3,4-methylenedioxyphenol (0.28 g, 2.0 mmol) following the general procedure B. A solution of this crude acid (63 mg, 0.25 mmol) in THF (3 mL) is reacted with 2-aminothiazole (60 mg, 0.6 mmol) following the general procedure E to obtain 2-(1,3-benzodioxol-5-yloxy)-N-1,3-thiazol-2-ylhexanamide (75 mg, 91%).
LCMS (*m*/*z*): 336 (M + 2H)⁺

### Example 16

### 2-(4-Methoxyphenoxy)-N-1,3-thiazol-2-ylheptanamide

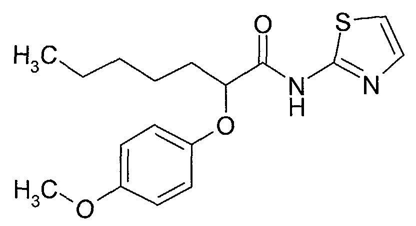

To a mixture of 4-methoxyphenol (0.25 g, 2.0 mmol) and potassium *t*-butoxide (235 mg, 2.1 mmol) in DMF (4 mL) is added a solution of ethyl 2-bromoheptanoate (0.47 g, 2.0 mmol) in DMF (2 mL) rapidly at 0°C. The reaction mixture is stirred at 25°C for 15 h. The contents are poured into water (20 mL) and extracted with ether (2x20 mL). The combined extracts are concentrated and the residue is dissolved in THF (5 mL). To the THF solution is added 1 N lithium hydroxide (10 mL) and the contents are stirred for 2 h. The resulting mixture is poured into cold 1 N HCI (10 mL) and extracted with ether (2x20 mL). The combined extracts are ished with brine, dried (Na₂SO₄) and concentrated *in vacuo* to obtain the desired acid (0.43 g, 82%).

A solution of this crude acid (63 mg, 0.25 mmol) in THF (3 mL) is reacted with 2-aminothiazole (60 mg, 0.6 mmol) following the general procedure E to obtain 2-(4-methoxy-phenoxy)-N-1,3-thiazol-2-ylheptanamide (65 mg, 78%).
LCMS (*m*/*z*): 336 (M + 2H)⁺

### Example 17

### 2-(4-Fluorophenoxy)-N-1,3-thiazol-2-ylheptanamide

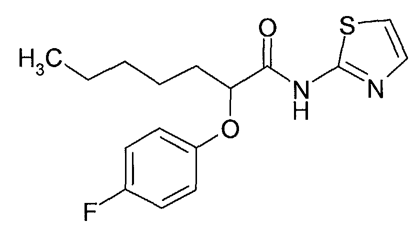

To a mixture of 4-fluorophenol (0.22 g, 2.0 mmol) and potassium *t*-butoxide (235 mg, 2.1 mmol) in DMF (4 mL) is added a solution of ethyl 2-bromoheptanoate (0.47 g, 2.0 mmol) in DMF (2 mL) rapidly at 0°C. The reaction mixture is stirred at 25°C for 15 h. The contents are poured into water (20 mL) and extracted with ether (2x20 mL). The combined extracts are concentrated and the residue is dissolved in THF (5 mL). To the THF solution is added 1 N lithium hydroxide (10 mL) and the contents are stirred for 2 h. The resulting mixture is poured into cold 1 N HCI (10 mL) and extracted with ether (2x20 mL). The combined extracts are washed with brine, dried (anhyd Na₂SO₄) and concentrated *in vacuo* to obtain the desired acid (0.35 g, 74%).

A solution of this crude acid (60 mg, 0.25 mmol) in THF (3 mL) is reacted with 2-aminothiazole (60 mg, 0.6 mmol) following the general procedure E to obtain 2-(4-fluoro-phenoxy)-N-1,3-thiazol-2-ylheptanamide (65 mg, 78%).
LCMS (*m*/*z*): 324 (M + 2H)⁺

### Example 18

### 2-(4-Chloro-phenylsulfanyl)-hexanoic acid thiazol-2-ylamide

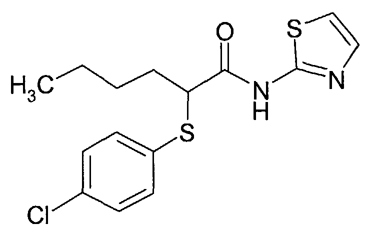

To a mixture of ethyl 2-hydroxyhexanoate (0.32 g, 2.0 mmol), 4-chlorobenzenethiol (0.35 g, 2.4 mmol) and triphenylphosphine (0.63 g, 2.4 mmol) in anhydrous THF (6 mL) is added dropwise diisopropyl azodicarboxylate (0.47 mL, 2.4 mmol) with stirring at 0°C. The mixture is stirred at 0°C for 1 h and then at 25°C for 8 h. The solvent is removed and the residue is purified by flash chromatography [silica, ethyl acetate-hexanes (1:20)] to afford the ethyl 2-(4-chlorophenylthio)hexanoate (0.22 g, 35%). This is dissolved in THF (4 mL) and a 1 M solution of lithium hydroxide (10 mL) is added and the resulting mixture is stirred for 2 h at 25°C. Acidification with dilute HCI followed by extraction with ether (2x20 mL) provided 2-(4-chlorophenylthio)hexanoic acid (183 mg, 91%).

A solution of this acid (64 mg, 0.25 mmol) in THF (3 mL) is reacted with 2-aminothiazole (60 mg, 0.6 mmol) following the general procedure E to obtain 2-(4-chloro-phenyl-sulfanyl)-hexanoic acid thiazol-2-ylamide (74 mg, 82%).
LCMS (*m*/*z*): 342 (M + 2H)⁺

### Example 19

### 2-(4-Chloro-phenylsulfanyl)-hexanoic acid pyridin-2-ylamide

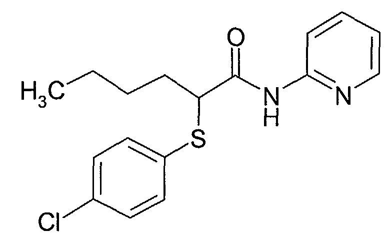

A solution of 2-(4-chlorophenylthio)hexanoic acid (64 mg, 0.25 mmol) in THF (3 mL) is reacted with 2-aminopyridine (56 mg, 0.6 mmol) following the general procedure E to obtain 2-(4-chloro-phenylsulfanyl)-hexanoic acid pyridin-2-ylamide (70 mg, 85%).
LCMS (*m*/*z*): 336 (M + 2H)⁺

### Example 20

### N-[1-(4-chlorophenyl)cyclopentyl]-N'-1,3-thiazol-2-ylurea

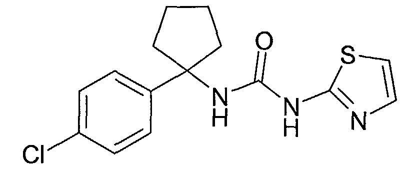

Following general procedure H, 1-(4-chlorophenyl)-1-cyclopentanecarboxylic acid (112 mg, 0. 5 mmol) is transformed into the corresponding acid chloride, which in turn gave the 1-(4-chlorophenyl)-1-cyclopentyl isocyanate. This isocyanate is reacted with 2-aminothiazole (100 mg, 1.0 mmol) to obtain N-[1-(4-chlorophenyl)cyclopentyl]-N'-1,3-thiazol-2-ylurea (115 mg, 72%).
LCMS (*m*/*z*): 323 (M + 2H)⁺

### Example 21

### N-[1-(4-chlorophenyl)cyclopentyl]-N'-pyridin-2-ylurea

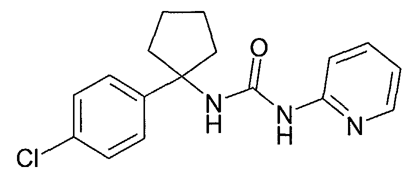

Following general procedure H, 1-(4-chlorophenyl)-1-cyclopentanecarboxylic acid (112 mg, 0.5 mmol) is transformed into the corresponding acid chloride, which in turn gave the 1-(4-chlorophenyl)-1-cyclopentyl isocyanate. This isocyanate is reacted with 2-aminopyridine (94 mg, 1.0 mmol) to obtain N-[1-(4-chlorophenyl)cyclopentyl]-N'-pyridin-2-ylurea (108 mg, 68%).
LCMS (*m*/*z*): 317 (M + 2H)⁺

### Example 22

### N-[1-(4-chlorophenyl)cyclohexyl]-N'-1,3-thiazol-2-ylurea

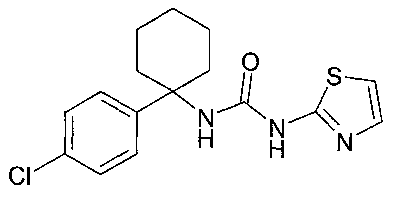

Following general procedure H, 1-(4-chlorophenyl)-1-cyclohexanecarboxylic acid (119 mg, 0. 5 mmol) is transformed into the corresponding acid chloride, which in turn gave the 1-(4-chlorophenyl)-1-cyclohexyl isocyanate. This isocyanate is reacted with 2-aminothiazole (100 mg, 1.0 mmol) to obtain N-[1-(4-chlorophenyl)cyclohexyl]-N'-1,3-thiazol-2-ylurea (104 mg, 62%).
LCMS (*m*/*z*): 337 (M + 2H)⁺

### Example 23

### N-[1-(4-Chlorophenyl)cyclohexyl]-N'-pyridin-2-ylurea

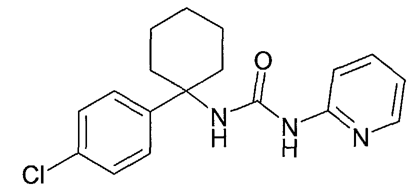

Following general procedure H, 1-(4-chlorophenyl)-1-cyclohexanecarboxylic acid (119 mg, 0.5 mmol) is transformed into the corresponding acid chloride, which in turn gave the 1-(4-chlorophenyl)-1-cyclohexyl isocyanate. This isocyanate is reacted with 2-aminopyridine (94 mg, 1.0 mmol) to obtain N-[1-(4-chlorophenyl)cyclohexyl]-N'-pyridin-2-ylurea (106 mg, 65%).
LCMS (*m*/*z*): 331 (M + 2H)⁺

### Example 24

### 3-(4-Chlorophenyl)-N-pyridin-2-yl-3-(tetrahydro-2H-thiopyran-4-ylamino)propanamide

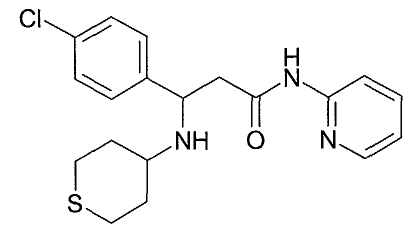

3-(4-Chlorophenyl)-N-pyridin-2-yl-3-(tetrahydro-2H-thiopyran-4-ylamino)propanamide (206 mg, 55%) is prepared from 3-N-Boc-3-(4-chloro phenyl)propionic acid (300 mg, 1 mmol), 2-amino pyridine (225 mg, 2.4 mmol ) and 4-tetrahydrothiopyranone (127 mg, 1.1 mmol), following the general procedure F.
LCMS (*m*/*z*): 377 (M + 2 H)⁺

### Example 25

### 3-(4-Chlorophenyl)-3-(tetrahydro-2H-thiopyran-4-ylamino)-N-1,3-thiazol-2-ylpropanamide

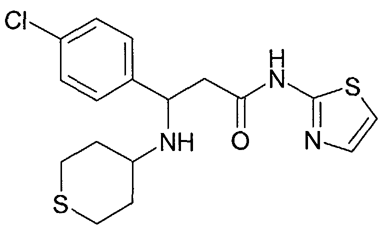

3-(4-Chlorophenyl)-3-(tetrahydro-2H-thiopyran-4-ylamino)-N-1,3-thiazol-2-ylpropanamide (198 mg, 52%) is prepared from 3-N-Boc-3-(4-chloro phenyl)propionic acid (300 mg, 1 mmol), 2-amino thiazole (240 mg, 2.4 ) and 4-tetrahydrothiopyranone(127 mg, 1.1 mmol), following the general procedure F.
LCMS (*m*/*z*): 383 (M + 2 H) ⁺

### Example 26

### 2-(4-Chlorophenyl)-2-(4-methylphenoxy)-N-pyridin-2-ylacetamide

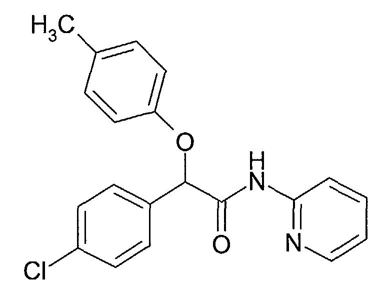

2-(4-Methyl-phenoxy)-2-(4-chlorophenyl) acetic acid (276 mg, 50%) is prepared from 4-chloro-mandalic acid methyl ester (402 mg, 2 mmol), 4-methyl phenol (260 mg 2.4 mmol) following the general procedure A. A solution of this acid (70 mg, 0.25 mmol) in THF is reacted with 2-aminopyridine (60 mg, 0.6 mmol) following the general procedure E to obtain 2-(4-chlorophenyl)-2-(4-methylphenoxy)-N-pyridin-2-ylacetamide (63 mg, 72%).
LCMS (*m*/*z*): 354 (M+H)⁺

### Example 27

### 2-(4-Chlorophenyl)-2-(4-methylphenoxy)-N-1,3-thiazol-2-ylacetamide

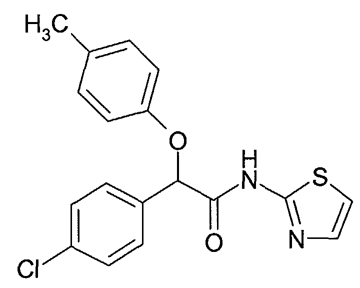

A solution of 2-(4-methyl-phenoxy)-2-(4-chlorophenly) acetic acid (70 mg, 0. 25 mmol) in THF is reacted with 2-aminothiazole (60 mg, 0.6 mmol) following the general procedure E to obtain 2-(4-chlorophenyl)-2-(4-methylphenoxy)-N-1,3-thiazol-2-ylacetamide (66 mg, 74%).
LCMS (*m*/*z*): 360 (M + 2H) ⁺

### Example 28

### 2-(4-Bromophenoxy)-2-(4-chlorophenyl)-N-pyridin-2-ylacetamide

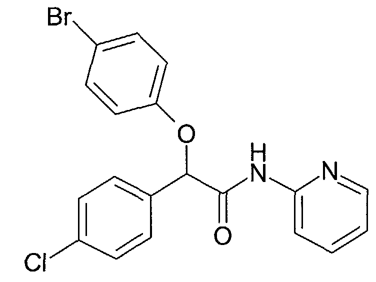

2-(4-Bromophenoxy)-2-(4-chlorophenyl) acetic acid (450 mg, 54%) is prepared from 4-chloromandelic acid methyl ester (402 mg, 2 mmol), 4-bromophenol (415 mg, 2.4 mmol) following general procedure A. A solution of this acid (104 mg, 0.25 mmol) in THF is reacted with 2-aminopyridine (56 mg, 0. 6 mmol) following the general procedure E to obtain 2-(4-bromophenoxy)-2-(4-chlorophenyl)-N-pyridin-2-ylacetamide (75 mg, 72%).
LCMS (*m*/*z*): 419 (M+ 2H)⁺

### Example 29

### 2-(4-Bromophenoxy)-2-(4-chlorophenyl)-N-1,3-thiazol-2-ylacetamide

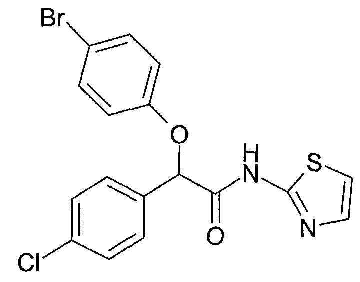

A solution of 2-(4-bromophenoxy)-2-(4-chlorophenly) acetic acid (104 mg, 0.25 mmol) in THF is reacted with 2-aminothiazole (60 mg, 0.60 mmol) following the general procedure E to obtain 2-(4-bromophenoxy)-2-(4-chlorophenyl)-N-1,3-thiazol-2-ylacetamide (78 g, 74%).
LCMS (*m*/*z*): 425 (M+ 2H)⁺

### Example 30

### 2-(4-Chlorophenyl)-2-(4-fluorophenoxy)-N-1,3-thiazol-2-ylacetamide

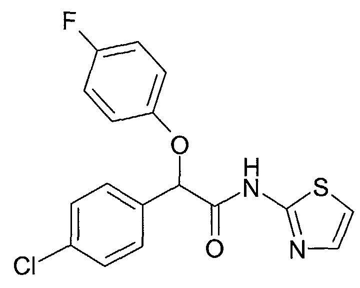

2-(4-Fluorophenoxy)-2-(4-chlorophenyl) acetic acid (296 mg, 53%) is prepared from 4-chloro-mandalic acid methyl ester (402 mg, 2 mmol), 4-fluorophenol (269 mg, 2.4 mmol) following the general procedure A. A solution of this acid (70 mg, 0.25 mmol) in THF is reacted with 2-aminothiazole (60 mg, 0.60 mmol) following the general procedure E to obtain 2-(4-chlorophenyl)-2-(4-fluorophenoxy)-N-1,3-thiazol-2-ylacetamide (71 mg, 78%).
LCMS (*m*/*z*): 364 (M+ 2H)⁺

### Example 31

### 2-(4-Chlorophenyl)-2-(3,4-dichlorophenoxy)-N-pyridin-2-ylacetamide

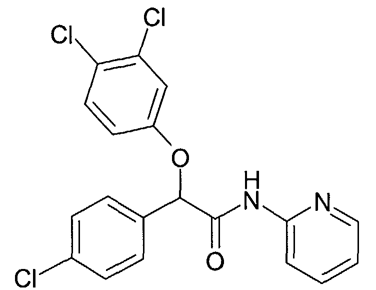

2-(3,4-Dichlorophenoxy)-2-(4-chlorophenly) acetic acid (363 mg, 55%) is prepared from 4-chloro-mandalic acid methyl ester (402 mg, 2 mmol), 3,4-dichlorophenol (390 mg 2.4 mmol) following the general procedure A. A solution of this acid (83 mg, 0.25 mmol) in THF is reacted with 2-aminopyridine (56 mg, 0.60 mmol) following the general procedure E to obtain 2-(4-chlorophenyl)-2-(3,4-dichlorophenoxy)-N-pyridin-2-ylacetamide (82 mg, 80%).
LCMS (*m*/*z*): 409 (M+ 2H)⁺

### Example 32

### 2-(4-Bromophenoxy)-2-(4-bromophenyl)-N-pyridin-2-ylacetamide

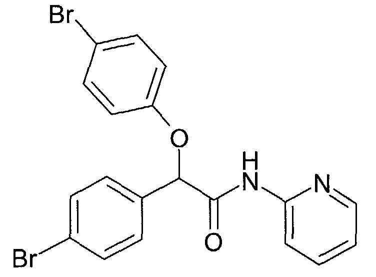

2-(4-Bromophenoxy)-2-(4-bromophenly) acetic acid (461 mg, 60%) is prepared from 4-bromomandalic acid methyl ester (490 mg, 2 mmol) and 4-bromophenol (415 mg, 2.4 mmol) following the general procedure A. A solution of this acid (96 mg, 0.25 mmol) in THF is reacted with 2-aminopyridine (56 mg, 0.60 mmol) following the general procedure E to obtain 2-(4-bromophenoxy)-2-(4-bromophenyl)-N-pyridin-2-ylacetamide (104 mg 90%).
LCMS (*m*/*z*): 464 (M+ 2H)⁺

### Example 33

### 2-(4-Bromophenoxy)-2-(4-bromophenyl)-N-1,3-thiazol-2-ylacetamide

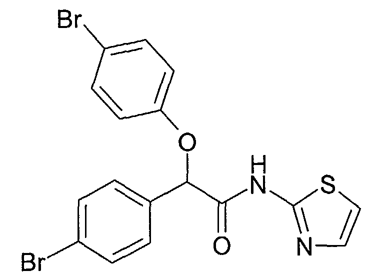

A solution of 2-(4-bromophenoxy)-2-(4-bromophenyl) acetic acid (96 mg, 0.25 mmol) in THF is reacted with 2-aminothiazole (60 mg, 0.60 mmol) following the general procedure E to obtain 2-(4-bromophenoxy)-2-(4-bromophenyl)-N-1,3-thiazol-2-ylacetamide (103 g, 88%).
LCMS (*m*/*z*): 470 (M+ 2H)⁺

### Example 34

### 2-(4-Bromophenyl)-2-(4-methylphenoxy)-N-pyridin-2-ylacetamide

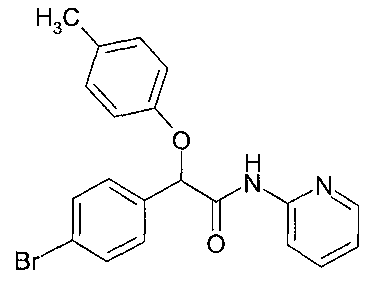

2-(4-Methylphenoxy)-2-(4-bromophenyl) acetic acid (372 mg, 58%) is prepared from 4-bromomandelic acid methyl ester (490 mg, 2 mmol), 4-methylphenol (260 mg, 2.4 mmol) following the general procedure A. A solution of this acid (80 mg, 0.25 mmol) in THF is reacted with 2-aminopyridine (56 mg, 0.6 mmol) following the general procedure E to obtain 2-(4-bromophenyl)-2-(4-methylphenoxy)-N-pyridin-2-ylacetamide (89 mg, 90%).
LCMS (*m*/*z*): 399 (M+ 2H)⁺

### Example 35

### 2-(4-Bromophenyl)-2-(4-fluorophenoxy)-N-1,3-thiazol-2-ylacetamide

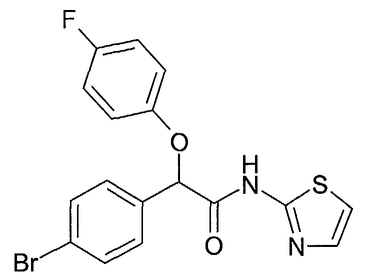

2-(4-Fluorophenoxy)-2-(4-bromophenyl) acetic acid (472 mg, 58%) is prepared from 4-bromomandelic acid methyl ester (490 mg, 2 mmol) and 4-fluorophenol (268 mg, 2.4 mmol) following the general procedure A. A solution of this acid (81 mg, 0.25 mmol) in THF is reacted with 2-aminothiazole (60 mg, 6.0 mmol) following the general procedure E to obtain 2-(4-bromophenyl)-2-(4-fluorophenoxy)-N-1,3-thiazol-2-ylacetamide (73g, 72%).
LCMS (*m*/*z*): 409 (M+ 2H)⁺

### Example 36

### 2-(4-Bromophenyl)-2-phenoxy-N-1,3-thiazol-2-ylacetamide

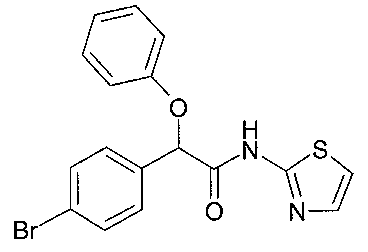

2-Phenoxy-2-(4-bromophenyl) acetic acid (319 mg, 52%) is prepared from 4-bromomandelic acid methyl ester (490 mg, 2 mmol), phenol (226 mg, 2.4mmol) following the general procedure A. A solution of this acid (76.78 mg, 0.25 mmol) in THF is reacted with 2-aminothiazole (60mg, 0.6 mmol) following the general procedure E to obtain 2-(4-bromophenyl)-2-phenoxy-N-1,3-thiazol-2-ylacetamide (82 mg, 85%).
LCMS (*m*/*z*): 391 (M + 2H)⁺

### Example 37

### 2-(4-Fluorophenoxy)-2-(4-fluorophenyl)-N-pyridin-2-ylacetamide

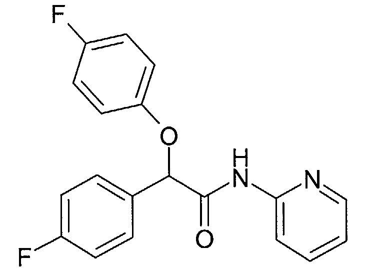

2-(4-Fluorophenoxy)-2-(4-fluorophenyl) acetic acid (317 mg, 60%) is prepared from 4-fluoromandelic acid methyl ester (368 mg, 2 mmol) and 4-fluorophenol (268 mg, 2.4 mmol) following the general procedure A. A solution of this acid (66 mg, 0.25 mmol) in THF is reacted with 2-aminopyridine (5 mg, 0.6 mmol) following the general procedure E to obtain 2-(4-fluorophenoxy)-2-(4-fluorophenyl)-N-pyridin-2-ylacetamide (73 mg, 76%).
LCMS (*m*/*z*): 342 (M +2H)⁺

### Example 38

### 2-(4-Fluorophenoxy)-2-(4-fluorophenyl)-N-1,3-thiazol-2-ylacetamide

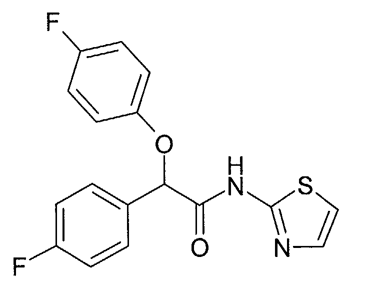

A solution of 2-(4-fluorophenoxy)-2-(4-fluorophenyl) acetic acid (66 mg g, 0.25 mmol) in THF is reacted with 2- aminothiazole (60 mg, 0.6 mmol) following the general procedure E to obtain 2-(4-fluorophenoxy)-2-(4-fluorophenyl)-N-1,3-thiazol-2-ylacetamide (73 mg, 84%).
LCMS (*m*/*z*): 348 (M +2H)⁺

### Example 39

### 2-(4-Fluorophenyl)-2-(4-methylphenoxy)-N-1,3-thiazol-2-ylacetamide

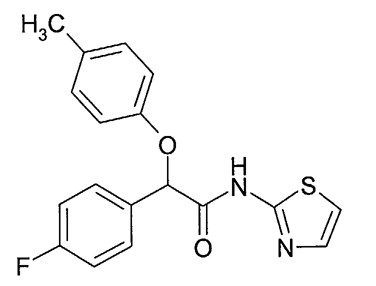

2-(4-Methylphenoxy)-2-(4-fluorophenyl) acetic acid (264 mg, 50%) is prepared from 4-fluoromandalic acid methyl ester (368 mg, 2 mmol) and 4-methylphenol (259 mg, 2.4 mmol) following the general procedure A. A solution of this acid (66 mg, 0.25 mmol) in THF is reacted with 2-aminothiazole (60 mg, 0.6 mmol) following the general procedure E to obtain 2-(4-fluorophenyl)-2-(4-methylphenoxy)-N-1,3-thiazol-2-ylacetamide (69 mg, 80%).
LCMS (*m*)/*z*: 348 (M +2H)⁺

### Example 40

### 2-(4-Fluorophenyl)-2-phenoxy-N-1,3-thiazol-2-ylacetamide

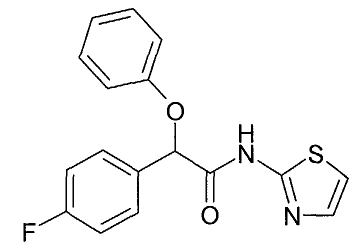

A solution of 2-phenoxy-2-(4-fluorophenyl) acetic acid (62 mg 0.25 mmol) in THF is reacted with 2-aminothiazole (60 mg, 0.6 mmol) following the general procedure E to obtain 2-(4-fluorophenyl)-2-phenoxy-N-1,3-thiazol-2-ylacetamide (62 mg, 75%).
LCMS (*m*/*z*): 330 (M + 2H)⁺

### Example 41

### 2-(4-Bromophenoxy)-2-(4-fluorophenyl)-N-pyridin-2-ylacetamide

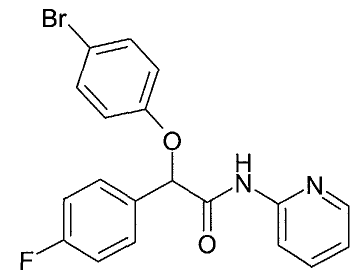

2-(4-Bromophenoxy-2-(4-fluorophenyl) acetic acid (338 mg, 52%) is prepared from 4-fluoromandelic acid methyl ester (368 mg, 2 mmol) and 4-bromophenol (415mg, 2.4 mmol) following the general procedure A. A solution of this acid (81 mg, 0.25 mmol) in THF is reacted with 2-aminopyridine (56 mg, 0.6 mmol) following the general procedure E to obtain 2-(4-bromophenoxy)-2-(4-fluorophenyl)-N-pyridin-2-ylacetamide (86 mg, 86%).
LCMS (*m*/*z*): 403 (M + 2H) ⁺

### Example 42

### 2-(4-Fluorophenoxy)-N-1,3-thiazol-2-yl-2-[4-(trifluoromethyl)phenyl]acetamide

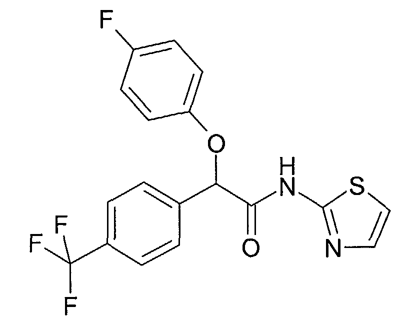

2-(4-Fluorophenoxy-2-(4-trifluoromethylphenyl) acetic acid (390 mg, 62%) is prepared from 4-trifluoromethylmandelic acid methyl ester (468 mg, 2 mmol) and 4-fluorophenol (269 mg, 2.4 mmol) following the general procedure A. A solution of this acid (79 mg, 0.5 mmol) in THF is reacted with 2-aminothiazole (60 mg, 0.6 mmol) following the general procedure E to obtain 2-(4-fluorophenoxy)-N-1,3-thiazol-2-yl-2-[4-(trifluoromethyl)phenyl]-acetamide (87 mg, 88%).
LCMS (*m*/*z*): 398 (M + 2H) ⁺

### Example 43

### 2-(4-Bromophenoxy)-N-pyridin-2-yl-2-[4-(trifluoromethyl)phenyl]acetamide

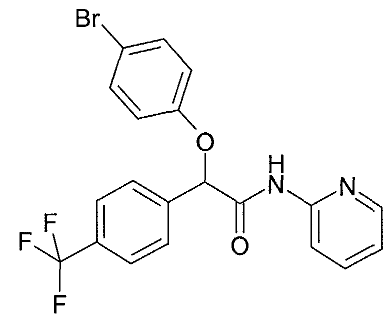

2-(4-Bromophenoxy-2-(4-trifluoromethylphenyl) acetic acid (405 mg, 54%) is prepared from 4-trifluoromethylmandelic acid methyl ester (468 mg, 2 mmol) and 4-bromophenol (415 mg, 2.4 mmol) following the general procedure A. A solution of this acid (94 mg, 0.25 mmol) in THF is reacted with 2-aminopyridine (56 mg, 0.6 mmol) following the general procedure E to obtain 2-(4-bromophenoxy)-N-pyridin-2-yl-2-[4-(trifluoromethyl)phenyl]-acetamide (102 mg, 90%).
LCMS (*m*/*z*): 453 (M+2H)⁺

### Example 44

### 2-Cyclopentylsulfanyl-2-(3,4-dichloro-phenyl)-N-thiazol-2-yl-acetamide

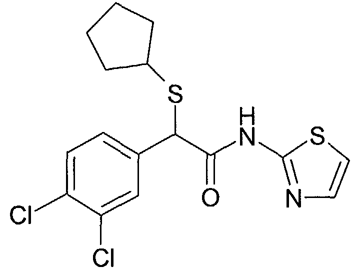

2-(Cyclopentylthio)-2-(3,4-dichloro phenyl) acetic acid (458 mg, 75%) is prepared from 2-bromo-2-(3,4-dichloro phenyl) acetic acid methyl ester (594 mg, 2 mmol) and cyclopentane thiol (245 mg, 2.4 mmol) following the general procedure C. A solution of this acid (76 mg, 0.25 mmol) in THF is reacted with 2-aminothiazole (60 mg, 0.6 mmol) following the general procedure E to obtain 2-cyclopentylsulfanyl-2-(3,4-dichloro-phenyl)-N-thiazol-2-yl-acetamide (87 mg, 90%).
LCMS (*m*/*z*): 388 (M + 2H) ⁺

### Example 45

### 2-Cyclopentylsulfanyl-2-(3,4-dichloro-phenyl)-N-pyridinyl-2-yl-acetamide

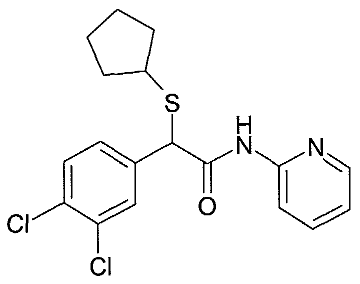

A solution of 2-(cyclopentylthio)-2-(3,4-dichloro phenyl) acetic acid (76 mg, 0.25 mmol) in THF is reacted with 2-aminopyridine (56 mg, 0.6 mmol) following the general procedure E to obtain 2-cyclopentylsulfanyl-2-(3,4-dichloro-phenyl)-N-pyridinyl-2-yl-acetamide (84 mg, 88%).
LCMS (*m*/*z*): 382 (M + 2H) ⁺

### Example 46

### 2-Cyclopentylsulfanyl-2-(3,4-dichloro-phenyl)-N-pyrimidin-2-yl-acetamide

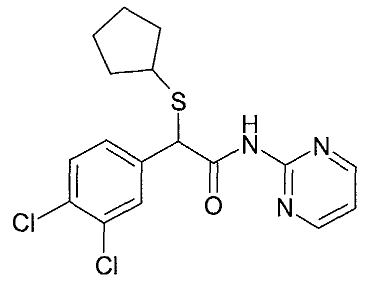

A solution of 2-(cyclopentylthio)-2-(3,4-dichloro phenyl) acetic acid (76 mg, 0.25 mmol) in THF is reacted with 2-aminopyrimidine (57 mg, 0.6 mmol) following the general procedure E to obtain 2-cyclopentylsulfanyl-2-(3,4-dichloro-phenyl)-N-pyrimidin-2-yl-acetamide (81 mg, 85%).
LCMS (*m*/*z*): 383 (M+H)⁺

### Example 47

### N-(5-bromo-1,3-thiazol-2-yl)-2-(cyclopentylthio)-2-(3,4-dichlorophenyl)acetamide

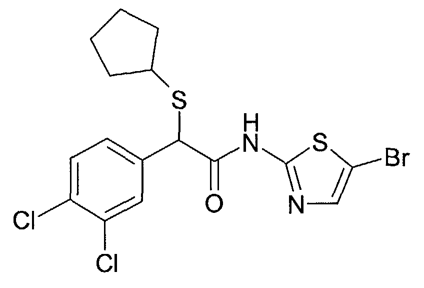

A solution of 2-(Cyclopentylthio)-2-(3,4-dichloro phenyl) acetic acid (76 mg, 0.25 mmol) in THF is reacted with 2-amino-5-bromo-thiazole (107 mg, 0.6 mmol) following the general procedure E to obtain N-(5-bromo-1,3-thiazol-2-yl)-2-(cyclopentylthio)-2-(3,4-dichlorophenyl)acetamide (93 mg, 80%).
LCMS 467 (*m*/*z*): (M + 2H)⁺

### Example 48

### 2-(Cyclopentylthio)-2-(3,4-dichlorophenyl)-N-1,3,4-thiadiazol-2-ylacetamide

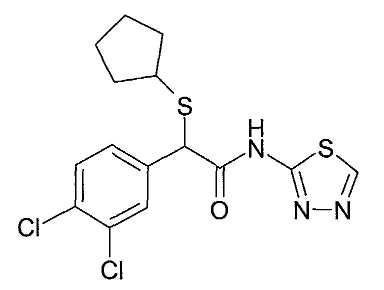

A solution of 2-(cyclopentylthio)-2-(3,4-dichloro phenyl) acetic acid (76 mg, 0.25 mmol) in THF is reacted with 2-amino thiadiazole (120 mg, 1.2 mmol) following the general procedure E to obtain 2-(cyclopentylthio)-2-(3,4-dichlorophenyl)-N-1,3,4-thiadiazol-2-ylacetamide (79 mg, 82%).
LCMS (*m*/*z*): 389 (M + 2H) ⁺

### Example 49

### 2-(3,4-Dichlorophenyl)-2-(isobutylthio)-N-pyridin-2-ylacetamide

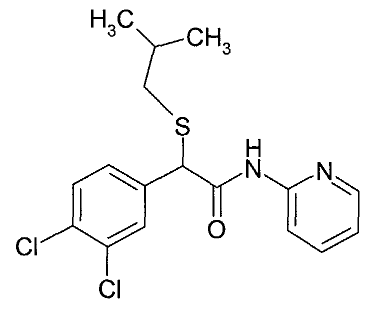

2-(2-Methylpropanethio)-2-(3,4-dichloro phenyl) acetic acid (457 mg, 78%) is prepared from 2-bromo-2-(3,4-dichloro phenyl) acetic acid methyl ester (594 mmol, 2 mmol) and 2-methylpropanethiol (216 mg, 2.4 mmol) following the general procedure C. A solution of this acid (73 mg, 0.25 mmol) in THF is reacted with 2-aminopyridine (56 mg, 0.6 mmol) following the general procedure E to obtain 2-(3,4-dichlorophenyl)-2-(isobutylthio)-N-pyridin-2-ylacetamide (69 mg, 88%).
LCMS (*m*/*z*): 315 (M+2H)⁺

### Example 50

### 2-(3,4-Dichlorophenyl)-2-(isobutylthio)-N-1,3-thiazol-2-ylacetamide

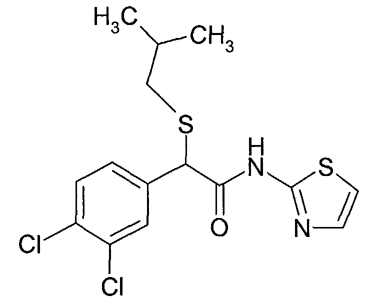

A solution of 2-(2-methylpropanethio)-2-(3,4-dichloro phenyl) acetic acid (73 mg 0.25 mmol) in THF is reacted with 2-aminothiazole (60 mg, 06 mmol) following the general procedure E to obtain 2-(3,4-dichlorophenyl)-2-(isobutylthio)-N-1,3-thiazol-2-ylacetamide (72 mg, 90%).
LCMS (*m*/*z*): 320 (M + 2H)⁺

### Example 51

### 2-(3,4-Dichlorophenyl)-2-[(2-furylmethyl)thio]-N-pyridin-2-ylacetamide

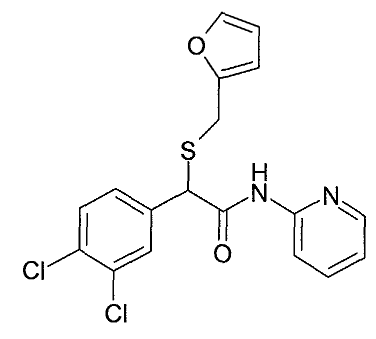

2-(2-Furanylmethylthio)-2-(3,4-dichloro phenyl) acetic acid (482 mg, 76%) is prepared from 2-bromo-2-(3,4-dichloro phenyl) acetic acid methyl ester (594 mg, 2 mmol) and 2-furanylmethylthiol (274 mg, 2.4 mmol) following the general procedure C. A solution of this acid (79 mg, 0.25 mmol) in THF is reacted with 2-aminopyridine (56 mg, 0.6 mmol) following the general procedure E to obtain 2-(3,4-dichlorophenyl)-2-[(2-furylmethyl)thio]-N-pyridin-2-ylacetamide (80 mg, 82%).
LCMS (*m*/*z*): 394 (M +2 H)⁺

### Example 52

### 2-(3,4-Dichlorophenyl)-2-[(2-furylmethyl)thio]-N-1,3-thiazol-2-ylacetamide

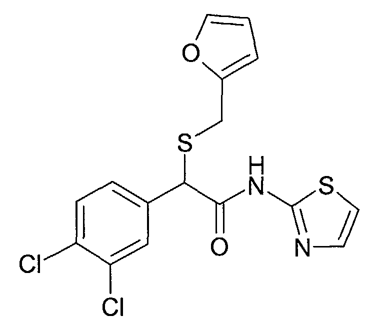

A solution of 2-(2-furanylmethylthio)-2-(3,4-dichloro phenyl) acetic acid (79 mg 0.25 mmol) in THF is reacted with 2-aminothiazole (60 mg, 0.62 mmol) following the general procedure E to obtain 2-(3,4-dichlorophenyl)-2-[(2-furylmethyl)thio]-N-1,3-thiazol-2-ylacetamide (85 mg, 85%).
LCMS (*m*/*z*): 400 (M + 2H) ⁺

### Example 53

### 2-(3,4-Dichlorophenyl)-2-[(2-furylmethyl)thio]-N-pyridin-2-ylacetamide

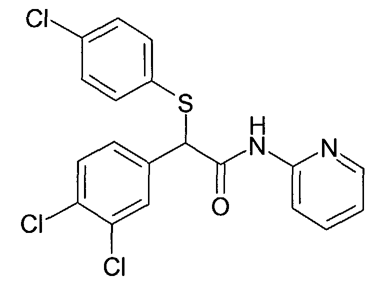

2-(4-Chlorophenlythio)-2-(3,4-dichlorophenyl)acetic acid (542 g, 78%) is prepared from 2-bromo-2-(3,4-dichlorophenyl)acetic acid methyl ester (594 mg, 2 mmol), 4-chlorobenzenethiol (347mg, 2.4 mmol) following the general procedure C. A solution of this acid (87g, 0.25 mmol) in THF is reacted with 2-aminopyridine (56 mg, 0.6 mmol) following the general procedure E to obtain 2-(3,4-dichlorophenyl)-2-[(2-furylmethyl)thio]-N-pyridin-2-yl-acetamide (85 mg, 80%).
LCMS (*m*/*z*): 424 (M+2H)⁺

### Example 54

### 2-[(4-Fluorophenyl)thio]-N-pyridin-2-yl-2-[4-(trifluoromethyl)phenyl]acetamide

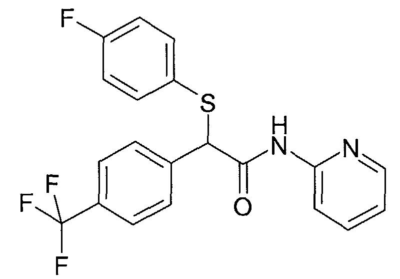

2-(4-Fluorophenlythio)-2-(4-trifluoromethylphenyl)acetic acid (760 mg, 92%) is prepared from 2-hydroxy-2-(4-trifluoromethyl phenyl)acetic acid methyl ester (860 mg, 2.5 mmol) and 4-fluorobenzenethiol (308 mg, 2.4 mmol) following the general procedure D. A solution of this acid (165 mg, 0.50 mmol) in THF is reacted with 2-aminopyridine (56 mg, 0.6 mmol) following the general procedure E to obtain 2-[(4-fluorophenyl)thio]-N-pyridin-2-yl-2-[4-(trifluoromethyl)phenyl]acetamide ( 0.12 g, 59%) as a solid.
LCMS (*m*/*z*): 408 (M +2H)⁺

### Example 55

### 2-[(4-Fluorophenyl)thio]-N-1,3-thiazol-2-yl-2-[4-(trifluoromethyl)phenyl]acetamide

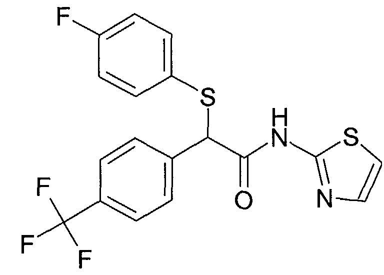

A solution of 2-(4-fluorophenlythio)-2-(4-trifluoromethyl phenyl) acetic acid (165 mg, 0.50 mmol) in THF is reacted with 2-aminothiazole (500 mg, 0.5 mmol) following the general procedure E to obtain 2-[(4-fluorophenyl)thio]-N-1,3-thiazol-2-yl-2-[4-(trifluoromethyl)phenyl]-acetamide ( 93 mg, 45%) as a solid.
LCMS (*m*/*z*): 414 (M+2H)⁺

### Example 56

### 2-[(4-Methylphenyl)thio]-N-1,3-thiazol-2-yl-2-[4-(trifluoromethyl)phenyl]acetamide

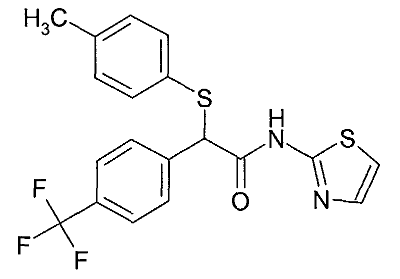

A solution of 2-(4-methylphenlythio)-2-(4-trifluoromethylphenyl)acetic acid (160 mg, 0.50 mmol) in THF is reacted with 2-aminothiazole (0.50 g, 0.50 mmol) following the general procedure E to obtain 2-[(4-methylphenyl)thio]-N-1,3-thiazol-2-yl-2-[4-(trifluoromethyl)phenyl]-acetamide (94 mg, 45%).
LCMS (*m*/*z*): 410 (M +2H)⁺

### Example 57

### 2-(4-Fluorophenyl)-2-[(4-fluorophenyl)thio]-N-pyridin-2-ylacetamide

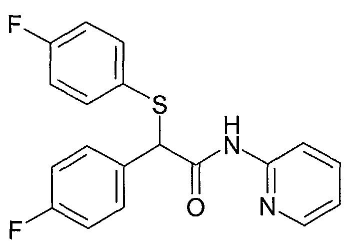

2-(4-Fluorophenlythio)-2-(4-fluorophenyl)acetic acid (400 mg, 75%) is prepared from 2-hydroxy-2-(4-fluorophenyl)acetic acid methyl ester (368 mg, 2 mmol) and 4-fluorobenzenethiol (307 mg, 2.4mmol) following the general procedure D. A solution of this acid (66 mg, 0.25 mmol) in THF is reacted with 2-aminopyridine (56 mg, 0.6 mmol) following the general procedure E to obtain 2-(4-fluorophenyl)-2-[(4-fluorophenyl)thio]-N-pyridin-2-ylacetamide (73 mg, 82%).
LCMS (*m*/*z*): 358 (M + 2 H)⁺

### Example 58

### 2-(4-Bromophenyl)-2-[(4-fluorophenyl)thio]-N-pyridin-2-ylacetamide

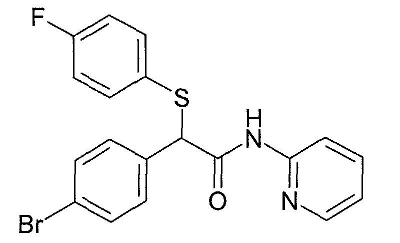

2-(4-Fluorophenlythio)-2-(4-bromophenyl) acetic acid (593 mg, 87%) is prepared from 2-hydroxy-2-(4-bromophenyl) acetic acid methyl ester (490 mg, 2 mmol) and 4-fiuorobenzenethiol (307 mg, 2.4 mmol) following the general procedure D. A solution of this acid (85 mg, 0.25 mmol) in THF is reacted with 2-aminopyridine (56 mg, 0.6 mmol) following the general procedure E to obtain 2-(4-bromophenyl)-2-[(4-fluorophenyl)thio]-N-pyridin-2-ylacetamide (88 mg, 85%).
LCMS (*m*/*z*): 419 (M + 2 H)⁺

### Example 59

### 2-(4-Bromophenyl)-2-[(4-methylphenyl)thio]-N-pyridin-2-ylacetamide

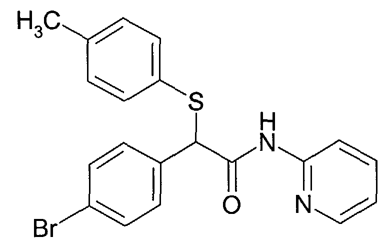

2-(4-Methylphenlythio)-2-(4-bromophenyl)acetic acid (559 mg, 83%) is prepared from 2-hydroxy-2-(4-bromophenyl) acetic acid methyl ester (490 mg, 2 mmol) and 4-methylbenzenethiol (298 mg, 2.4 mmol) following the general procedure D. A solution of this acid (84 mg, 0.25 mmol) in THF is reacted with 2-aminopyridine (56 mg, 0.6 mmol) following the general procedure E to obtain 2-(4-bromophenyl)-2-[(4-methylphenyl)thio]-N-pyridin-2-ylacetamide (85 mg, 82%).
LCMS (*m*/*z*): 415 (M +2 H) ⁺

### Example 60

### 1-Cyclopentylmethyl-1-(3,4-dichloro-phenyl)-3-thiazol-2-yl-urea

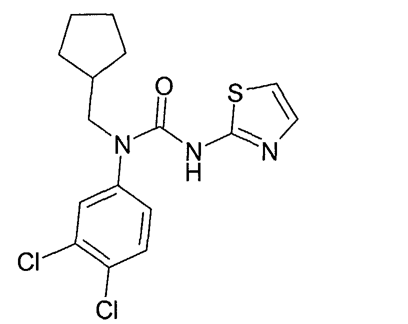

3,4-Dichloroaniline (16.1 g, 100 mmol) is dissolved in dry THF (200 ml) in a round-bottomed flask, and then cyclopenthylcarboxyaldehyde (9.81 g, 100 mmol) is added to the solution and stirred for 10 min at rt. Molecular sieves (4 g, 4 A⁰) is added to the reaction mixture followed by addition of sodium triacetoxyborohydride (21.1 g, 200 mmol) and the mixture stirred at 25°C for 12 h. The reaction mixture is then filtered through silica gel, washed with saturated sodium bicarbonate (200 ml) and extracted with ethyl acetate (3x200 ml). The organic extracts are combined, dried (sodium sulfate), filtered, and condensed *in vacuo* to give *N*-cyclopenthylmethyl-3,4-dichloroaniline (23. 8 g, 98.7%) as an oil (LC-MS:
*m*/*z* = 245 (M+1), Rₜ = 2.92 min).

*N*-cyclopentylmethyl-3,4-dichloroaniline (1.21 g, 5.00 mmol) is dissolved in dry DCM (25 ml) and triethylamine (1.4 ml, 10.0 mmol) is added to the solution. Then triphosgene (2.97 g, 10.0 mmol) is added at -20°C and resulting mixture is stirred and allowed to warm up to 25°C within 3 h. On warming to rt., 2-aminothiazole (0.50 g, 5.0 mmol) is added to the reaction mixture and stirred for 12 h. Followed by TLC and LC-MS, upon consumption of starting material, the mixture is washed with water (50 ml) and extracted with ethyl acetate (3x50 ml). The organic extracts are combined, dried (sodium sulfate), filtered, and condensed *in vacuo* to give 1-cyclopentyl-1-(3,4-dichloro-phenyl)-3-thiazol-2-yl-urea (1.42 g, 76.5%) as a pale yellow solid.
LC-MS: *m*/*z* 371 (M+1)⁺

### Example 61

### 1-Cyclopentyl-1-(3,4-dichloro-phenyl)-3-thiazol-2-yl-urea

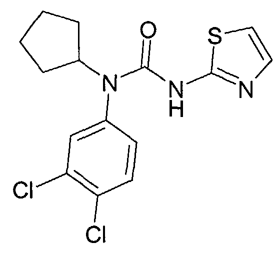

Sodium triacetoxyborohydride (5.3 g, 25.0 mmol) is added batchwise to a stirred solution of 3,4-dichloroaniline (1.62 g, 10.0 mmol), cyclopentanone (1.80 mL, 20.0 mmol) and acetic acid (3.4 mL, 60.0 mmol) in 75 mL 1,2-dichloroethane. The resulting suspension is stirred for 48 h at 25°C. The reaction mixture is given an aqueous workup with aqueous sodium bicarbonate and ethyl acetate. The combined organic extracts are dried and concentrated in vacuo to give N-cyclopentyl-3,4-dichloroaniline which is used without further purification.

Carbonyldiimidazole (178 mg, 1.1 mmol) is added to a THF solution of N-cyclopentyl-3,4-dichloroaniline (230 mg, 1.0 mmol). The mixture is stirred for 1 h at 25°C, after which time 2-aminothiazole (100 mg, 1.0 mmol) is added. The reaction is then stirred for 16 h at 25°C The reaction mixture is concentrated and residue purified by silica gel chromatography to afford 1-cyclopentyl-1-(3,4-dichloro-phenyl)-3-thiazol-2-yl-urea as a solid (30 mg, 10% yield).
LC-MS: *m*/*z* 379 (M+1)⁺

### Example 62

### 1-(3,4-Dichloro-phenyl)-1-propyl-3-thiazol-2-yl-urea

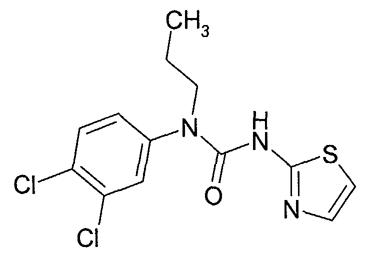

2-Aminothiazole (5.0 g, 50.0 mmol) is dissolved in DCE (20 ml), then solid supported 2-(4-formyl-3-methoxyphenoxy) ethyl polystyrene (6.9 g, 10.0 mmol, loading: 1.46 mmol/g) is added to the solution and stirred for 30 min. Acetic acid (2.1 ml, 5.0 mmol) is added to the mixture followed by addition of sodium triacetoxyborohydride (10.56 g, 50.0 mmol). The resin mixture is shaken at rt for 16 h, then washed with three cycles of DMF/methanol/DCM. Then the resin is dried *in vacuo* to give solid-supported 2-aminothiazole.

The corresponding above solid-supported starting material 2-amino thiazoyl-N-2-(3-methoxybenzyloxy)ethyl polystyrene (5.0 g, 7.3 mmol) is treated with triphosgene (5.30 g, 17. 8 mmol) in the presence of DCM (25 ml) and diisopropylethylamine (7.63 ml, 43.8 mmol). The product on solid support is then washed with DCM. Then the resin is dried *in vacuo* to give corresponding carbamyl chloride *N* [2-(3-methoxybenzyloxy)ethyl polystyrene-2-amino thiazoyl chloride.

The corresponding carbamyl chloride resin (1.0 g, 1.46 mmol) is treated with 3,4-dichloroaniline (1.17 g, 7.30 mmol) in the presence of DCE (25 ml) and diisopropylethylamine (2.54 ml, 14.6 mmol). The product on solid support is then washed with three cycles of DMF/methanol/DCM, dried *in vacuo* to give corresponding urea *N* [2-(3-methoxybenzyloxy) ethyl polystyrene-2-amino thiazoyl-*N*'-3,4-dichlorophenyl urea.

To the corresponding urea (*N* [2-(3-methoxybenzyloxy) ethyl polystyrene-2-amino thiazoyl-3,4-dichlorophenyl urea) (0.117 g, 0.73 mmol) is added potassium *t*-butoxide (3.65 ml, 3.65 mmol, 1M solution in THF). The resin mixture is shaken for 1 h at rt, and then 1-bromopropane (0.332 ml, 3.65 mmol) is added to the resin mixture and shaken for 16 h. The product on solid support is then washed with three cycles of DMF/methanol/DCM, dried *in vacuo* to give corresponding propylated urea *N* [2-(3-methoxybenzyloxy) ethyl polystyrene-2-amino thiazoyl, *N*'-(3,4-dichlorophenyl)-*N*'- propyl urea.

The corresponding above urea is then treated with TFA (5 ml, 5% DCM solution) to cleave the solid support to give the title compound 1-(3,4-dichloro-phenyl)-1-propyl-3-thiazol-2-yl-urea (0.163 g, 67.9%) as a pale yellow solid.
LC-MS: *m*/*z* 331 (M+1)⁺

### Example 63

### 2-Cyclopentyl-1-(3,4-dichlorophenyl)ethylpyridin-2-ylcarbamate

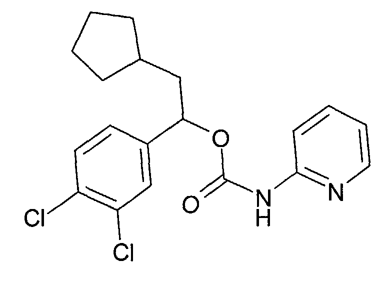

To a solution of cyclopentyl acetic acid (0.64 g, 5.00 mmol) in DCM (50 ml) is added *N,O*-dimethylhydroxyl amine hydrochloride (0.80 g, 5.00 mmol) and triethylamine (0.696 ml, 5.00 mmol). The mixture is stirred at rt for 5 min. DCC (1.03 g, 5.00 mmol) is added to solution. After 3 h the solution is then oncentrated *in vacuo*, the residue is suspended in a minimum of acetone and insoluble white solid is removed by filtration. The filtrate is evaporated to dryness. Silica gel chromatography with hexanes - ethyl acetate (95:5 to 70:30) as eluent afforded *N,O*-dimethyl cyclopentylamide (0.72 g, 84.2%) as white solid.
LC-MS: *m*/*z* 172 (M+1)⁺.

Metallic magnesium (0.073 g, 3.00 mmol) in THF (25 ml) is treated with 1-bromo-3,4-dichlorobenzene (0.565 g, 2.50 mmol). A catalytic amount of iodine (0.005 g) is added, and the solution is stirred at rt until orange color disappeared. *N,O*-dimethyl cyclopentylamide (0.43 g, 2.50 mmol) is added to the reaction mixture. After 4 h the mixture is given an aqueous workup. Concentration in vacuo afforded 1-(3,4-dichlorophenyl)-2-cyclopenthyl ethanol (0.613 g, 94.5%) as an oil.

1-(3,4-Dichlorophenyl)-2-cyclopenthyl ethanol (0.259 g, 1.0 mmol) in DCM (10 ml) is treated with TEA (0.2 ml, 1.50 mmol) and phosgene (0.75 ml, 1.50 mmol, in 20% toluene) at -20 °C. After 3 h excess phosgene and triethylamine are removed *in vacuo* to give the desired product 1-(3,4-dichlorophenyl) 2-cyclopentylethyl chloroformate (0.25 g, 77.6%) as an oil.

To a solution of 1-(3,4-dichlorophenyl)-2-cyclopentylethyl chloroformate (0.16 g, 0.50 mmol) in DCM (5 ml) is added DIEA (0.17 ml, 1.0 mmol) and 2-aminopyridine (0.047 g, 0.5 mmol). The mixture is given an aqueous workup. Concentration in vacuo affords 2-cyclo-pentyl-1-(3,4-dichlorophenyl)ethyl pyridin-2-ylcarbamate (0.153 g, 80.9%) as an oil.
LC-MS: *m*/*z* 380 (M+1)⁺

### Example 64

### 2-Cyclopentyl-1-(3,4-dichlorophenyl)ethyl 1,3-thiazol-2-ylcarbamate

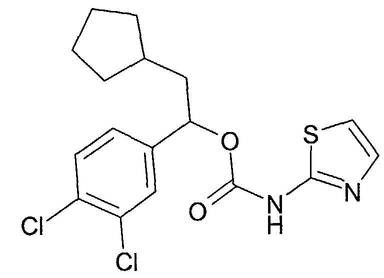

1-(3,4-dichlorophenyl)-2-cyclopentylethyl chloroformate (0.16 g, 0.50 mmol) is synthesized from 1-(3,4-dichlorophenyl)-2-cyclopenthylethanol according to general procedure T. The chloroformate is subjected to general procedure U employing 2-aminothiazole to afford 2-cyclopentyl-1-(3,4-dichlorophenyl)ethyl 1,3-thiazol-2-ylcarbamate (0.175 g, 90.9%) as an oil.
LC-MS: *m*/*z* 386 (M+1)⁺

### BIOLOGICAL ASSAY

### Glucokinase Activity Assay (I)

Glucokinase activity is assayed spectrometrically coupled to glucose 6-phosphate dehydrogenase to determine compound activation of glucokinase. The final assay contains 50 mM Hepes, pH 7.1, 50 mM KCI, 5 mM MgCl₂, 2 mM dithiothreitol, 0.6 mM NADP, 1 mM ATP, 0.195 µM G-6-P dehydrogenase (from Sigma), 15 nM recombinant human glucokinase. The glucokinase is human liver glucokinase N-terminally truncated with an N-terminal His-tag ((His)₈-VEQILA.....Q466) and is expressed in E.coli as a soluble protein with enzymatic activity comparable to liver extracted GK.

The compound to be tested is added into the well in final 2.5% DMSO concentration in an amount sufficient to give a desired concentration of compound, for instance 50 µM. The reaction starts after glucose is added to a final concentration of 2 mM. The assay uses a 96-well UV plate and the final assay volume used is 200 µl/well. The plate is incubated at 25°C for 5 min and kinetics is measured at 340 nm in SpectraMax every 30 seconds for 5 minutes. Results for each compound are expressed as the fold activation of the glucokinase activity compared to the activation of the glucokinase enzyme in an assay without compound after having been subtracted from a "blank", which is without glucokinase enzyme and without compound. The compounds in each of the Examples exhibits activation of glucokinase in this assay. A compound, which at a concentration of at or below 30 µM gives 1.3 - fold higher glucokinase activity than the result from the assay without compound, is deemed to be an activator of glucokinase.

While the invention has been described and illustrated with reference to certain preferred embodiments thereof, those skilled in the art will appreciate that various changes, modifications and substitutions can be made therein without departing from the spirit and scope of the invention. For example, effective dosages other than the preferred dosages as set forth herein may be applicable as a consequence of variations in the responsiveness of the mammal being treated for glucokinase-deficiency mediated disease(s). Likewise, the specific pharmacological responses observed may vary according to and depending on the particular active compound selected or whether there are present pharmaceutical carriers, as well as the type of formulation and mode of administration employed, and such expected variations or differences in the results are contemplated in accordance with the objects and practices of the present invention.

## Claims

1. A carboxamide or sulfonamide activator of glucokinase having a heteroatom in the alpha, beta, or gamma position relative to the carboxamide or sulfonamide, respectively.

2. A compound of the general formula (I) 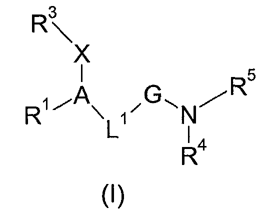 wherein
G is -S(O₂)-, or -C(O)-;
A is >N-, and
X is a direct bond, -O-, -S-, -S(O)-, -S(O₂)-, or -N(R⁶) -, wherein
R⁶ is hydrogen or alkyl, which may optionally be substituted with one or more substituents R¹⁶, R¹⁷, and R¹⁸, and
L¹ is -(CH₂)ₙ-C(R⁹)(R¹⁰)ₘ-Y-, or a direct bond, wherein
n is an integer of from 1 to 6,
R⁹ and R¹⁰ independently are selected from alkyl, or cycloalkyl, optionally substituted by one or more substituents R¹⁹, R²⁰, and R²¹; or from aryl optionally substituted by one or more substituents R⁴⁰, R⁴¹, R⁴², and R⁴³,
m is an integer of 0 to 1, and
Y is a direct bond, -O- or -N(R⁷)-, wherein
R⁷ is hydrogen or alkyl, which may optionally be substituted with one or more substituents R²², R²³, and R²⁴;
or
X is alkylene, which may optionally be substituted with one or more substituents R²⁵, R²⁶, and R²⁷, or a direct bond, and
L¹ is -O-, or -N(R⁸)-, wherein
R⁸ is hydrogen or alkyl, which may optionally be substituted with one or more substituents R²⁸, R²⁹, and R³⁰;
or
A is >C(R²)-, wherein R² is hydrogen or alkyl, optionally substituted with one or more substituents R³¹, R³²_{,} and R³³, and
X is -O-, -S-, -S(O)-, -S(O₂)-, or -N(R⁶)-, wherein
R⁶ is as defined above, and
L¹ is -(CH₂)ₙ-Y-, or a direct bond, wherein
n is an integer of from 1 to 6, and
Y is a direct bond, O, or -N(R⁷)-, wherein
R⁷ is as defined above;
or
X is alkylene, which may optionally be substituted with one or more substituents R²⁵, R²⁶, and R²⁷, or a direct bond, and
L¹ is -O-, or -N(R⁸)-, wherein
R⁸ is as defined above;
R¹ and R³ independently are selected from alkyl, alkenyl, alkynyl, cycloalkyl, and heterocyclyl, optionally substituted with one or more substituents R³⁴, R³⁵, and R³⁶; or from aryl, heteroaryl, fused heterocyclylaryl, fused heteroarylheterocyclyl, fused heterocyclylheteroaryl, fused arylcycloalkyl, fused cycloalkylaryl, fused heteroarylcycloalkyl, and fused cycloalkylheteroaryl, optionally substituted with one or more substituents R⁴⁴, R⁴⁵, R⁴⁶, and R⁴⁷;
or
R¹ and R³ may be taken together with the atoms to which they are attached to form a cycloalkyl or heterocyclyl ring, optionally substituted with one or more substituents R³⁴, R³⁵, and R³⁶, and optionally fused to a heteroaryl or aryl ring, optionally substituted with one or more substituents R⁴⁴, R⁴⁵, R⁴⁶, and R⁴⁷;
or, when A is >C(R²)-, then
R¹ and R² may be taken together with the atoms to which they are attached to form a cycloalkyl or heterocyclyl ring, optionally substituted with one or more substituents R³⁴, R³⁵, and R³⁶, and optionally fused to a heteroaryl or aryl ring, optionally substituted with one or more substituents R⁴⁴, R⁴⁵, R⁴⁶, and R⁴⁷;
or
R² and R³ may be taken together with the atoms to which they are attached to form a cycloalkyl or heterocyclyl ring, optionally substituted with one or more substituents R³⁴, R³⁵, and R³⁶, and optionally fused to a heteroaryl or aryl ring, optionally substituted with one or more substituents R⁴⁴, R⁴⁵, R⁴⁶, and R⁴⁷;
R⁴ is hydrogen or alkyl, optionally substituted with one or more substituents R³⁷, R³⁸, and R³⁹; and
R⁵ is aryl or heteroaryl, optionally substituted with one or more substituents R⁴⁸, R⁴⁹, R⁵⁰, and R⁵¹;
wherein
R¹⁶, R¹⁷, R¹⁸, R¹⁹, R²⁰, R²¹, R²², R²³, R²⁴; R²⁵, R²⁶, R²⁷ R²⁸, R²⁹, R³⁰; R³¹, R³², R³³, R³⁴, R³⁵, R³⁶, R³⁷, R³⁸, and R³⁹ independently are selected from
-CHF₂, -CF₃, -OCF₃, -OCHF₂, -OCH₂CF₃, -OCF₂CHF₂, -S(O)₂CF₃, -SCF₃, -OR⁵², -NR⁵²R⁵³, -SR⁵², -NR⁵²S(O)₂R⁵³, -S(O)₂NR⁵²R⁵³, -S(O)NR⁵²R⁵³, -S(O)R⁵², -S(O)₂R⁵², -C(O)NR⁵²R⁵³, -OC(O)NR⁵²R⁵³, -NR⁵²C(O)R⁵³, -CH₂C(O)NR⁵²R⁵³, -OCH₂C(O)NR⁵²R⁵³, -CH₂OR⁵², -CH₂NR⁵²R⁵³, -OC(O)R⁵², -C(O)R⁵² and -C(O)OR⁵²; C₂₋₆-alkenyl and C₂₋₆-alkynyl, which may optionally be substituted with one or more substituents selected from -CN, -CF₃, -OCF₃, -OR⁵², -NR⁵²R⁵³ and C₁₋₆-alkyl;
C₃₋₈-cycloalkyl, C₄₋₈-cycloalkenyl, heterocyclyl, C₃₋₈-cycloalkyl-C₁₋₆-alkyl, C₃₋₈-cycloalkyl-C₁₋₆-alkoxy, C₃₋₈-cycloalkyloxy, C₃₋₈-cycloalkyl-C₁₋₆-alkylthio, C₃₋₈-cycloalkylthio, C₃₋₈-cycloalkyl-C₂₋₆-alkenyl, C₃₋₈-cycloalkyl-C₂₋₆-alkynyl, C₄₋₈-cycloalkenyl-C₁₋₆-alkyl, C₄₋₈-cycloalkenyl-C₂₋₆-alkenyl, C₄₋₈-cycloalkenyl-C₂₋₆-alkynyl, heterocyclyl-C₁₋₆-alkyl, heterocyclyl-C₂₋₆-alkenyl, heterocyclyl-C₂₋₆-alkynyl, aryl, aryloxy, aryloxycarbonyl, aroyl, aryl-C₁₋₆-alkoxy, aryl-C₁₋₆-alkyl, aryl-C₂₋₆-alkenyl, aryl-C₂₋₆-alkynyl, heteroaryl, heteroaryl-C₁₋₆-alkyl, heteroaryl-C₂₋₆-alkenyl and heteroaryl-C₂₋₆-alkynyl, of which the aryl and heteroaryl moieties optionally may be substituted with one or more substituents selected from halogen, -C(O)OR⁵², -CN, -CF₃, -OCF₃, -NO₂, -OR⁵², -NR⁵²R⁵³ and C₁₋₆-alkyl; and
R⁴⁰, R⁴¹, R⁴², R⁴³, R⁴⁴, R⁴⁵, R⁴⁶, and R⁴⁷ independently are selected from
halogen, -CN, -CH₂CN, -CHF₂, -CF₃, -OCF₃, -OCHF₂, -OCH₂CF₃, -OCF₂CHF₂, -S(O)₂CF₃, -SCF₃, -NO₂, -OR⁵², -NR⁵²R⁵³, -SR⁵², -NR⁵²S(O)₂R⁵³, -S(O)₂NR⁵²R⁵³, -S(O)NR⁵²R⁵³, -S(O)R⁵², -S(O)₂R⁵², -C(O)NR⁵²R⁵³, -OC(O)NR⁵²R⁵³, -NR⁵²C(O)R⁵³, -CH₂C(O)NR⁵²R⁵³, -OCH₂C(O)NR⁵²R⁵³, -CH₂OR⁵², -CH₂NR⁵²R⁵³, -OC(O)R⁵², -C(O)R⁵², and -C(O)OR⁵²;
C₁₋₆-alkyl, C₂₋₆-alkenyl and C₂₋₆-alkynyl, which may optionally be substituted with one or more substituents selected from -CN, -CF₃, -OCF₃, -OR⁵², -NR⁵²R⁵³, and C₁₋₆-alkyl;
C₃₋₈-cycloalkyl, C₄₋₈-cycloalkenyl, heterocyclyl, C₃₋₈-cycloalkyl-C₁₋₆-alkyl, C₃₋₈-cyclalkyl-C₁₋₆-alkoxy, C₃₋₈-cycloalkyloxy, C₃₋₈-cycloalkyl-C₁₋₆-alkylthio, C₃₋₈-cycloalkylthio, C₃₋₈-cycloalkyl-C₂₋₆-alkenyl, C₃₋₈-cycloalkyl-C₂₋₆-alkynyl, C₄₋₈-cycloalkenyl-C₁₋₆-alkyl, C₄₋₈-cycloalkenyl-C₂₋₆-alkenyl, C₄₋₈-cycloalkenyl-C₂₋₆-alkynyl, heterocyclyl-C₁₋₆-alkyl, heterocyclyl-C₂₋₆-alkenyl, heterocyclyl-C₂₋₆-alkynyl, aryl, aryloxy, aryloxycarbonyl, aroyl, aryl-C₁₋₆-alkoxy, aryl-C₁₋₆-alkyl, aryl-C₂₋₆-alkenyl, aryl-C₂₋₆-alkynyl, heteroaryl, heteroaryl-C₁₋₆-alkyl, heteroaryl-C₂₋₆-alkenyl and heteroaryl-C₂₋₆-alkynyl, of which the aryl and heteroaryl optionally may be substituted with one or more substituents selected from halogen, -C(O)OR⁵², -CN, -CF₃, -OCF₃, -NO₂, -OR⁵², -NR⁵²R⁵³, and C₁₋₆-alkyl;
and
two of R⁴⁰, R⁴¹, R⁴², and R⁴³, or two of R⁴⁴, R⁴⁵, R⁴⁶, and R⁴⁷ on adjacent carbon atoms may independently be taken together to form -O-CH₂-O-;
wherein
R⁵² and R⁵³ independently are hydrogen, C₁₋₆-alkyl, aryl-C₁₋₆-alkyl or aryl;
or
R⁵² and R⁵³,when attached to the same nitrogen atom, together with the said nitrogen atom may form a 3 to 8 membered heterocyclic ring optionally containing one or two further heteroatoms selected from nitrogen, oxygen and sulfur, and optionally containing one or two double bonds; and
R⁴⁸, R⁴⁹, R⁵⁰, and R⁵¹ independently are selected from
halogen, perfluoroalkyl, cyano, alkyl-Z-, aryl-Z-, aryl-alkylene-Z-, N(R⁶³)(R⁶⁴)-alkylene-Z-; and R⁶⁵-W-alkylene-Z-; wherein
Z and W independently are selected from a direct bond, alkylene, -O-, -N(R⁶⁶)-, -S-, -SO₂-, -C(O)N(R⁶⁶)-, -N(R⁶⁶)C(O)-, -N(R⁶⁶)C(O)N(R⁶⁷)-, -N(R⁶⁶)SO₂-, -SO₂N(R⁶⁶)-, -C(O)C-, -OC(O)-, and -N(R⁶⁶)SO₂N(R⁶⁷)-;
wherein
R⁶⁶ and R⁶⁷ independently are hydrogen or alkyl;
R⁶³, R⁶⁴, and R⁶⁵ are selected from the group consisting of hydrogen, aryl, alkyl, and aryl-alkylene-; or
R⁶³ and R⁶⁴ may be taken together to form a ring having the formula -(CH₂)ⱼ-E-(CH₂)ₖ- bonded to the nitrogen atom to which R⁶³ and R⁶⁴ are attached, wherein
j is an integer of from 1 to 4;
k is an integer of from 1 to 4; and
E is a direct bond, -CH₂-, -O-, -S-, -S(O₂)-, -C(O)-, -C(O)N(H)-, -NHC(O)-, -NHC(O)N(H)-, -NHSO₂-, -SO₂NH-, -C(O)O-, -OC(O)-, -NHSO₂NH-, 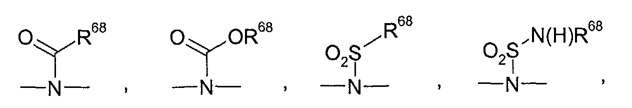 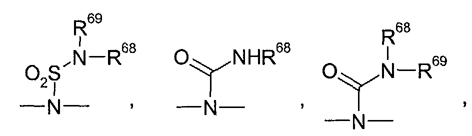 and 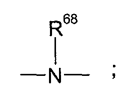
wherein
R⁸⁸ and R⁶⁹ are selected from the group consisting of hydrogen, aryl, alkyl, and aryl-alkylene-;
or a pharmaceutically acceptable salt, solvate or prodrug thereof.

3. A compound according to claim 2, wherein at least one of R¹ and R³ is selected from aryl-, and heteroaryl-, optionally substituted with one or more substituents R⁴⁴, R⁴⁵, R⁴⁶, and R⁴⁷.

4. A compound according to any of claims 2 to 3, wherein R⁵ is selected from the group consisting of thiazole, thiadiazole, isothiazole, pyridine, and pyrimidine; optionally substituted with one or more substituents R⁴⁸, R⁴⁹, R⁵⁰, and R⁵¹, wherein R⁴⁸, R⁴⁹, R⁵⁰, and R⁵¹ are as defined in claim 8.

5. A compound according to claim 4, wherein R⁴⁸, R⁴⁹, R⁵⁰, and R⁵¹ independently are selected from halogen, perfluoroalkyl, cyano, alkyl-Z-, aryl-alkylene-Z-, and R⁶⁵-W-alkylene-Z-; wherein
Z and W independently are selected from a direct bond, -O-, -N(R⁶⁶)-, -N(R⁶⁶)C(O)-, and -OC(O)-; wherein
R⁶⁶ is hydrogen or alkyl; and
R⁶⁵ is hydrogen or alkyl.

6. A compound according to claim 5, wherein R⁴⁸, R⁴⁹, R⁵⁰, and R⁵¹ independently are selected from the group consisting of halogen, perfluoroalkyl, cyano, alkyl-OC(O)-alkylene-, alkyl-NHC(O)-alkylene-, N(alkyl)₂-C(O)-alkylene-, alkyl-OC(O)-, NH(alkyl)-C(O)-, N(alkyl)₂-C(O)-, aryl-alkylene-O-, and aryl-alkylene-NH-.

7. A compound according to claim 2, which compound is of the general formula (II) 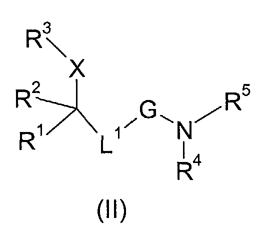 wherein
G is -S(O₂)-, or -C(O)-;
R² is hydrogen or alkyl, which may optionally be substituted with one or more substituents R³¹, R³², and R³³;
X is -O-, -S-, -S(O)-, -S(O₂)-, or -N(R⁶)-, wherein
R⁶ is as defined in claim 2;
L¹ is -(CH₂)ₙ-Y-, or a direct bond, wherein
n is an integer of from 1 to 6,
Y is a direct bond, O, or -N(R⁷)-, wherein
R⁷ is as defined in claim 2;
R¹ and R³ independently are selected from alkyl, alkenyl, alkynyl, cycloalkyl, and heterocyclyl, optionally substituted with one or more substituents R³⁴, R³⁵, and R³⁶; or from aryl, heteroaryl, fused heterocyclylaryl, fused heteroarylheterocyclyl, fused heterocyclylheteroaryl, fused arylcycloalkyl, fused cycloalkylaryl, fused heteroarylcycloalkyl, and fused cycloalkylheteroaryl, optionally substituted with one or more substituents R⁴⁴, R⁴⁵, R⁴⁶, and R⁴⁷;
or
R¹ and R² may be taken together with the atoms to which they are attached to form a cycloalkyl or heterocyclyl ring, optionally substituted with one or more substituents R³⁴, R³⁵, and R³⁶, and optionally fused to a heteroaryl or aryl ring, optionally substituted with one or more substituents R⁴⁴, R⁴⁵, R⁴⁶, and R⁴⁷;
R⁴ is as defined in claim 2; and
R⁵ is as defined in claim 2;
wherein R³¹, R³², R³³, R³⁴, R³⁵, R³⁶, R⁴⁴, R⁴⁵, R⁴⁶, and R⁴⁷ are as defined in claim 2, or a pharmaceutically acceptable salt, solvate or prodrug thereof.

8. A compound according to claim 7, wherein R⁵ is selected from the group consisting of thiazole, thiadiazole, isothiazole, pyridine, and pyrimidine; optionally substituted with one or more substituents R⁴⁸, R⁴⁹, R⁵⁰, and R⁵¹, wherein R⁴⁸, R⁴⁹, R⁵⁰, and R⁵¹ are as defined in claim 2.

9. A compound according to claim 8, wherein R⁴⁸, R⁴⁹, R⁵⁰, and R⁵¹ independently are selected from halogen, perfluoroalkyl, cyano, alkyl-Z-, aryl-alkylene-Z-, and R⁶⁵-W-alkylene-Z-; wherein
Z and W independently are selected from a direct bond, -O-, -N(R⁶⁶)-, -N(R⁶⁶)C(O)-, and -OC(O)-; wherein
R⁶⁶ is hydrogen or alkyl; and
R⁶⁵ is hydrogen or alkyl.

10. A compound according to claim 9, wherein R⁴⁸, R⁴⁹, R⁵⁰, and R⁵¹ independently are selected from the group consisting of halogen, perfluoroalkyl, cyano, alkyl-OC(O)-alkylene-, alkyl-NHC(O)-alkylene-, N(alkyl)₂-C(O)-alkylene-, alkyl-OC(O)-, NH(alkyl)-C(O)-, N(alkyl)₂-C(O)-, aryl-alkylene-O-, and aryl-alkylene-NH-.

11. A compound according to any of claims 7 to 10, wherein X is -S-, -O-, or -N(R⁶)-, wherein R⁶ is as defined in claim 7.

12. A compound according to claim 11, wherein X is -S-, -O-, or -N(R⁶)-, wherein R⁶ is hydrogen or alkyl.

13. A compound according to claim 2, which compound is of the general formula (II) 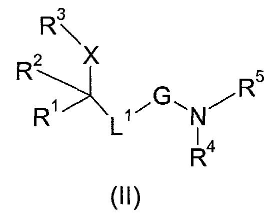 wherein
G is -S(O₂)-, or -C(O)-, and
X is -O-, -S-, -S(O)-, -S(O₂)-, or -N(R⁶)-, wherein
R⁶ is as defined in claim 2, and
L¹ is -(CH₂)ₙ-Y-, or a direct bond, wherein
n is an integer of from 1 to 6, and
Y is a direct bond, O, or -N(R⁷)-, wherein
R⁷ is as defined in claim 2;
or
X is alkylene, which may optionally be substituted with one or more substituents R²⁵, R²⁶, and R²⁷, or a direct bond, and
L¹ is -O-, or -N(R⁸)-, wherein
R⁸ is as defined in claim 2;
R¹ is selected from alkyl, alkenyl, alkynyl, cycloalkyl, and heterocyclyl, optionally substituted with one or more substituents R³⁴, R³⁵, and R³⁶; or from aryl, heteroaryl, fused heterocyclylaryl, fused heteroarylheterocyclyl, fused heterocyclylheteroaryl, fused arylcycloalkyl, fused cycloalkylaryl, fused heteroarylcycloalkyl, and fused cycloalkylheteroaryl, optionally substituted with one or more substituents R⁴⁴, R⁴⁵, R⁴⁶, and R⁴⁷;
R² and R³ are taken together with the atoms to which they are attached to form a cycloalkyl or heterocyclyl ring, optionally substituted with one or more substituents R³⁴, R³⁵, and R³⁶, and optionally fused to a heteroaryl or aryl ring, optionally substituted with one or more substituents R⁴⁴, R⁴⁵, R⁴⁶, and R⁴⁷;
R⁴ is as defined in claim 2; and
R⁵ is as defined in claim 2;
wherein R²⁵, R²⁶, R²⁷, R³⁴, R³⁵, R³⁶, R⁴⁴, R⁴⁵, R⁴⁶, and R⁴⁷ are as defined in claim 2; or a pharmaceutically acceptable salt, solvate or prodrug thereof.

14. A compound according to claim 13 of the general formula (IIa) 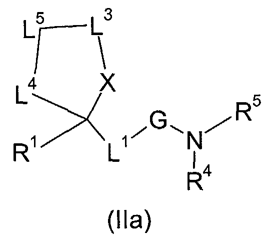 wherein
G, X, L¹, R¹, R⁴ and R⁵ are as defined in claim 13;
L₃ is -(C(R¹¹)(R¹²))ₚ-;
L₄ is -(C(R¹³)(R¹⁴))_{q}-; wherein
R¹¹, R¹², R¹³, and R¹⁴ independently are hydrogen or alkyl optionally substituted with lower alkyl, phenyl or hydroxy,
p is an integer of from 0 to 3, and
q is an integer of from 0 to 3;
and
L⁵ is a direct bond, alkenylene, alkynylene, arylene, heteroarylene, -O-, -C(O)-, -S-, -S(O)-, -S(O₂)-, or -N(R¹⁵)-, wherein
R¹⁵ is hydrogen or alkyl, which may optionally be substituted with one or more substituents R⁵⁴, R⁵⁵, and R⁵⁶, wherein
R⁵⁴, R⁵⁵, and R⁵⁶ are independently selected from -CHF₂, -CF₃, -OCF₃, -OCHF₂, -OCH₂CF₃, -OCF₂CHF₂, -S(O)₂CF₃, -SCF₃, -OR⁵⁷, -NR⁵⁷R⁵⁸, -SR⁵⁷, -NR⁵⁷S(O)₂R⁵⁸, -S(O)₂NR⁵⁷R⁵⁸, -S(O)NR⁵⁷R⁵⁸, -S(O)R⁵⁷, -S(O)₂R⁵⁷, -C(O)NR⁵⁷R⁵⁸, -OC(O)NR⁵⁷R⁵⁸, -NR⁵⁷C(O)R⁵⁸, -CH₂C(O)NR⁵⁷R⁵⁸, -OCH₂C(O)NR⁵⁷R⁵⁸, -CH₂OR⁵⁷, -CH₂NR⁵⁷R⁵⁸, -OC(O)R⁵⁷, -C(O)R⁵⁷, and -C(O)OR⁵⁷;
C₂₋₆-alkenyl and C₂₋₆-alkynyl, which may optionally be substituted with one or more substituents selected from -CN, -CF₃, -OCF₃, -OR⁵⁷, -NR⁵⁷R⁵⁸ and C₁₋₆-alkyl;
C₃₋₈-cycloalkyl, C₄₋₈-cycloalkenyl, heterocyclyl, C₃₋₈-cycloalkyl-C₁₋₆-alkyl, C₃₋₈-cycloalkyl-C₁₋₆-alkoxy, C₃₋₈-cycloalkyloxy, C₃₋₈-cycloalkyl-C₁₋₆-alkylthio, C₃₋₈-cycloalkylthio, C₃₋₈-cycloalkyl-C₂₋₆-alkenyl, C₃₋₈-cycloalkyl-C₂₋₆-alkynyl, C₄₋₈-cycloalkenyl-C₁₋₆-alkyl, C₄₋₈-cycloalkenyl-C₂₋₆-alkenyl, C₄₋₈-cycloalkenyl-C₂₋₆-alkynyl, heterocyclyl-C₁₋₆-alkyl, heterocyclyl-C₂₋₆-alkenyl, heterocyclyl-C₂₋₆-alkynyl, aryl, aryloxy, aryloxycarbonyl, aroyl, aryl-C₁₋₆-alkoxy, aryl-C₁₋₆-alkyl, aryl-C₂₋₆-alkenyl, aryl-C₂₋₆-alkynyl, heteroaryl, heteroaryl-C₁₋₆-alkyl, heteroaryl-C₂₋₆-alkenyl and heteroaryl-C₂₋₆-alkynyl, of which the aryl and heteroaryl moities optionally may be substituted with one or more substituents selected from halogen, -C(O)OR⁵⁷, -CN, -CF₃, -OCF₃, -NO₂, -OR⁵⁷, -NR⁵⁷R⁵⁸ and C₁₋₆-alkyl;
wherein
R⁵⁷ and R⁵⁸ independently are hydrogen, C₁₋₆-alkyl, aryl-C₁₋₆-alkyl or aryl,
or
R⁵⁷ and R⁵⁸,when attached to the same nitrogen atom, together with the said nitrogen atom may form a 3 to 8 membered heterocyclic ring optionally containing one or two further heteroatoms selected from nitrogen, oxygen and sulfur, and optionally containing one or two double bonds.

15. A compound according to claim 14, wherein p is an integer of from 2 to 3, and q is an integer of from 2 to 3.

16. A compound according to claim 14 or claim 15, wherein L⁵ is a direct bond.

17. A compound according to any of claims 14 to 15, wherein L⁵ is an arylene, or heteroarylene.

18. A compound according to any of claims 14 to 15 wherein L⁵ is -O-.

19. A compound according to any of claims 13 to 18, wherein R⁵ is selected from the group consisting of thiazole, thiadiazole, isothiazole, pyridine, and pyrimidine; optionally substituted with one or more substituents R⁴⁸, R⁴⁹, R⁵⁰, and R⁵¹, wherein R⁴⁸, R⁴⁹, R⁵⁰, and R⁵¹ are as defined in claim 2.

20. A compound according to claim 19, wherein R⁴⁸, R⁴⁹, R⁵⁰, and R⁵¹ independently are selected from halogen, perfluoroalkyl, cyano, alkyl-Z-, aryl-alkylene-Z-, and R⁶⁵-W-alkylene-Z-; wherein
Z and W independently are selected from a direct bond, -O-, -N(R⁶⁶)-, -N(R⁶⁶)C(O)-, and -OC(O)-; wherein
R⁶⁶ is hydrogen or alkyl; and
R⁶⁵ is hydrogen or alkyl.

21. A compound according to claim 20, wherein R⁴⁸, R⁴⁹, R⁵⁰, and R⁵¹ independently are selected from the group consisting of halogen, perfluoroalkyl, cyano, alkyl-OC(O)-alkylene-, alkyl-NHC(O)-alkylene-, N(alkyl)₂-C(O)-alkylene-, alkyl-OC(O)-, NH(alkyl)-C(O)-, N(alkyl)₂-C(O)-, aryl-alkylene-O-, and aryl-alkylene-NH-.

22. A compound according to any of claims 13 to 21, wherein
X is a direct bond; and
L¹ is -O-, or -N(R⁸) -, wherein
R⁸ is as defined in claim 13.

23. A compound according to any of claims 13 to 21, wherein
X is alkylene, which may optionally be substituted with one or more substituents R²⁵, R²⁶, and R²⁷, or a direct bond; and
L¹ is -N(R⁸) -, wherein
R⁸ is as defined in claim 13.

24. A compound according to claim 22 or 23, wherein L¹ is -N(R⁸) -, wherein R⁸ is hydrogen or alkyl.

25. A compound according to claim 24, wherein L¹ is -N(R⁸) -, wherein R⁸ is hydrogen.

26. A compound according to claim 2, which compound is of the general formula (III) 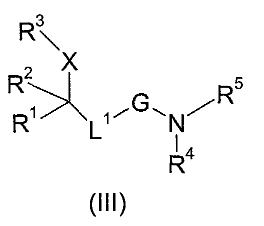 wherein
G is -S(O₂)-, or -C(O)-;
R² is hydrogen or alkyl, which may optionally be substituted with one or more substituents R³¹, R³², and R³³;
X is alkylene, which may optionally be substituted with one or more substituents R²⁵, R²⁶, and R²⁷, or a direct bond;
L¹ is -O-, or -N(R⁸)-, wherein R⁸ is as defined in claim 2;
R¹ and R³ independently are selected from alkyl, alkenyl, alkynyl, cycloalkyl, and heterocyclyl, optionally substituted with one or more substituents R³⁴, R³⁵, and R³⁶; or from aryl, heteroaryl, fused heterocyclylaryl, fused heteroarylheterocyclyl, fused heterocyclylheteroaryl, fused arylcycloalkyl, fused cycloalkylaryl, fused heteroarylcycloalkyl, and fused cycloalkylheteroaryl, optionally substituted with one or more substituents R⁴⁴, R⁴⁵, R⁴⁶, and R⁴⁷;
or
R¹ and R² may be taken together with the atoms to which they are attached to form a cycloalkyl or heterocyclyl ring, optionally substituted with one or more substituents R³⁴, R³⁵, and R³⁶, and optionally fused to a heteroaryl or aryl ring, optionally substituted with one or more substituents R⁴⁴, R⁴⁵, R⁴⁶, and R⁴⁷;
or
R² and R³ may be taken together with the atoms to which they are attached to form a cycloalkyl or heterocyclyl ring, optionally substituted with one or more substituents R³⁴, R³⁵, and R³⁶, and optionally fused to a heteroaryl or aryl ring, optionally substituted with one or more substituents R⁴⁴, R⁴⁵, R⁴⁶, and R⁴⁷;
R⁴ is as defined in claim 2; and
R⁵ is as defined in claim 2;
wherein R²⁵, R²⁶, R²⁷, R³¹, R³², R³³, R³⁴, R³⁵, R³⁶, R⁴⁴, R⁴⁵, R⁴⁶, and R⁴⁷ are as defined in claim 2;
or a pharmaceutically acceptable salt, solvate or prodrug thereof.

27. A compound according to claim 26, wherein R⁵ is selected from the group consisting of thiazole, thiadiazole, isothiazole, pyridine, and pyrimidine; optionally substituted with one or more substituents R⁴⁸, R⁴⁹, R⁵⁰, and R⁵¹, wherein R⁴⁸, R⁴⁹, R⁵⁰, and R⁵¹ are as defined in claim 2.

28. A compound according to claim 27, wherein R⁴⁸, R⁴⁹, R⁵⁰, and R⁵¹ independently are selected from halogen, perfluoroalkyl, cyano, alkyl-Z-, aryl-alkylene-Z-, and R⁶⁵-W-alkylene-Z-; wherein
Z and W independently are selected from a direct bond, -O-, -N(R⁶⁶)-, -N(R⁶⁶)C(O)-, and -OC(O)-; wherein
R⁶⁶ is hydrogen or alkyl; and
R⁶⁵ is hydrogen or alkyl.

29. A compound according to claim 28, wherein R⁴⁸, R⁴⁹, R⁵⁰, and R⁵¹ independently are selected from the group consisting of halogen, perfluoroalkyl, cyano, alkyl-OC(O)-alkylene-, alkyl-NHC(O)-alkylene-, N(alkyl)₂-C(O)-alkylene-, alkyl-OC(O)-, NH(alkyl)-C(O)-, N(alkyl)₂-C(O)-, aryl-alkylene-O-, and aryl-alkylene-NH-.

30. A compound according to claim 2, which compound is of the general formula (IV); 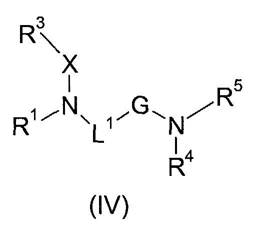 wherein
G is -S(O₂)-, or -C(O)-; and
X is a direct bond, -O-, -S-, -S(O)-, -S(O₂)-, or -N(R⁶)-, wherein
R⁶ is as defined in claim 2, and
L¹ is -(CH₂)ₙ- C(R⁹)(R¹⁰)ₘ-Y-, or a direct bond, wherein
n is an integer of from 1 to 6,
R⁹ and R¹⁰ are as defined in claim 2;
m is an integer of 0 to 1, and
Y is a direct bond, -O- or -N(R⁷)-, wherein
R⁷ is as defined in claim 2;
or
X is alkylene, which may optionally be substituted with one or more substituents R²⁵, R²⁶, and R²⁷, or a direct bond, and
L¹ is -O-, or -N(R⁸)-; wherein
R⁸ is as defined in claim 2;
R¹ and R³ independently are selected from alkyl, alkenyl, alkynyl, cycloalkyl, and heterocyclyl, optionally substituted with one or more substituents R³⁴, R³⁵, and R³⁶, or from aryl, heteroaryl, fused heterocyclylaryl, fused heteroarylheterocyclyl, fused heterocyclylheteroaryl, fused arylcycloalkyl, fused cycloalkylaryl, fused heteroarylcycloalkyl, and fused cycloalkylheteroaryl, optionally substituted with one or more substituents R⁴⁴, R⁴⁵, R⁴⁶, and R⁴⁷;
or
R¹ and R³ may be taken together with the atoms to which they are attached to form a cycloalkyl or heterocyclyl ring, optionally substituted with one or more substituents R³⁴, R³⁵, and R³⁶, and optionally fused to a heteroaryl or aryl ring, optionally substituted with one or more substituents R⁴⁴, R⁴⁵, R⁴⁶, and R⁴⁷;
R⁴ is as defined in claim 2; and
R⁵ is as defined in claim 2;
wherein R²⁵, R²⁶, R²⁷, R³⁴, R³⁵, R³⁶, R⁴⁴, R⁴⁵, R⁴⁶, and R⁴⁷ are as defined in claim 2; or a pharmaceutically acceptable salt, solvate or prodrug thereof.

31. A compound according to claim 30, wherein R⁵ is selected from the group consisting of thiazole, thiadiazole, isothiazole, pyridine, and pyrimidine; optionally substituted with one or more substituents R⁴⁸, R⁴⁹, R⁵⁰, and R⁵¹, wherein R⁴⁸, R⁴⁹, R⁵⁰, and R⁵¹ are as defined in claim 2.

32. A compound according to claim 31, wherein R⁴⁸, R⁴⁹, R⁵⁰, and R⁵¹ independently are selected from halogen, perfluoroalkyl, cyano, alkyl-Z-, aryl-alkylene-Z-, and R⁶⁵-W-alkylene-Z-; wherein
Z and W independently are selected from a direct bond, -O-, -N(R⁶⁶)-, -N(R⁶⁶)C(O)-, and -OC(O)-; wherein
R⁶⁶ is hydrogen or alkyl; and
R⁶⁵ is hydrogen or alkyl.

33. A compound according to claim 32, wherein R⁴⁸, R⁴⁹, R⁵⁰, and R⁵¹ independently are selected from the group consisting of halogen, perfluoroalkyl, cyano, alkyl-OC(O)-alkylene-, alkyl-NHC(O)-alkylene-, N(alkyl)2-C(O)-alkylene-, alkyl-OC(O)-, NH(alkyl)-C(O)-, N(alkyl)₂-C(O)-, aryl-alkylene-O-, and aryl-alkylene-NH-.

34. A compound according to any of claims 2 to 33, wherein G is -C(O)-.

35. A compound according to any of claims 1 to 34, which compound is an activator of glucokinase, when tested in the Glucokinase Activation Assay (I) disclosed herein.

36. A compound according to any of claims 1 to 34, which compound, at a concentration of 30 µM, is capable of providing an at least 1.3 fold activation of glucokinase in the Glucokinase Activation Assay (I) disclosed herein.

37. A carboxamide or sulfonamide compound having a heteroatom in the alpha, beta, or gamma position relative to the carboxamide or sulfonamide, respectively, which compound, at a concentration of 30 µM, is capable of providing an at least 1.3 fold activation of glucokinase in the Glucokinase Activation Assay (I) disclosed herein.

38. A compound according to claim 36 or claim 37, which compound, at a concentration of 30 µM, is capable of providing an at least 1.5, such as at least 1.7, for instance at least 2.0 fold activation of glucokinase in the Glucokinase Activation Assay (I) disclosed herein.

39. A compound according to any of claims 1 to 34, which compound, at a concentration of 5 µM, is capable of providing an at least 1.3 fold activation of glucokinase in the Glucokinase Activation Assay (I) disclosed herein.

40. A carboxamide or sulfonamide compound having a heteroatom in the alpha, beta, or gamma position relative to the carboxamide or sulfonamide, respectively, which compound, at a concentration of 5 µM, is capable of providing an at least 1.3 fold activation of glucokinase in the Glucokinase Activation Assay (I) disclosed herein.

41. A compound according to claim 39 or claim 40, which at a concentration of 5 µM is capable of providing an at least 1.5, such as at least 1.7, for instance at least 2.0 fold activation of glucokinase in the Glucokinase Activation Assay (I) disclosed herein.

42. A compound according to any one of claims 1 to 41, which is an agent useful for the treatment of an indication selected from the group consisting of hyperglycemia, IGT, insulin resistance syndrome, syndrome X, type 2 diabetes, type 1 diabetes, dyslipidemia, hypertension, and obesity.

43. A compound according to claim 1 or claim 2, which is 2-(3,4-dichloro-phenoxy)-hexanoic acid thiazol-2-yl amide; 2-(4-fluorophenoxy)-N-1,3-thiazol-2-ylhexanamide; 2-(4-methoxyphenoxy)-N-1,3-thiazol-2-ylhexanamide; 2-(4-methoxyphenoxy)-N-pyridin-2-ylhexanamide; 2-(3,4-dichlorophenoxy)-4-methyl-N-1,3-thiazol-2-ylpentanamide; 2-(1,1'-biphenyl-4-yloxy)-N-1,3-thiazol-2-ylhexanamide; 2-(4-isopropylphenoxy)-N-1,3-thiazol-2-ylhexanamide; 2-(3-methoxyphenoxy)-N-1,3-thiazol-2-ylhexanamide; 2-(2,3-dimethoxyphenoxy)-N-1,3-thiazol-2-ylhexanamide; 2-(3,4-dimethoxyphenoxy)-N-1,3-thiazol-2-ylhexanamide; 2-(3,5-dimethoxyphenoxy)-N-1,3-thiazol-2-ylhexanamide; 2-(2-naphthyloxy)-N-1,3-thiazol-2-ylhexanamide; 2-(2,4-difluorophenoxy)-N-1,3-thiazol-2-ylhexanamide; 2-(3,4-difluorophenoxy)-N-1,3-thiazol-2-ylhexanamide; 2-(1,3-benzodioxol-5-yloxy)-N-1,3-thiazol-2-ylhexanamide; 2-(4-methoxyphenoxy)-N-1,3-thiazol-2-ylheptanamide; 2-(4-fluorophenoxy)-N-1,3-thiazol-2-ylheptanamide; 2-(4-Chloro-phenylsulfanyl)-hexanoic acid thiazol-2-ylamide; 2-(4-Chloro-phenylsulfanyl)-hexanoic acid pyridin-2-ylamide; N-[1 -(4-chlorophenyl)cyclopentyl]-N'-1,3-thiazol-2-ylurea; N-[1-(4-chlorophenyl)cyclopentyl]-N'-pyridin-2-ylurea; N-[1 -(4-chlorophenyl)cyclohexyl]-N'-1,3-thiazol-2-ylurea; N-[1-(4-chlorophenyl)cyclohexyl]-N'-pyridin-2-ylurea; 3-(4-chlorophenyl)-N-pyridin-2-yl-3-(tetrahydro-2H-thiopyran-4-ylamino)propanamide; 3-(4-chlorophenyl)-3-(tetrahydro-2H-thiopyran-4-ylamino)-N-1,3-thiazol-2-ylpropanamide; 2-(4-chlorophenyl)-2-(4-methylphenoxy)-N-pyridin-2-ylacetamide; 2-(4-chlorophenyl)-2-(4-methylphenoxy)-N-1,3-thiazol-2-ylacetamide; 2-(4-bromophenoxy)-2-(4-chlorophenyl)-N-pyridin-2-ylacetamide; 2-(4-bromophenoxy)-2-(4-chlorophenyl)-N-1,3-thiazol-2-ylacetamide; 2-(4-chlorophenyl)-2-(4-fluorophenoxy)-N-1,3-thiazol-2-ylacetamide; 2-(4-chlorophenyl)-2-(3,4-dichlorophenoxy)-N-pyridin-2-ylacetamide; 2-(4-bromophenoxy)-2-(4-bromophenyl)-N-pyridin-2-ylacetamide; 2-(4-bromophenoxy)-2-(4-bromophenyl)-N-1,3-thiazol-2-ylacetamide; 2-(4-bromophenyl)-2-(4-methylphenoxy)-N-pyridin-2-ylacetamide; 2-(4-bromophenyl)-2-(4-fluorophenoxy)-N-1,3-thiazol-2-ylacetamide; 2-(4-bromophenyl)-2-phenoxy-N-1,3-thiazol-2-ylacetamide; 2-(4-fluorophenoxy)-2-(4-fluorophenyl)-N-pyridin-2-ylacetamide; 2-(4-fluorophenoxy)-2-(4-fluorophenyl)-N-1,3-thiazol-2-ylacetamide; 2-(4-fluorophenyl)-2-(4-methylphenoxy)-N-1,3-thiazol-2-ylacetamide; 2-(4-fluorophenyl)-2-phenoxy-N-1,3-thiazol-2-ylacetamide; 2-(4-bromophenoxy)-2-(4-fluorophenyl)-N-pyridin-2-ylacetamide; 2-(4-fluorophenoxy)-N-1,3-thiazol-2-yl-2-[4-(trifluoromethyl)phenyl]acetamide; 2-(4-bromophenoxy)-N-pyridin-2-yl-2-[4-(trifluoromethyl)phenyl]acetamide; 2-cyclopentylsulfanyl-2-(3,4-dichloro-phenyl)-N-thiazol-2-yl-acetamide; 2-cyclopentylsulfanyl-2-(3,4-dichloro-phenyl)-N-pyridinyl-2-yl-acetamide; 2-cyclopentylsulfanyl-2-(3,4-dichloro-phenyl)-N-pyrimidin-2-yl-acetamide; N-(5-bromo-1,3-thiazol-2-yl)-2-(cyclopentyithio)-2-(3,4-dichlorophenyl)acetamide; 2-(cyclopentylthio)-2-(3,4-dichlorophenyl)-N-1,3,4-thiadiazol-2-ylacetamide; 2-(3,4-dichlorophenyl)-2-(isobutylthio)-N-pyridin-2-ylacetamide; 2-(3,4-dichlorophenyl)-2-(isobutylthio)-N-1,3-thiazol-2-ylacetamide; 2-(3,4-dichlorophenyl)-2-[(2-furylmethyl)thio]-N-pyridin-2-ylacetamide; 2-(3,4-dichlorophenyl)-2-[(2-furylmethyl)thio]-N-1,3-thiazol-2-ylacetamide; 2-(3,4-dichlorophenyl)-2-[(2-furylmethyl)thio]-N-pyridin-2-ylacetamide; 2-[(4-fluorophenyl)thio]-N-pyridin-2-yl-2-[4-(trifluoromethyl)phenyl]acetamide; 2-[(4-fluorophenyl)thio]-N-1,3-thiazol-2-yl-2-[4-(trifluoromethyl)phenyl]acetamide; 2-[(4-methylphenyl)thio]-N-1,3-thiazol-2-yl-2-[4-(trifluoromethyl)phenyl]acetamide; 2-(4-fluorophenyl)-2-[(4-fluorophenyl)thio]-N-pyridin-2-ylacetamide; 2-(4-bromophenyl)-2-[(4-fluorophenyl)thio]-N-pyridin-2-ylacetamide; 2-(4-bromophenyl)-2-[(4-methylphenyl)thio]-N-pyridin-2-ylacetamide; 1-Cyclopentylmethyl-1-(3,4-dichloro-phenyl)-3-thiazol-2-yl-urea; N-cyclopentyl-N-(3,4-dichlorophenyl)-N'-1,3-thiazol-2-ylurea; 1-(3,4-Dichloro-phenyl)-1 -propyl-3-thiazol-2-yl-urea; 2-cyclopentyl-1-(3,4-dichlorophenyl)ethyl pyridin-2-ylcarbamate; or 2-cyclopentyl-1-(3,4-dichlorophenyl)ethyl 1,3-thiazol-2-ylcarbamate.

44. A compound according to any one of claims 1 to 43 for use as a medicament.

45. A compound according to any one of claims 1 to 43 for treatment of hyperglycemia, IGT, Syndrome X, type 2 diabetes, type 1 diabetes, dyslipidemia, hyperlipidemia or hypertension.

46. A compound according to any one of claims 1 to 43 for the treatment or prophylaxis of obesity.

47. A compound according to any one of claims 1 to 43 for lowering of food intake, appetite regulation and/or regulating feeding behaviour.

48. A compound according to any one of claims 1 to 43 for enhancing the secretion of enteroincretins.

49. A compound according to claim 48, said enteroincretin is GLP-1.

50. A pharmaceutical composition comprising, as an active ingredient, at least one compound according to any one of claims 1 to 49 together with one or more pharmaceutically acceptable carriers or excipients.

51. A pharmaceutical composition according to claim 50 comprising a further antidiabetic agent.

52. Use of a compound according to any of claims 1 to 49 for increasing the activity of glucokinase.

53. Use of a compound according to any one of claims 1 to 49 for the preparation of a medicament for the treatment of hyperglycemia, for the treatment of IGT, for the treatment of Syndrome X, for the treatment of type 2 diabetes, for the treatment of type 1 diabetes, for the treatment of dyslipidemia or hyperlipidemia, or for the treatment of hypertension.

54. Use of a compound according to any one of claims 1 to 49 for the preparation of a medicament for lowering of food intake, for appetite regulation or for the treatment or prophylaxis of obesity.

55. Use according to claim 53 or 54 in a regimen which comprises treatment with a further antidiabetic agent.

56. Use according to any one of claims 53 to 55 in a regimen which comprises treatment with a further antihyperlipidemic agent.

57. Use according to any one of claims 53 to 56 in a regimen which comprises treatment with a further antiobesity agent.

58. Use according to any one of claims 53 to 57 in a regimen which comprises treatment with a further antihypertensive agent.

59. Use of a compound according to any of claims 1 to 49 or a pharmaceutical composition according to claim 50 or 51 for treatment of IGT, for treatment of Syndrome X, for treatment of type 2 diabetes, for treatment of type 1 diabetes, for treatment of dyslipidemia or hyperlipidemia, or for the treatment of hypertension.

60. Use of a compound according to any of claims 1 to 49 or a pharmaceutical composition according to claim 50 or 51 for the treatment or prophylaxis of obesity, for lowering of food intake, for appetite regulation and/or for regulating feeding behaviour.

61. Use of a compound according to any of claims 1 to 49 or a pharmaceutical composition according to claim 50 or 51 for enhancing the secretion of enteroincretins.

62. Use according to claim 61, where said enteroincretin is GLP-1.
